# EUROPEAN PATENT SPECIFICATION

(11) **EP 2 764 370 B1**
(45) Date of publication and mention of the grant of the patent: **13.09.2017**
(21) Application number: 12778001.3
(22) Date of filing: 06.10.2012
(51) Int. Cl.: G01N 33/68

(54) **MYOSIN BINDING PROTEIN-C FOR USE IN METHODS RELATING TO DIASTOLIC HEART FAILURE**
MYOSINBINDENDES PROTEIN-C ZUR VERWENDUNG BEI VERFAHREN IM ZUSAMMENHANG MIT DIASTOLISCHER HERZINSUFFIZIENZ
PROTÉINE-C DE LIAISON À LA MYOSINE À UTILISER DANS DES MÉTHODES ASSOCIÉES À L'INSUFFISANCE CARDIAQUE DIASTOLIQUE

(30) Priority: 06.10.2011 US 201161544030 P; 02.04.2012 US 201261618900 P; 29.05.2012 US 201261652734 P; 12.06.2012 US 201261658585 P; 30.07.2012 US 201261677292 P
(43) Date of publication of application: 13.08.2014
(73) Proprietor: The Board of Trustees of the University of Illinois, Urbana, IL 61801 (US); The United States Government as represented by The Department of Veterans Affairs, Washington, DC 20420 (US)
(72) Inventor: DUDLEY, Samuel, Providence, RI 02906 (US); SOLARO, Ross, John, Chicago, IL 60611 (US); JEONG, Euy-Myoung, Wilmette, IL 60091 (US)
(74) Representative: Potter Clarkson LLP
(86) International application number: PCT/US2012/059150
(87) International publication number: WO 2013/052933

(56) References cited:
- WO-A1-2010/130985
- PALMER BRADLEY M ET AL: "Role of cardiac myosin binding protein C in sustaining left ventricular systolic stiffening", CIRCULATION RESEARCH, GRUNE AND STRATTON, BALTIMORE, US, vol. 94, no. 9, 14 May 2004 (2004-05-14), pages 1249-1255, XP002482093, ISSN: 0009-7330, DOI: 10.1161/01.RES.0000126898.95550.31
- STAHRENBERG RAOUL ET AL: "The novel biomarker growth differentiation factor 15 in heart failure with normal ejection fraction.", EUROPEAN JOURNAL OF HEART FAILURE DEC 2010, vol. 12, no. 12, December 2010 (2010-12), pages 1309-1316, XP009166146, ISSN: 1879-0844

### SEQUENCE LISTING

<110> Dudley, Samuel
<120> METHODS FOR DIFFERENTIATING BETWEEN DIASTOLIC AND SYSTOLIC HEART FAILURE
<130> 30303/47002 PCT
<160> 21
<170> PatentIn version 3.5
<210> 1
   <211> 1274
   <212> PRT
   <213> Homo sapiens
<300>
   <308> NCBI / NP_000247.2
   <309> 2012-06-02
   <313> (1)..(1274)
<400> 1
<210> 2
   <211> 20
   <212> PRT
   <213> Artificial Sequence
<220>
   <223> Synthetic peptide
<400> 2
   Ile His Leu Asp Cys Pro Gly Ser Thr Pro Asp Thr Ile Val Val Val
   1 5 10 15
   Ala Gly Asn Lys
   20
<210> 3
   <211> 15
   <212> PRT
   <213> Artificial Sequence
<220>
   <223> Synthetic peptide
<400> 3
   Val Glu Phe Glu Cys Glu Val Ser Glu Glu Gly Ala Gln Val Lys
   1 5 10 15
<210> 4
   <211> 28
   <212> PRT
   <213> Artificial Sequence
<220>
   <223> Synthetic peptide
<400> 4
   Leu Thr Ile Asp Asp Val Thr Pro Ala Asp Glu Ala Asp Tyr Ser Phe
   1 5 10 15
   Val Pro Glu Gly Phe Ala Cys Asn Leu Ser Ala Lys
   20 25
<210> 5
   <211> 20
   <212> PRT
   <213> Artificial Sequence
<220>
   <223> Synthetic peptide
<220>
   <221> MISC_FEATURE
   <222> (5)..(5)
   <223> S-glutathionylated
<400> 5
   Ile His Leu Asp Cys Pro Gly Ser Thr Pro Asp Thr Ile Val Val Val
   1 5 10 15
   Ala Gly Asn Lys
   20
<210> 6
   <211> 15
   <212> PRT
   <213> Artificial Sequence
<220>
   <223> Synthetic peptide
<220>
   <221> MISC_FEATURE
   <222> (5)..(5)
   <223> S-glutathionylated
<400> 6
   Val Glu Phe Glu Cys Glu Val Ser Glu Glu Gly Ala Gln Val Lys
   1               5                   10                  15
<210> 7
   <211> 28
   <212> PRT
   <213> Artificial Sequence
<220>
   <223> Synthetic peptide
<220>
   <221> MISC_FEATURE
   <222> (23)..(23)
   <223> S-glutathionylated
<400> 7
   Leu Thr Ile Asp Asp Val Thr Pro Ala Asp Glu Ala Asp Tyr Ser Phe
   1               5                   10                  15
   Val Pro Glu Gly Phe Ala Cys Asn Leu Ser Ala Lys
               20                  25
<210> 8
   <211> 20
   <212> PRT
   <213> Artificial Sequence
<220>
   <223> Synthetic peptide
<220>
   <221> DISULFID
   <222> (5)..(5)
   <223> Disulfide bonded to glutathione
<400> 8
   Ile His Leu Asp Cys Pro Gly Ser Thr Pro Asp Thr Ile Val Val Val
   1               5                   10                  15
   Ala Gly Asn Lys
               20
<210> 9
   <211> 15
   <212> PRT
   <213> Artificial Sequence
<220>
   <223> Synthetic peptide
<220>
   <221> DISULFID
   <222> (5)..(5)
   <223> Disulfide bonded to glutathione
<400> 9
   Val Glu Phe Glu Cys Glu Val Ser Glu Glu Gly Ala Gln Val Lys
   1               5                   10                  15
<210> 10
   <211> 28
   <212> PRT
   <213> Artificial Sequence
<220>
   <223> Synthetic peptide
<220>
   <221> DISULFID
   <222> (23)..(23)
   <223> Disulfide bonded to glutathione
<400> 10
   Leu Thr Ile Asp Asp Val Thr Pro Ala Asp Glu Ala Asp Tyr Ser Phe
   1               5                   10                  15
   Val Pro Glu Gly Phe Ala Cys Asn Leu Ser Ala Lys
               20                  25
<210> 11
   <211> 22
   <212> PRT
   <213> Artificial Sequence
<220>
   <223> Synthetic peptide
<400> 11
   Lys Ile His Leu Asp Cys Pro Gly Ser Thr Pro Asp Thr Ile Val Val
   1               5                   10                  15
   Val Ala Gly Asn Lys Leu
               20
<210> 12
   <211> 22
   <212> PRT
   <213> Artificial Sequence
<220>
   <223> Synethtic peptide
<400> 12
   Lys Ile His Leu Asp Cys Pro Gly Ser Thr Pro Asp Thr Ile Val Val
   1 5 10 15
   Val Ala Gly Asn Lys Leu
   20
<210> 13
   <211> 22
   <212> PRT
   <213> Artificial Sequence
<220>
   <223> Synthetic peptide
<400> 13
   Lys Ile His Leu Asp Cys Pro Gly Ser Thr Pro Asp Thr Ile Val Val
   1 5 10 15
   Val Ala Gly Asn Lys Leu
   20
<210> 14
   <211> 22
   <212> PRT
   <213> Artificial Sequence
<220>
   <223> Synthetic peptide
<400> 14
   Lys Ile His Leu Asp Cys Pro Gly Ser Thr Pro Asp Thr Ile Val Val
   1 5 10 15
   Val Ala Gly Asn Lys Leu
   20
<210> 15
   <211> 22
   <212> PRT
   <213> Artificial Sequence
<220>
   <223> Synthetic peptide
<400> 15
   Lys Ile His Leu Asp Cys Pro Gly Ser Thr Pro Asp Thr Ile Val Val
   1 5 10 15
   Val Ala Gly Asn Lys Leu
   20
<210> 16
   <211> 17
   <212> PRT
   <213> Artificial Sequence
<220>
   <223> Synthetic peptide
<400> 16
   Arg Val Glu Phe Glu Cys Glu Val Ser Glu Glu Gly Ala Gln Val Lys
   1               5                   10                  15
   Trp
<210> 17
   <211> 17
   <212> PRT
   <213> Artificial Sequence
<220>
   <223> Synthetic peptide
<400> 17
   Arg Val Glu Phe Glu Cys Glu Val Ser Glu Glu Gly Ala Gln Val Lys
   1               5                   10                  15
   Trp
<210> 18
   <211> 17
   <212> PRT
   <213> Artificial Sequence
<220>
   <223> Synthetic peptide
<400> 18
   Arg Val Glu Phe Glu Cys Glu Val Ser Glu Glu Gly Ala Gln Val Lys
   1               5                   10                  15
   Trp
<210> 19
   <211> 30
   <212> PRT
   <213> Artificial Sequence
<220>
   <223> Synthetic peptide
<400> 19
   Lys Leu Thr Ile Asp Asp Val Thr Pro Ala Asp Glu Ala Asp Tyr Ser
   1               5                   10                  15
   Phe Val Pro Glu Gly Phe Ala Cys Asn Leu Ser Ala Lys Leu
               20                  25                  30
<210> 20
   <211> 30
   <212> PRT
   <213> Artificial Sequence
<220>
   <223> Synthetic peptide
<400> 20
   Lys Leu Thr Ile Asp Asp Val Thr Pro Ala Asp Glu Ala Asp Tyr Ser
   1               5                   10                  15
   Phe Val Pro Glu Gly Phe Ala Cys Asn Leu Ser Ala Lys Leu
               20                  25                  30
<210> 21
   <211> 1274
   <212> PRT
   <213> Homo sapiens
<300>
   <308> GenBank / CAA58882.1
   <309> 1995-07-07
   <313> (1)..(1274)
<400> 21
   Met Pro Glu Pro Gly Lys Lys Pro Val Ser Ala Phe Ser Lys Lys Pro
   1               5                   10                  15
   Arg Ser Val Glu Val Ala Ala Gly Ser Pro Ala Val Phe Glu Ala Glu
               20                  25                  30
   Thr Glu Arg Ala Gly Val Lys Val Arg Trp Gln Arg Gly Gly Ser Asp
           35                  40                  45
   Ile Ser Ala Ser Asn Lys Tyr Gly Leu Ala Thr Glu Gly Thr Arg His
       50                  55                  60
   Thr Leu Thr Val Arg Glu Val Gly Pro Ala Asp Gln Gly Ser Tyr Ala
   65                  70                  75                  80
   Val Ile Ala Gly Ser Ser Lys Val Lys Phe Asp Leu Lys Val Ile Glu
                   85                  90                  95
   Ala Glu Lys Ala Glu Pro Met Leu Ala Pro Ala Pro Ala Pro Ala Glu
               100                 105                 110
   Ala Thr Gly Ala Pro Gly Glu Ala Pro Ala Pro Ala Ala Glu Leu Gly
   115 120 125
   Glu Ser Ala Pro Ser Pro Lys Gly Ser Ser Ser Ala Ala Leu Asn Gly
   130 135 140
   Pro Thr Pro Gly Ala Pro Asp Asp Pro Ile Gly Leu Phe Val Met Arg
   145 150 155 160
   Pro Gln Asp Gly Glu Val Thr Val Gly Gly Ser Ile Thr Phe Ser Ala
   165 170 175
   Arg Val Ala Gly Ala Ser Leu Leu Lys Pro Pro Val Val Lys Trp Phe
   180 185 190
   Lys Gly Lys Trp Val Asp Leu Ser Ser Lys Val Gly Gln His Leu Gln
   195 200 205
   Leu His Asp Ser Tyr Asp Arg Ala Ser Lys Val Tyr Leu Phe Glu Leu
   210 215 220
   His Ile Thr Asp Ala Gln Pro Ala Phe Thr Gly Ser Tyr Arg Cys Glu
   225 230 235 240
   Val Ser Thr Lys Asp Lys Phe Glu Cys Ser Asn Phe Asn Leu Thr Val
   245 250 255
   His Glu Ala Met Gly Thr Gly Asp Leu Asp Leu Leu Ser Ala Phe Arg
   260 265 270
   Arg Thr Ser Leu Ala Gly Gly Gly Arg Arg Ile Ser Asp Ser His Glu
   275 280 285
   Asp Thr Gly Ile Leu Asp Phe Ser Ser Leu Leu Lys Lys Arg Asp Ser
   290 295 300
   Phe Arg Thr Pro Arg Asp Ser Lys Leu Glu Ala Pro Ala Glu Glu Asp
   305 310 315 320
   Val Trp Glu Ile Leu Arg Gln Ala Pro Pro Ser Glu Tyr Glu Arg Ile
   325 330 335
   Ala Phe Gln Tyr Gly Val Thr Asp Leu Arg Gly Met Leu Lys Arg Leu
   340 345 350
   Lys Gly Met Arg Arg Asp Glu Lys Lys Ser Thr Ala Phe Gln Lys Lys
   355 360 365
   Leu Glu Pro Ala Tyr Gln Val Ser Lys Gly His Lys Ile Arg Leu Thr
       370                 375                 380
   Val Glu Leu Ala Asp His Asp Ala Glu Val Lys Trp Leu Lys Asn Gly
   385                 390                 395                 400
   Gln Glu Ile Gln Met Ser Gly Ser Lys Tyr Ile Phe Glu Ser Ile Gly
                   405                 410                 415
   Ala Lys Arg Thr Leu Thr Ile Ser Gln Cys Ser Leu Ala Asp Asp Ala
               420                 425                 430
   Ala Tyr Gln Cys Val Val Gly Gly Glu Lys Cys Ser Thr Glu Leu Phe
           435                 440                 445
   Val Lys Glu Pro Pro Val Leu Ile Thr Arg Pro Leu Glu Asp Gln Leu
       450                 455                 460
   Val Met Val Gly Gln Arg Val Glu Phe Glu Cys Glu Val Ser Glu Glu
   465                 470                 475                 480
   Gly Ala Gln Val Lys Trp Leu Lys Asp Gly Val Glu Leu Thr Arg Glu
                   485                 490                 495
   Glu Thr Phe Lys Tyr Arg Phe Lys Lys Asp Gly Gln Arg His His Leu
               500                 505                 510
   Ile Ile Asn Glu Ala Met Leu Glu Asp Ala Gly His Tyr Ala Leu Cys
           515                 520                 525
   Thr Ser Gly Gly Gln Ala Leu Arg Glu Leu Ile Val Gln Glu Lys Lys
       530                 535                 540
   Leu Glu Val Tyr Gln Ser Ile Ala Asp Leu Met Val Gly Ala Lys Asp
   545                 550                 555                 560
   Gln Ala Val Phe Lys Cys Glu Val Ser Asp Glu Asn Val Arg Gly Val
                   565                 570                 575
   Trp Leu Lys Asn Gly Lys Glu Leu Val Pro Asp Ser Arg Ile Lys Val
               580                 585                 590
   Ser His Ile Gly Arg Val His Lys Leu Thr Ile Asp Asp Val Thr Pro
           595                 600                 605
   Ala Asp Glu Ala Asp Tyr Ser Phe Val Pro Glu Gly Phe Ala Cys Asn
       610                 615                 620
   Leu Ser Ala Lys Leu His Phe Met Glu Val Lys Ile Asp Phe Val Pro
   625                 630                 635                 640
   Arg Gln Glu Pro Pro Lys Ile His Leu Asp Cys Pro Gly Arg Ile Pro
                   645                 650                 655
   Asp Thr Ile Val Val Val Ala Gly Asn Lys Leu Arg Leu Asp Val Pro
               660                 665                 670
   Ile Ser Gly Asp Pro Ala Pro Thr Val Ile Trp Gln Lys Ala Ile Thr
           675                 680                 685
   Gln Gly Asn Lys Ala Pro Ala Arg Pro Ala Pro Asp Ala Pro Glu Asp
       690                 695                 700
   Thr Gly Asp Ser Asp Glu Trp Val Phe Asp Lys Lys Leu Leu Cys Glu
   705                 710                 715                 720
   Thr Glu Gly Arg Val Arg Val Glu Thr Thr Lys Asp Arg Ser Ile Phe
                   725                 730                 735
   Thr Val Glu Gly Ala Glu Lys Glu Asp Glu Gly Val Tyr Thr Val Thr
               740                 745                 750
   Val Lys Asn Pro Val Gly Glu Asp Gln Val Asn Leu Thr Val Lys Val
           755                 760                 765
   Ile Asp Val Pro Asp Ala Pro Ala Ala Pro Lys Ile Ser Asn Val Gly
       770                 775                 780
   Glu Asp Ser Cys Thr Val Gln Trp Glu Pro Pro Ala Tyr Asp Gly Gly
   785                 790                 795                 800
   Gln Pro Ile Leu Gly Tyr Ile Leu Glu Arg Lys Lys Lys Lys Ser Tyr
                   805                 810                 815
   Arg Trp Met Gln Leu Asn Phe Asp Leu Ile Gln Glu Leu Ser His Glu
               820                 825                 830
   Ala Arg Arg Met Ile Glu Gly Val Val Tyr Glu Met Arg Val Tyr Ala
           835                 840                 845
   Val Asn Ala Ile Gly Met Ser Arg Pro Ser Pro Ala Ser Gln Pro Phe
       850                 855                 860
   Met Pro Ile Gly Pro Pro Ser Glu Pro Thr His Leu Ala Val Glu Asp
   865                 870                 875                 880
   Val Ser Asp Thr Thr Val Ser Leu Lys Trp Arg Pro Pro Glu Arg Val
                   885                 890                 895
   Gly Ala Gly Gly Leu Asp Gly Tyr Ser Val Glu Tyr Cys Pro Glu Gly
               900                 905                 910
   Cys Ser Glu Trp Val Ala Ala Leu Gln Gly Leu Thr Glu His Thr Ser
           915                 920                 925
   Ile Leu Val Lys Asp Leu Pro Thr Gly Ala Arg Leu Leu Phe Arg Val
       930                 935                 940
   Arg Ala His Asn Met Ala Gly Pro Gly Ala Pro Val Thr Thr Thr Glu
   945                 950                 955                 960
   Pro Val Thr Val Gln Glu Ile Leu Gln Arg Pro Arg Leu Gln Leu Pro
                   965                 970                 975
   Arg His Leu Arg Gln Thr Ile Gln Lys Lys Val Gly Glu Pro Val Asn
               980                 985                 990
   Leu Leu Ile Pro Phe Gln Gly Lys  Pro Arg Pro Gln Val  Thr Trp Thr
           995                 1000                 1005
   Lys Glu  Gly Gln Pro Leu Ala  Gly Glu Glu Val Ser  Ile Arg Asn
       1010                 1015                 1020
   Ser Pro  Thr Asp Thr Ile Leu  Phe Ile Arg Ala Ala  Arg Arg Val
       1025                 1030                 1035
   His Ser  Gly Thr Tyr Gln Val  Thr Val Arg Ile Glu  Asn Met Glu
       1040                 1045                 1050
   Asp Lys  Ala Thr Leu Val Leu  Gln Val Val Asp Lys  Pro Ser Pro
       1055                 1060                 1065
   Pro Gln  Asp Leu Arg Val Thr  Asp Ala Trp Gly Leu  Asn Val Ala
       1070                 1075                 1080
   Leu Glu  Trp Lys Pro Pro Gln  Asp Val Gly Asn Thr  Glu Leu Trp
       1085                 1090                 1095
   Gly Tyr  Thr Val Gln Lys Ala  Asp Lys Lys Thr Met  Glu Trp Phe
       1100                 1105                 1110
   Thr Val  Leu Glu His Tyr Arg  Arg Thr His Cys Val  Val Pro Glu
           1115                1120                1125
   Leu Ile  Ile Gly Asn Gly Tyr  Tyr Phe Arg Val Phe  Ser Gln Asn
       1130                1135                1140
   Met Val  Gly Phe Ser Asp Arg  Ala Ala Thr Thr Lys  Glu Pro Val
       1145                1150                1155
   Phe Ile  Pro Arg Pro Gly Ile  Thr Tyr Glu Pro Pro  Asn Tyr Lys
       1160                1165                1170
   Ala Leu  Asp Phe Ser Glu Ala  Pro Ser Phe Thr Gln  Pro Leu Val
       1175                1180                1185
   Asn Arg  Ser Val Ile Ala Gly  Tyr Thr Ala Met Leu  Cys Cys Ala
       1190                1195                1200
   Val Arg  Gly Ser Pro Lys Pro  Lys Ile Ser Trp Phe  Lys Asn Gly
       1205                1210                1215
   Leu Asp  Leu Gly Glu Asp Ala  Arg Phe Arg Met Phe  Ser Lys Gln
       1220                1225                1230
   Gly Val  Leu Thr Leu Glu Ile  Arg Lys Pro Cys Pro  Phe Asp Gly
       1235                1240                1245
   Gly Ile  Tyr Val Cys Arg Ala  Thr Asn Leu Gln Gly  Glu Ala Arg
       1250                1255                1260
   Cys Glu  Cys Arg Leu Glu Val  Arg Val Pro Gln
       1265                1270

## Description

### BACKGROUND

Currently, about 5.7 million people in the United States suffer from heart failure (HF), and about 670,000 people are diagnosed with the condition each year. Up to 50% of HF patients have diastolic heart failure, resulting in a patient population of approximately 2.3 million people in the United States. The clinical manifestation of diastolic heart failure demonstrates normal left ventricular (LV) end diastolic volume, a normal ejection fraction, delayed active relaxation, and increased passive stiffness of LV.¹

About one in five people who have HF die within one year from diagnosis with over 280,000 deaths annually. Successful treatment of patients suffering from heart failure depends on the type of cardiac dysfunction present, since the treatment of diastolic heart failure is very different from the treatments used for patients suffering from systolic heart failure.

Methods of diagnosing diastolic dysfunction are known in the art and include, for example, Doppler echocardiography, cardiac catheterization, magnetic resonance imaging, tissue Doppler imaging, and measurement of left ventricular end diastolic pressure and systolic function. However, these methods can be invasive, time-consuming, and costly. Accordingly, there exists a need in the art for non-invasive, time-effective, and cost-effective methods of accurately diagnosing diastolic heart failure.

Furthermore, while there are commercially available diagnostics for diagnosing heart failure, there are no tests that differentiate between systolic dysfunction and diastolic dysfunction or systolic heart failure and diastolic heart failure. Accordingly, there exists a need in the art for non-invasive, time-effective, and cost-effective methods of differentiating between systolic dysfunction and diastolic dysfunction or systolic heart failure and diastolic heart failure.

### SUMMARY

Provided herein are methods of diagnosing diastolic dysfunction or diastolic heart failure in a subject, methods of differentiating between diastolic dysfunction and systolic dysfunction in a subject suffering from heart failure, methods of characterizing heart failure as diastolic heart failure or systolic heart failure in a subject, methods of determining a subject's need for treatment of diastolic dysfunction or diastolic heart failure, methods of identifying a patient at risk for diastolic dysfunction or diastolic heart failure, methods of reducing a subject's risk of diastolic dysfunction or diastolic heart failure, methods of monitoring a subject's risk for diastolic dysfunction or diastolic heart failure, methods of screening a compound for an ability to treat diastolic dysfunction or diastolic heart failure, methods of determining efficacy of a treatment of diastolic dysfunction or diastolic heart failure, as well as other related methods.

In exemplary embodiments, each of the methods comprises determining a level of a myosin binding protein-C (MyBP-C), or a post-translationally modified form thereof, or a fragment thereof, in a biological sample obtained from the subject. When the level of MyBP-C, or a post-translationally modified form thereof, or a fragment thereof, is increased, relative to a control level, the subject is diagnosed with diastolic dysfunction or diastolic heart failure or is determined to need treatment for diastolic heart failure or diastolic dysfunction. In exemplary aspects of these methods, the methods further comprises the step of administering to the subject a therapeutic agent for the treatment of diastolic heart failure or diastolic dysfunction, when the level of MyBP-C, or a post-translationally modified form or fragment thereof, of the biological sample is increased, relative to a control.

Also disclosed is a method comprising determining a level of asymmetric dimethylarginine (ADMA) and a level of either or both of symmetric dimethyl arginine (SDMA) and/or L-Arginine, in a biological sample obtained from the subject. In exemplary aspects, the method comprises determining a ratio of the level of L-Arginine in the biological sample to the level of ADMA in the biological sample. The subject is diagnosed with diastolic dysfunction or diastolic heart failure or is determined to need treatment for diastolic heart failure or diastolic dysfunction, when the level of ADMA is increased relative to a control level of ADMA and the level of SDMA is unchanged relative to a control level, or when the ratio of L-Arginine to ADMA is decreased relative to a control ratio.

The invention also provides methods of diagnosing diastolic dysfunction or diastolic heart failure in a subject. The methods comprise determining a level of a myosin binding protein-C (MyBP-C), or a post-translationally modified form thereof, or a fragment thereof, in a biological sample obtained from the subject, and determining a level of B-type Natriuretic Protein (BNP) in a biological sample obtained from the subject.

The invention further provides methods of treating a subject. In exemplary embodiments, the subject suffers from heart failure and the method comprises determining a level of a myosin binding protein-C (MyBP-C), or a post-translationally modified form or fragment thereof, in a biological sample obtained from the subject; and administering to the subject a therapeutic agent for the treatment of diastolic heart failure or diastolic dysfunction, when the level of MyBP-C, or a post-translationally modified form or fragment thereof, of the biological sample is increased, relative to a control. In other exemplary embodiments, the subject has been assayed for a level of a myosin binding protein-C (MyBP-C), or a post-translationally modified form or fragment thereof, and the method comprises administering to the subject a therapeutic agent for the treatment of diastolic heart failure or diastolic dysfunction, when the subject has been determined to have an increased level of MyBP-C, or a post-translationally modified form or fragment thereof.

In alternative or additional embodiments of the treatment methods, the subject suffers from heart failure and the method comprises determining a level of a ADMA and a level of either or both of SDMA and/or L-Arginine, in a biological sample obtained from the subject; and administering to the subject a therapeutic agent for the treatment of diastolic heart failure or diastolic dysfunction, when the level of ADMA of the biological sample is increased, relative to a control level, and the level of SDMA of the biological sample is unchanged, relative to a control level, or when the ratio of the level of L-Arginine of the biological sample to the level of ADMA of the biological sample is decreased, relative to a control ratio. In other additional or exemplary embodiments of the treatment methods, the subject has been assayed for a level of ADMA and a level of either or both of SDMA and/or L-Arginine, and the method comprises administering to the subject a therapeutic agent for the treatment of diastolic heart failure or diastolic dysfunction, when the level of ADMA of the biological sample is increased, relative to a control level, and the level of SDMA of the biological sample is unchanged, relative to a control level, or when the ratio of the level of L-Arginine of the biological sample to the level of ADMA of the biological sample is decreased, relative to a control ratio.

Isolated binding agents which are useful in the inventive methods are also provided herein. In exemplary embodiments, the isolated binding agent specifically binds to S-glutathionylated MyBP-C. In exemplary aspects, the MyBP-C is S-glutathionylated at one or more of Cys 239, Cys249, Cys 426, Cys 436, Cys 443, Cys 475 , Cys528, Cys566, Cys 623, Cys 651, and/or Cys 719, according to the amino acid position numbering of SEQ ID NO: 21. In exemplary embodiments, the isolated binding agent specifically binds to an epitope within MyBP-C, wherein the epitope comprises one or more of Cys 239, Cys249, Cys 426, Cys 436, Cys 443, Cys 475 , Cys528, Cys566, Cys 623, Cys 651, and/or Cys 719, according to the amino acid position numbering of SEQ ID NO: 21. In exemplary embodiments, the isolated binding agent specifically binds to a Cys residue of MyBP-C, wherein the Cys residue is Cys 239, Cys249, Cys 426, Cys 436, Cys 443, Cys 475 , Cys528, Cys566, Cys 623, Cys 651, and/or Cys 719, according to the amino acid position numbering of SEQ ID NO: 21.

Kits for diagnosing diastolic heart failure or diastolic dysfunction in a subject are also provided herein.

### BRIEF DESCRIPTION OF THE DRAWINGS

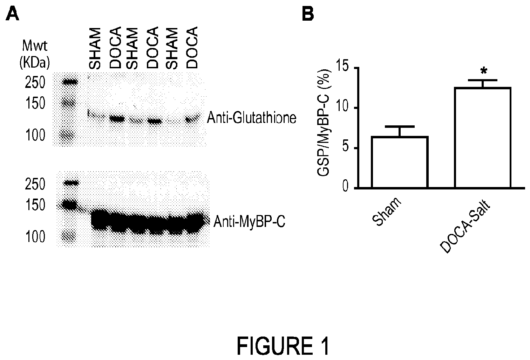

Figure 1. A comparison in the post-translational modifications of the myofilaments from sham and DOCA-salt hearts. A: Western blot analysis demonstrating detection of glutathionylated proteins when comparing myofilament samples from sham and DOCA-salt treated hearts. The blots were also analyzed for myosin binding protein C, indicating that this protein was the major modification. Note that one pair of comparisons was removed from the gel. B: Quantification of the difference between glutathionylated proteins (GSH) normalized to myosin binding protein C (MyBP-C) in sham and DOCA-salt myofilaments. (*p < 0.05, n=3).

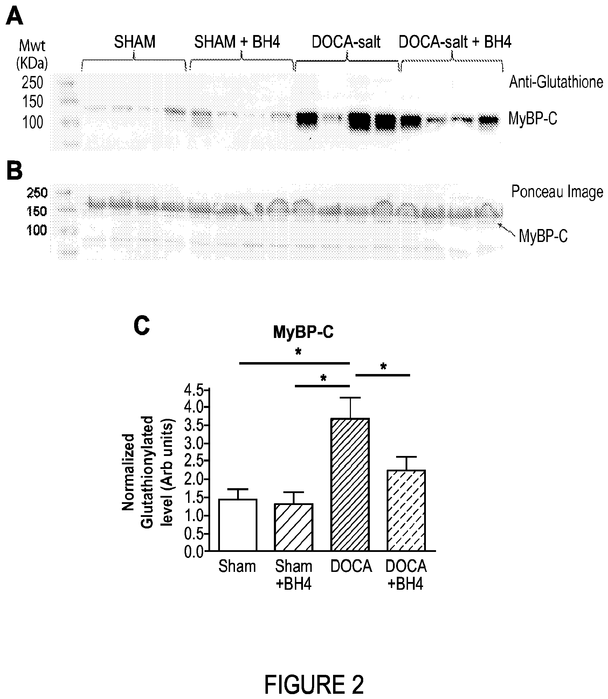

Figure 2. Glutathionylation levels of MyBP-C. (A) Representative Anti-Glutathione gel. (B) Representative Ponceau Image. (C) MyBP-C glutathionylation level normalized to total lane. Band densitometry data were represented mean ± SEM. N=8 mice per group. Data were statistically analyzed using JMP statistical software by two-way ANOVA followed by Student's ttest. *P < 0.05

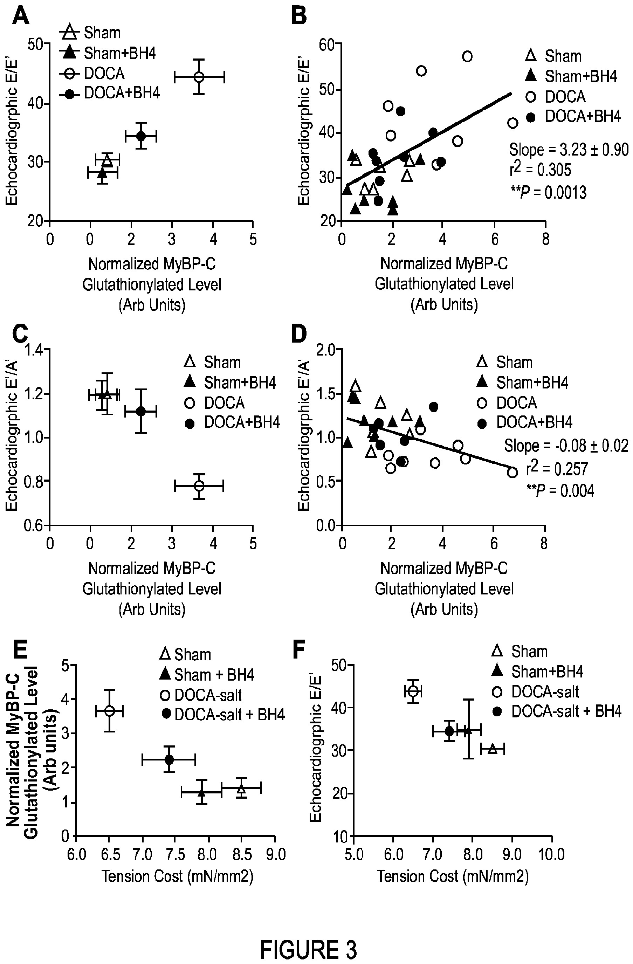

Figure 3A-3F. Relationship between MyBP-C glutathionylation, diastolic dysfunction, and tension cost. (A-B) Echocardiographic parameter, E/E' ratio was positively correlated with normalized MyBP-C glutathionylation level. (C-D) Echocardiographic parameter, E'/A' ratio was negatively correlated with normalized MyBP-C glutathionylation level. Tension cost vs. normalized MyBP-C glutathionylation level (E) and Tension cost vs. echocardiographic E/E' (F) was negatively correlated. N=7-8 mice per group. * indicates linear regression **P< 0.01.

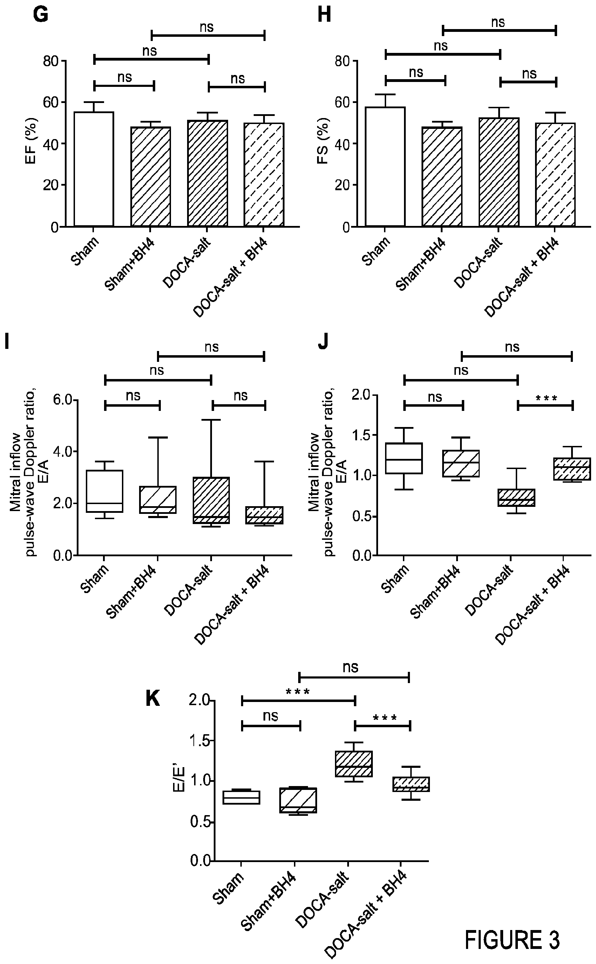

Figure 3G-3M. Thoracic echocardiographic parameters in WT and DOCA-salt mice treated with or without BH4. (G) ejection fraction (%, EF) and (H) fractional shortening (%, FS) were deteremined in short axis M-mode view. (I) mitral inflow pulse-wave Doppler ratio (E/A) (J) mitral tissue Doppler ratio, E'/A' (K) E/E' (L &M) representative images from apical four chamber view of pulse wave (L) and TDI (M). Data was represented mean ± SEM. N=7-9 per group. * indicates Student's t-test compared indicated groups, *P < 0.05' **P <0.01' ***P < 0.001. ns, not significant.

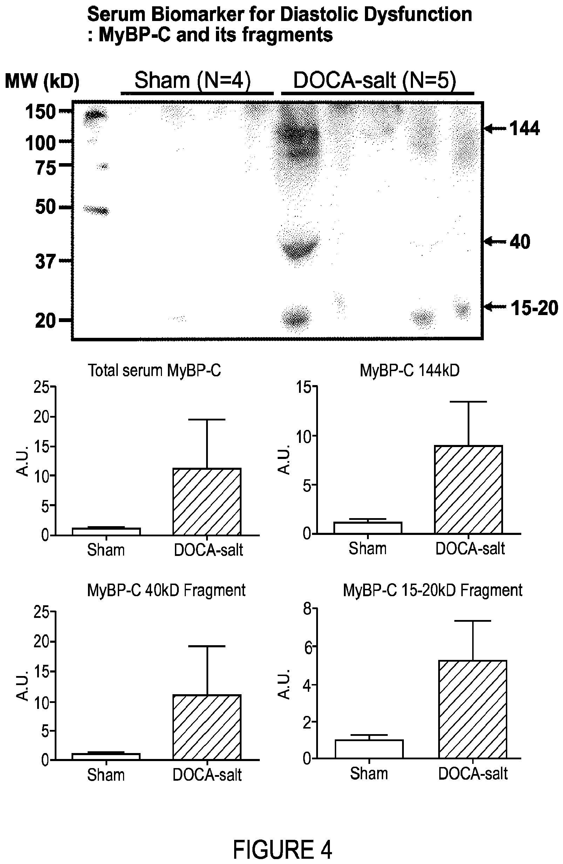

Figure 4 represents a collection of images that depict serum MyBP-C isoforms and fragments as a biomarker for DD. (upper) Western blotting was detected with cardiac MyBP-C Ab. (lower) Densitometry result. Data represents mean ± SEM. N=3-4. P <0.05 vs. Sham, Student *t*-test

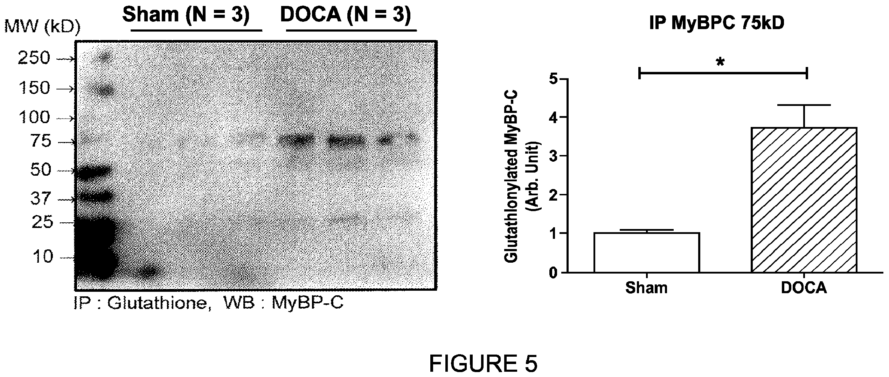

Figure 5 (left panel) is a MyBP-C Western blot of glutathione immunoprecipitates from plasma samples obtained from DOCA-salt mice or sham control mice. Figure 5 (right panel) is a graph of the quantitation of the bands in the Western blot of the left panel. Data are represented as mean ± SE<. P<0.05 in *Student's unpaired t test.*

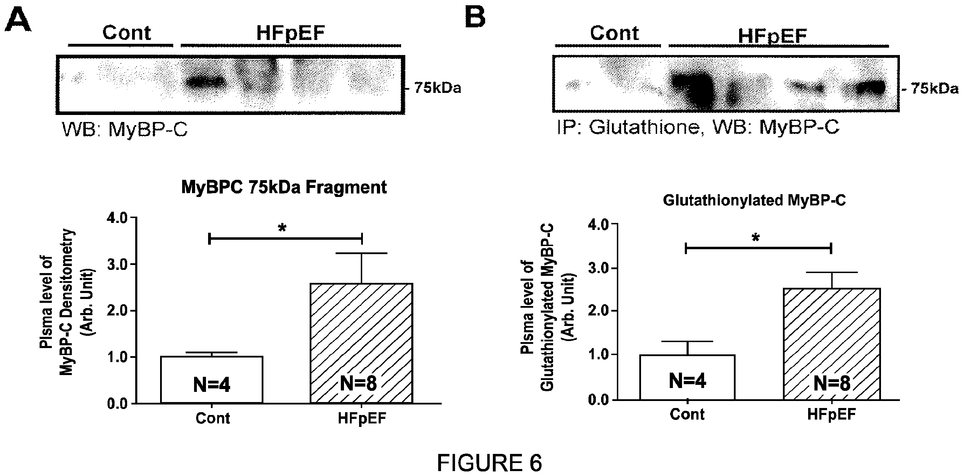

Figure 6 (top left panel) is a MyBP-C Western blot of the 75 kDa bands of plasma samples obtained from control patients or heart failure patients with preserved ejection fraction (HFpEF; diastolic heart failure) and (bottom left panel) is a graph of the quantitation of the bands in the Western blot of the top left panel.

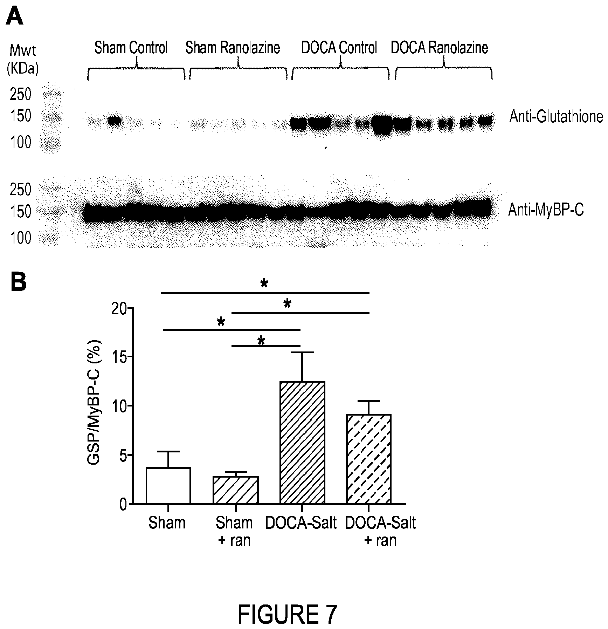

Figures 7A and 7B: Representative gels showing changes in S-glutathionylation of myosin binding protein-C (A). Comparison of myosin binding protein C (MyBP-C) S-glutathionylation in hearts from DOCA-salt or sham mice after or without chronic adminstration of ranolazine (B). Values are given as means ± SEM for 5 determinations.

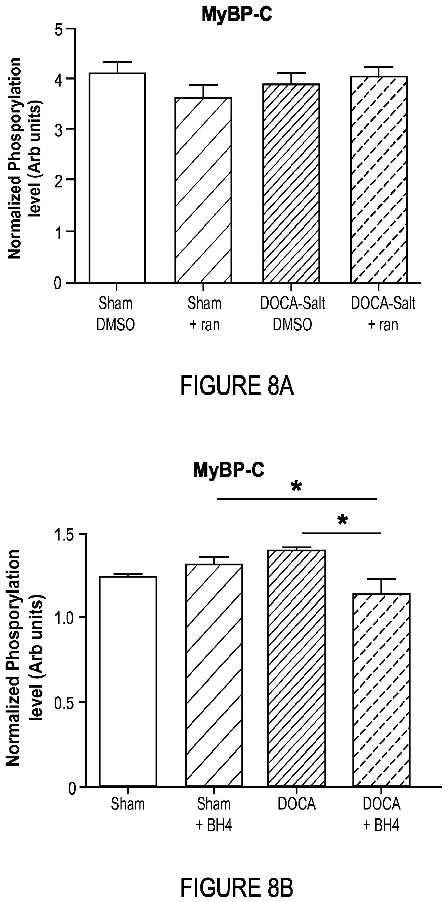

Figure 8A: Representative comparisons of the phosphorylation of the myofilaments from sham and DOCA-salt hearts. A: Comparison of myosin binding protein C (MyBP-C) phosphorylations in fiber bundles from sham and DOCA-salt hearts. There were no significant differences. Values are given as means ± SEM for 6-9 determinations.
Figure 8B: Phosphorylation levels of MyBP-C in myofilament as assessed by ProQ.

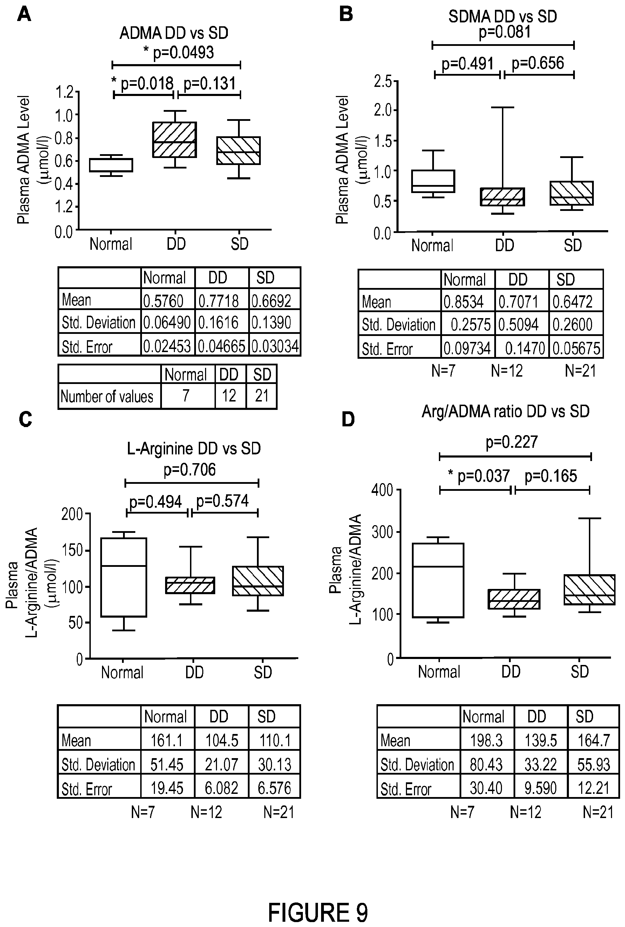

Figures 9A to 9D: Human plasma ADMA levels from patients of diastolic dysfunction (DD) and systolic dysfunction (SD) compared to control group. Figure 9A, ADMA; Figure 9B, SDMS; Figure 9C, Arginine; and Figure 9D, Arginine/ADMA ratio. Data were represented meant SEM. Significance defined with unpaired Student's t-test in each group. (*P<0.05, **P<0.01).

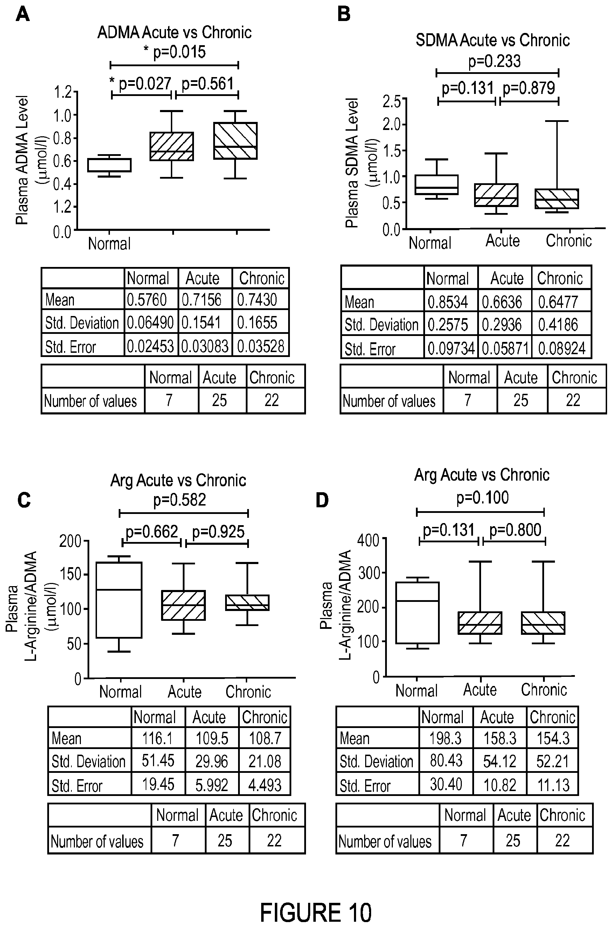

Figures 10A to 10D: Human plasma levels of ADMA (Figure 10A), SDMA (Figure 10B), L-Arginine (Figure 10C), and ratio of [L-Arginine/ADMA] (Figure 10D) from acute and chronic HF patient groups.

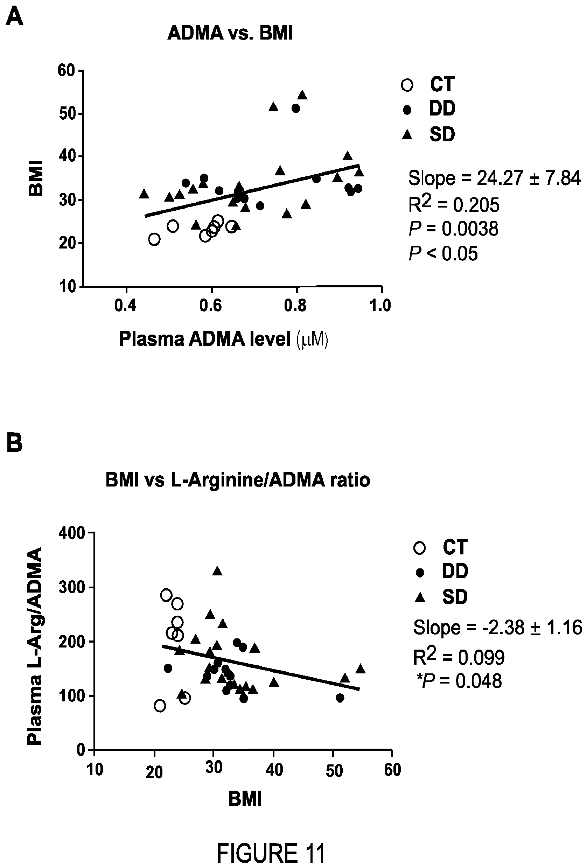

Figure 11A: A gragph of the correlation between human plasma levels of ADMA to BMI of patients (Slope = 24.27 ± 7.84, R² = 0.205, *P* = 0.0038).
Figure 11B: A graph of the correlation between BMI and the ration of L-arginine/ADMA (Slope = -2.38 ± 1.16, R² = 0.099, *P* = 0.048).

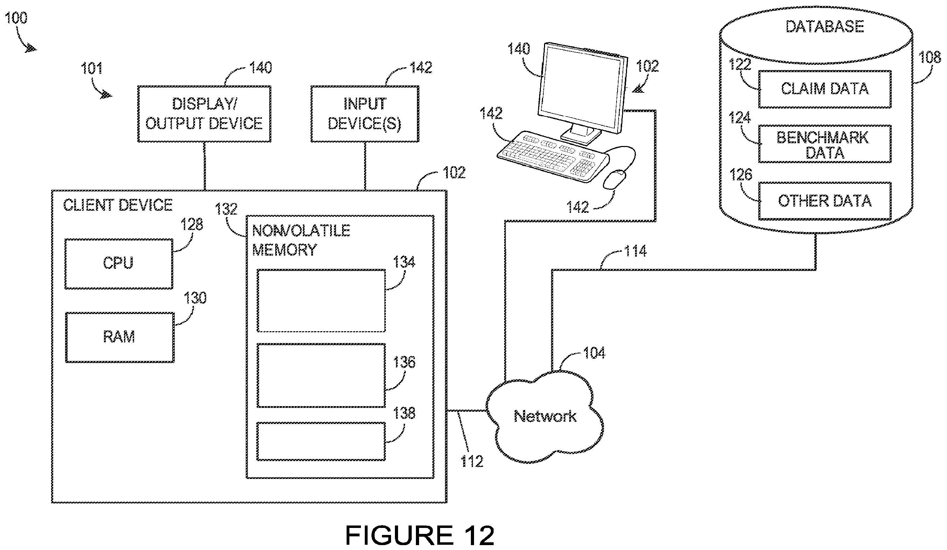

Figure 12 is a schematic of an exemplary embodiment 101 of a system 100 for assessing a subject's need for an implanted cardiac defibrillator (ICD).

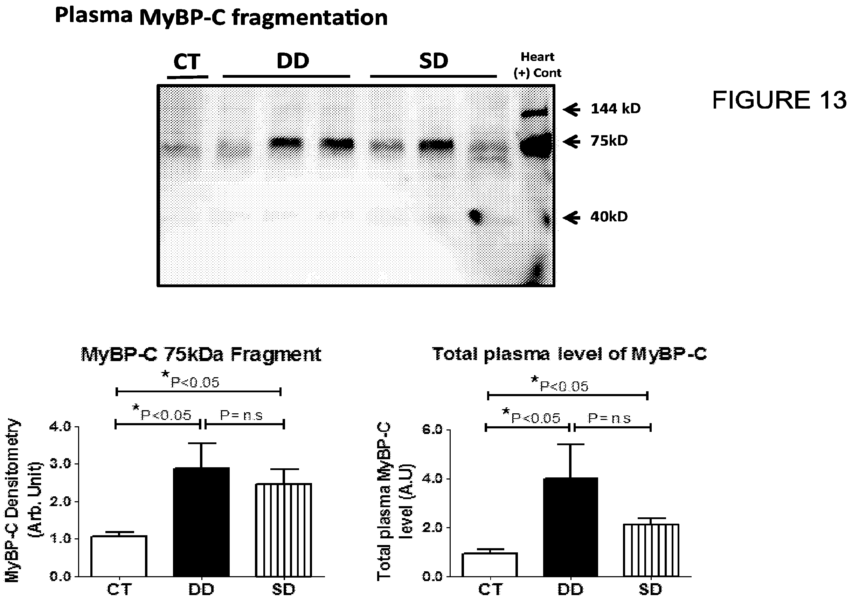

Figure 13 (top panel) is a MyBP-C Western blot of plasma obtained from control patients, diastolic dysfunction patients, and systolic dysfunction patients. The data of the Western blot was quantified and plotted in the graphs of the bottom panel: 75 kDa fragment of MyBP-C (bottom left panel) and total plasma levels of MyBP-C (bottom right panel).

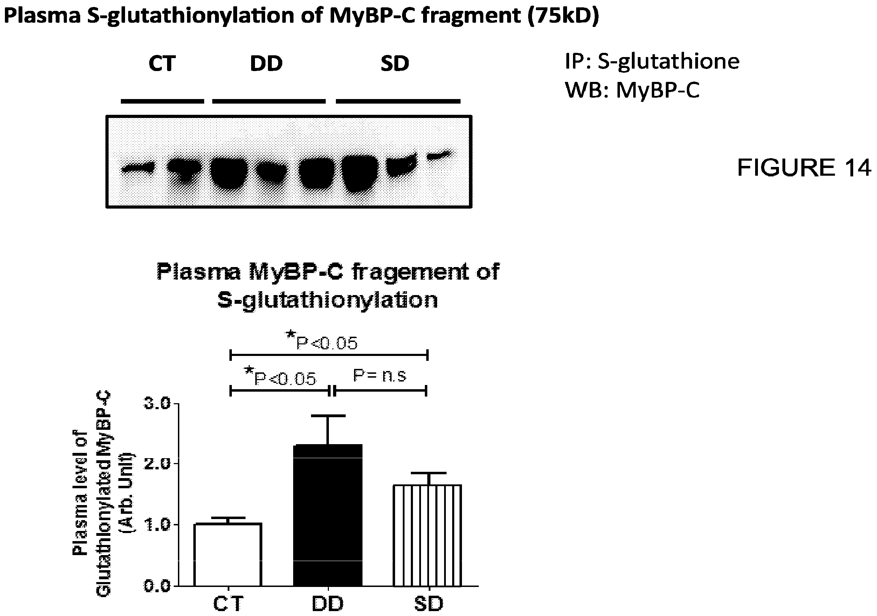

Figure 14 (top panel) is a MyBP-C Western blot (75 kDa MW shown) of S-glutathione immunoprecipitates from plasma obtained from control patients, diastolic dysfunction patients, and systolic dysfunction patients. The data of the Western blot was quantified and plotted in the graph at the bottom.

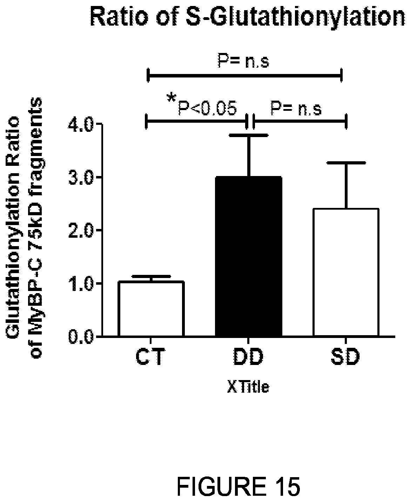

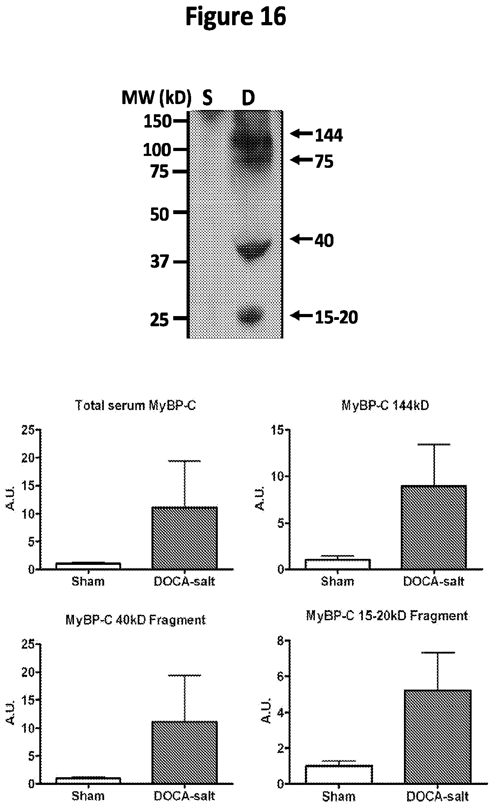

Figure 15 Ratio of S-glutathionylated MyBP-C fragment. 75kD, and protein amount of MyBP-C 75 kD from human plasma of DD and SD patients. Immunoprecipitated data of S-glutathionylation and MyBP-C were normalized with immunoblotting data detecting MyBP-C, 75kD. Data were represented mean ± SEM. N=8-22. One way ANOVA with Bonferroni's posthoc test were defined the significance. (*P<0.05).

### DETAILED DESCRIPTION

### Heart Failure

Heart failure (HF) is a condition in which the heart can no longer pump enough blood to the rest of the body. In some embodiments, the signs and symptoms of heart failure include dyspnea (e.g., orthopnea, paroxysmal nocturnal dyspnea), coughing, cardiac asthma, wheezing, dizziness, confusion, cool extremities at rest, chronic venous congestion, ankle swelling, peripheral edema or anasarca, nocturia, ascites, heptomegaly, jaundice, coagulopathy, fatigue, exercise intolerance, jugular venous distension, pulmonary rales, peripheral edema, pulmonary vascular redistribution, interstitial edema, pleural effusions, or a combination thereof. In some embodiments, the signs and symptoms of heart failure include dyspnea (e.g., orthopnea, paroxysmal nocturnal dyspnea), fatigue, exercise intolerance, jugular venous distension, pulmonary rales, peripheral edema, pulmonary vascular redistribution, interstitial edema, pleural effusions, or a combination thereof. In some embodiments, the symptom of heart failure is one of the symptoms listed in the following table, which provides a basis for classification of heart failure according to the New York Heart Association (NYHA).

| **NYHA Class** | **Symptoms** |
|---|---|
| I | No symptoms and no limitation in ordinary physical activity, e.g. shortness of breath when walking, climbing stairs etc. |
| II | Mild symptoms (mild shortness of breath and/or angina) and slight limitation during ordinary activity. |
| III | Marked limitation in activity due to symptoms, even during less-than-ordinary activity, e.g. |
| | walking short distances (20-100 m). |
| | Comfortable only at rest. |
| IV | Severe limitations. Experiences symptoms even while **at rest.** Mostly bedbound patients. |

Patients presenting with signs and/or symptoms of heart failure may be suffering from systolic dysfunction, diastolic dysfunction, or a combination of the two. Successful treatment of patients suffering from heart failure depends on the type of cardiac dysfunction present, since the treatment of diastolic heart failure is very different from the treatments used for patients suffering from systolic heart failure, e.g., systolic heart failure in the absence of diastolic dysfunction.

### Diastolic dysfunction

As used herein, the term "diastolic dysfunction" refers to a condition in which abnormalities in mechanical function are present during diastole. Diastolic dysfunction can occur in the presence or absence of heart failure and can co-exist with or without abnormalities in systolic function (Zile et al., JACC 41: 1519-1522 (2003)). Diastolic dysfunction in the absence of systolic dysfunction may be referred to as "diastolic dysfunction with preserved ejection fraction." As used herein, the term "preserved ejection fraction" refers to a left ventricular ejection fraction which is greater than or about 45%, e.g., greater than or about 50%. In some aspects, the preserved ejection fraction is one which is greater than or about 50%. In some embodiments, the diastolic dysfunction is an early diastolic dysfunction. As used herein, the term "early diastolic dysfunction" refers to a medical condition in which ventricle filling is impaired as evidenced by the ratio of the peak velocities of blood across the mitral valve in diastole in early filling, the E wave to that during atrial contraction, the A wave, (E/A ratio) ≤ 1 and peak early (E') and late (A') mitral annular velocities recorded by conventional pulsed wave Doppler method also ≤ 1 (Vasan et al., J Am Coll Cardiol 26:1565-1574 (1995); Xie et al., J Am Coll Cardiol 24:132-139 (1994); Moller et al., J Am Coll Cardiol 35:363-370 (2000)).

Diastolic heart failure (DHF) refers to heart failure caused by or linked to diastolic dysfunction. DHF is the clinical manifestation of diastolic dysfunction.

### Systolic dysfunction

In simple terms, systolic dysfunction is a condition in which the pump function or contraction of the heart (i.e., systole), fails. Systolic dysfunction may be characterized by a decreased or reduced ejection fraction, e.g., an ejection fraction which is less than 45%, and an increased ventricular end-diastolic pressure and volume. In some aspects, the strength of ventricular contraction is weakened and insufficient for creating an appropriate stroke volume, resulting in less cardiac output. Systolic dysfunction may occur in the presence or absence of diastolic dysfunction.

Systolic heart failure (SHF) refers to heart failure caused by or linked to systolic dysfunction.

### Methods Relating to Myosin Binding Protein-C (MyBP-C)

The invention provides a method of diagnosing diastolic dysfunction or diastolic heart failure in a subject. In exemplary embodiments, the method comprises determining a level of a myosin binding protein-C (MyBP-C), or a post-translationally modified form thereof, or a fragment thereof, in a biological sample obtained from the subject. In exemplary aspects, the method further comprises diagnosing diastolic dysfunction or diastolic heart failure when the level of MyBP-C, or the post-translationally modified form thereof, or the fragment thereof, is increased, relative to a control level. In exemplary aspects, the method comprises determining a level of an S-glutathionylated MyBP-C or an S-glutathionylated fragment of MyBP-C. In exemplary aspects, the method comprises determining a level of an S-glutathionylated, 75 kDa fragment of MyBP-C.

In exemplary aspects, the inventive method is a method of diagnosing diastolic dysfunction in a subject, and the method comprises determining a level of a myosin binding protein-C (MyBP-C), or a post-translationally modified form thereof, or a fragment thereof, in a biological sample obtained from the subject. In exemplary aspects of this inventive method, the method further comprises diagnosing diastolic dysfunction, when the level of MyBP-C, or the post-translationally modified form thereof, or the fragment thereof, is increased, relative to a control level. In exemplary aspects, the method comprises determining a level of an S-glutathionylated MyBP-C or an S-glutathionylated fragment of MyBP-C. In exemplary aspects, the method comprises determining a level of an S-glutathionylated, 75 kDa fragment of MyBP-C.

In alternative exemplary aspects, the inventive method is a method of diagnosing diastolic heart failure in a subject, and the method comprises determining a level of a myosin binding protein-C (MyBP-C), or a post-translationally modified form thereof, or a fragment thereof, in a biological sample obtained from the subject. In exemplary aspects of this inventive method, the method further comprises diagnosing diastolic heart failure, when the level of MyBP-C, or the post-translationally modified form thereof, or the fragment thereof, is increased, relative to a control level. In exemplary aspects, the method comprises determining a level of an S-glutathionylated MyBP-C or an S-glutathionylated fragment of MyBP-C. In exemplary aspects, the method comprises determining a level of an S-glutathionylated, 75 kDa fragment of MyBP-C.

The invention also provides a method of differentiating between diastolic dysfunction and systolic dysfunction in a subject suffering from heart failure. In exemplary embodiments, the method comprises determining a level of a myosin binding protein-C (MyBP-C), or a post-translationally modified form thereof, or a fragment thereof, in a biological sample obtained from the subject. In exemplary aspects, the method differentiates diastolic dysfunction from systolic dysfunction, and the method identifies the subject as a subject suffering from diastolic dysfunction, when the level of MyBP-C, or the post-translationally modified form thereof, or the fragment thereof, is increased, relative to a control level. In exemplary aspects, the method comprises determining a level of an S-glutathionylated MyBP-C or an S-glutathionylated fragment of MyBP-C, e.g., an S-glutathionylated, 75 kDa fragment of MyBP-C.

The invention further provides a method of characterizing heart failure as diastolic heart failure or systolic heart failure in a subject. In exemplary embodiments, the method comprises determining a level of a myosin binding protein-C (MyBP-C), or a post-translationally modified form thereof, or a fragment thereof, in a biological sample obtained from the subject. In exemplary aspects, the method comprises characterizing the heart failure in the subject as diastolic heart failure, when the level of MyBP-C, or the post-translationally modified form thereof, or the fragment thereof, is increased, relative to a control level. In exemplary aspects, the method comprises characterizing the heart failure in the subject as systolic heart failure when the level of MyBP-C, or the post-translationally modified form thereof, or the fragment thereof, is increased, relative to a level from a subject not suffering from heart failure and decreased relative to a level from a subject suffering from diastolic heart failure. In exemplary aspects, the method comprises determining a level of an S-glutathionylated MyBP-C or an S-glutathionylated fragment of MyBP-C, e.g., an S-glutathionylated, 75 kDa fragment of MyBP-C.

The invention furthermore provides a method of determining a subject's need for treatment of diastolic dysfunction or diastolic heart failure. In exemplary embodiments, the method comprises determining a level of a myosin binding protein-C (MyBP-C), or a post-translationally modified form thereof, or a fragment thereof, in a biological sample obtained from the subject. When the level of MyBP-C, or the post-translationally modified form thereof, or the fragment thereof, is increased, relative to a control level, the subject is determined to need treatment for diastolic heart failure. In exemplary aspects, the method comprises determining a level of an S-glutathionylated MyBP-C or an S-glutathionylated fragment of MyBP-C, e.g., an S-glutathionylated, 75 kDa fragment of MyBP-C.

The invention furthermore provides a method of determining a subject's need for treatment of diastolic dysfunction. In exemplary embodiments, the method comprises determining a level of a myosin binding protein-C (MyBP-C), or a post-translationally modified form thereof, or a fragment thereof, in a biological sample obtained from the subject. When the level of MyBP-C, or the post-translationally modified form thereof, or the fragment thereof, is increased, relative to a control level, the subject is determined to need treatment for diastolic dysfunction. In exemplary aspects, the method comprises determining a level of an S-glutathionylated MyBP-C or an S-glutathionylated fragment of MyBP-C, e.g., an S-glutathionylated, 75 kDa fragment of MyBP-C.

The invention moreover provides a method of identifying a patient at risk for diastolic dysfunction or diastolic heart failure. In exemplary embodiments, the method comprises determining a level of a myosin binding protein-C (MyBP-C), or a post-translationally modified form thereof, or a fragment thereof, in a biological sample obtained from the subject. When the level of MyBP-C, or the post-translationally modified form thereof, or the fragment thereof, is increased, relative to a control level, the subject is identified as a patient at risk for diastolic dysfunction or diastolic heart failure. In exemplary aspects, the method comprises determining a level of an S-glutathionylated MyBP-C or an S-glutathionylated fragment of MyBP-C, e.g., an S-glutathionylated, 75 kDa fragment of MyBP-C.

The invention additionally provides a method of reducing a subject's risk for diastolic dysfunction or diastolic heart failure. In exemplary embodiments, the method comprises (i) determining a level of a myosin binding protein-C (MyBP-C), or a post-translationally modified form thereof, or a fragment thereof, in a biological sample obtained from the subject and (ii) administering to the subject a therapeutic agent for the treatment of diastolic dysfunction or diastolic heart failure, when the level of MyBP-C, or a post-translationally modified form or fragment thereof, is increased, relative to a control level. In exemplary aspects, the method comprises determining a level of an S-glutathionylated MyBP-C or an S-glutathionylated fragment of MyBP-C, e.g., an S-glutathionylated, 75 kDa fragment of MyBP-C.

The invention provides a method of monitoring a subject's risk for diastolic dysfunction or diastolic heart failure. In exemplary aspects, the method comprises (a) determining a first level of a myosin binding protein-C (MyBP-C), or a post-translationally modified form thereof or a fragment thereof in a biological sample obtained from the subject at a first timepoint; and (b) determining a second level the MyBP-C, or the post-translationally modified form thereof or the fragment thereof, in a biological sample obtained from the subject at a second timepoint which occurs after the first timepoint, wherein the subject's risk for diastolic dysfunction or diastolic heart failure is decreased, when the second level is less than the first level. In exemplary aspects, the post-translationally modified form of MyBP-C is an S-glutathionylated MyBP-C or an S-glutathionylated fragment of MyBP-C, e.g., an S-glutathionylated, 75 kDa fragment of MyBP-C.

Also disclosed is a method of screening a compound for an ability to treat diastolic dysfunction or diastolic heart failure in a subject. In exemplary aspects, the method comprises (a) determining a first level of a myosin binding protein-C (MyBP-C), or a post-translationally modified form thereof or a fragment thereof, in a biological sample obtained from the subject prior to administration of the compound; and (b) determining a second level of the myosin binding protein-C (MyBP-C), or the post-translationally modified form thereof or the fragment thereof, in a biological sample obtained from the subject after administration of the compound, wherein the compound is able to treat diastolic dysfunction or diastolic heart failure, when the second level is less than the first level. In exemplary aspects, the post-translationally modified form of MyBP-C is an S-glutathionylated MyBP-C or an S-glutathionylated fragment of MyBP-C, e.g., an S-glutathionylated, 75 kDa fragment of MyBP-C.

The invention also provides a method of determining efficacy of a treatment of diastolic dysfunction or diastolic heart failure in a subject. In exemplary aspects, the method comprises (a) determining a first level of a myosin binding protein-C (MyBP-C), or a post-translationally modified form thereof or a fragment thereof in a biological sample obtained from the subject before treatment; and (b) determining a second level the myosin binding protein-C (MyBP-C), or the post-translationally modified form thereof or the fragment thereof, in a biological sample obtained from the subject after treatment, wherein the treatment is effective for treating diastolic dysfunction or diastolic heart failure in the subject, when the second level is less than the first level. In exemplary aspects, the post-translationally modified form of MyBP-C is an S-glutathionylated MyBP-C or an S-glutathionylated fragment of MyBP-C, e.g., an S-glutathionylated, 75 kDa fragment of MyBP-C.

The invention moreover provides a method of treating a subject suffering from heart failure. In exemplary embodiments, the method comprises (a) determining a level of a myosin binding protein-C (MyBP-C), or a post-translationally modified form or fragment thereof, in a biological sample obtained from the subject; and (b) administering to the subject a therapeutic agent for the treatment of diastolic dysfunction or diastolic heart failure, when the level of MyBP-C, or a post-translationally modified form or fragment thereof, is increased, relative to a control level. In exemplary aspects, the post-translationally modified form of MyBP-C is an S-glutathionylated MyBP-C or an S-glutathionylated fragment of MyBP-C, e.g., an S-glutathionylated, 75 kDa fragment of MyBP-C.

The invention also provides a method of treating a subject who has been assayed for a level of a myosin binding protein-C (MyBP-C), or a post-translationally modified form or fragment thereof. In exemplary embodiments, the method comprises administering to the subject a therapeutic agent for the treatment of diastolic dysfunction or diastolic heart failure, when the level of MyBP-C, or a post-translationally modified form or fragment thereof, is increased, relative to a control level. In exemplary aspects, the post-translationally modified form of MyBP-C is an S-glutathionylated MyBP-C or an S-glutathionylated fragment of MyBP-C, e.g., an S-glutathionylated, 75 kDa fragment of MyBP-C.

With regard to the inventive methods of treatment, suitable therapeutic agents for the treatment of diastolic dysfunction or diastolic heart failure known in the art some of which are described herein may be used. See the subsection entitled *Therapeutic agents for the treatment of diastolic dysfunction or diastolic heart failure*. In exemplary aspects, the therapeutic is BH₄ or ranolazine.

As used herein, the term "myosin binding protein-C" or "MyBP-C" refers to a cardiac-type myosin binding protein (cMyBP-C), which is associated with the thick filaments of vertebrate hearts and is expressed exclusively in cardiac muscle. cMyBP-C is different from the fast-skeletal MyBP-C and the slow-skeletal MyBP-C, which are exclusively expressed in skeletal muscle. In the cardiac sarcomere, cMyBP-C is localized within the inner two-thirds of the A-band, i.e., in the C-zone. cMyBP-C belongs to the intracellular immunoglobulin (Ig) superfamily of proteins. Like other sarcomeric proteins, such as titin, myosin, and M-protein, it is composed of repeating domains of Ig and fibronectin type-3. (Barefield and Sadayappan, J Molec Cell Cardiol 48: 866-875 (2010)). The full-length amino acid sequence of human cMyBP-C, which is publicly available at the National Center for Biotechnology Information (NCBI) website as Reference Sequence NP_000247.2, and is also set forth herein as SEQ ID NO: 21, encodes a protein of 1274 amino acids having a molecular weight of approximately 140 or 144 kDa.

Under certain conditions, cMyBP-C undergoes post-translational modifications, e.g., phosphorylation, S-glutathionylation. Without being bound to any particular theory, S-glutathionylation of cMyBP-C occurs under conditions of stress, e.g., oxidative stress. In exemplary aspects, MyBP-C is S-glutathionylated at one or more Cys residues within the N-terminal portion of the protein, e.g., the first 720 amino acids of SEQ ID NO :21. In exemplary aspects, MyBP-C is S-glutathionylated at one or more of Cys 239, Cys249, Cys 426, Cys 436, Cys 443, Cys 475 , Cys528, Cys566, Cys 623, Cys 651, and/or Cys 719, according to the amino acid position numbering of SEQ ID NO: 21. In exemplary aspects, S-glutathionylation of cMyBP-C occurs at Cys475. In exemplary aspects, S-glutathionylation of cMyBP-C occurs at Cys623. In exemplary aspects, S-glutathionylation of cMyBP-C occurs at Cys651. In exemplary aspects, S-glutathionylation of cMyBP-C occurs at Cys475 and Cys 623 or at Cys475 and Cys 651. In exemplary aspects, S-glutathionylation of cMyBP-C occurs at Cys 623 and Cys 651. In exemplary aspects, S-glutathionylation of cMyBP-C occurs at Cys475, Cys 623, and Cys 651.

Under certain conditions, cMyBP-C degrades or is cleaved into fragments of a variety of sizes. Without being bound to any particular theory, under conditions of stress, e.g., oxidative stress, cMyBP-C degrades or is cleaved into fragments, and the fragments are secreted from cells (e.g., cardiac cells) into the blood plasma. The cMyBP-C fragment in exemplary aspects has a molecular weight of about 70 kDa to about 80 kDa. The cMyBP-C fragment in exemplary aspects has a molecular weight of about 75 kDa or about 79 kDa. In other exemplary aspects, the cMyBP-C fragment has a molecular weight of about 40 kDa. In other exemplary aspects, the cMyBP-C fragment has a molecular weight of about 25 kDa. In other exemplary aspects, the cMyBP-C fragment has a molecular weight of about 15-20 kDa. In exemplary aspects, any one of the above fragments of MyBP-C is S-glutathionylated.

For purposes of the inventive methods, when the method comprises determining a level of a myosin binding protein-C (MyBP-C), or a post-translationally modified form thereof, or a fragment thereof, the method in exemplary aspects comprises determining the level of full length or intact MyBP-C or comprises determining a MyBP-C having a molecular weight of about 140 kDa or 144 kDa. Alternatively or additionally, the method in exemplary aspects comprises determining a post-translationally modified form of MyBP-C or a post-translationally modified form of a MyBP-C fragment. In exemplary aspects, the method comprises determining a level of S-glutathionylated MyBP-C or an S-glutathionylated fragment of MyBP-C. In alternative or additional aspects, the method comprises determining a level of a MyBP-C fragment, including, but not limited to a MyBP-C fragment having a molecular weight of about 75 kDa or 79 kDa, a MyBP-C fragment having a molecular weight of about 40 kDa, a MyBP-C fragment having a molecular weight of about 25 kDa, a MyBP-C fragment having a molecular weight of about 15-20 kDa, or a combination thereof. In alternative or additional aspects, the method comprises determining a level of an S-glutathionylated MyBP-C fragment. In exemplary aspects, the method comprises determining (a) a level of full length or intact MyBP-C or a MyBP-C having a molecular weight of about 140 kDa or 144 kDa, (b) a level of a post-translationally modified form of MyBP-C, e.g., an S-glutathionylated MyBP-C, (c) a level of a MyBP-C fragment, optionally, wherein the fragment is S-glutathionylated, or (d) a combination thereof

In exemplary aspects, the level of MyBP-C, or a post-translationally modified form thereof, or a fragment thereof, is an absolute level. In alternative aspects, the level of MyBP-C, or a post-translationally modified form thereof, or a fragment thereof, is a normalized or relative level. In exemplary aspects, the level of MyBP-C, or a post-translationally modified form thereof, or a fragment thereof, is relative to the level of MyBP-C, or a post-translationally modified form thereof, or a fragment thereof, of a control sample, wherein the control sample is obtained from a subject known to not have diastolic dysfunction or diastolic heart failure. In exemplary aspects, the level of MyBP-C, or a post-translationally modified form thereof, or a fragment thereof, of the control sample is set to 1, and the level of the subject (the test sample, the unknown sample, the non-control sample) is expressed relative to the control sample. An increased level in such aspects is determined when the the relative level is greater than 1, e.g., when the relative level is greater than one + one standard deviation.

In exemplary aspects, the level of MyBP-C, or a post-translationally modified form thereof, or a fragment thereof, is normalized to another protein, e.g., a gene product of a housekeeping gene. In alternative exemplary aspects, the level of MyBP-C is a level of MyBP-C normalized to a level of one or more fragments of MyBP-C. In exemplary aspects, the level of MyBP-C is a ratio of [concentration of a 144 kDa MyBP-C]:[sum concentration of MyBP-C fragments]. In exemplary aspects, the level of MyBP-C or the level of MyBP-C fragment is normalized to a total level of MyBP-C in the sample. As used herein, the term "total level of MyBP-C" refers to the sum of the level of post-translationally modified, full length MyBP-C, the level of unmodified, full length MyBP-C, the sum level of all post-translationally modified MyBP-C fragments, and the sum level of all unmodified MyBP-C fragments. In exemplary aspects, the level of post-translationally modified form of MyBP-C is a level of MyBP-C comprising a post-translational modification normalized to a level of MyBP-C not comprising the post-translational modification or is a level of MyBP-C comprising a post-translational modification normalized to a total level of MyBP-C. In yet other exemplary aspects, the level determined in the methods of the invention is a ratio of the level of S-glutathionylated, 75 kDa fragments of MyBP-C to the total level of S-glutathionylated MyBP-C intact proteins and S-glutathionylated MyBP-C fragments.

### Methods Relating to Dimethylarginine

Here disclosed is a method of diagnosing diastolic dysfunction or diastolic heart failure in a subject, wherein, in exemplary embodiments, the method comprises determining a level of asymmetric dimethylarginine (ADMA) and a level of either or both of (a) symmetric dimethyl arginine (SDMA) and/or (b) L-Arginine, in a biological sample obtained from the subject. In exemplary aspects, the method further comprises diagnosing diastolic dysfunction or diastolic heart failure, when the level of ADMA is increased relative to a control level and the level of SDMA is substantially unchanged relative to a control level, or when the ratio of L-Arginine to ADMA is decreased relative to a control ratio. In specific aspects, diastolic dysfunction or diastolic heart failure is diagnosed, when the level of ADMA is above or about 0.65 µM and the level of SDMA is substantially unchanged relative to a control level, or when the ratio of L-Arginine to ADMA is below 170.

In exemplary aspects, said method is a method of diagnosing diastolic dysfunction in a subject, and the method comprises determining a level of asymmetric dimethylarginine (ADMA) and a level of either or both of (a) symmetric dimethyl arginine (SDMA) and/or (b) L-Arginine, in a biological sample obtained from the subject. In exemplary aspects, the method further comprises diagnosing diastolic dysfunction, when the level of ADMA is increased relative to a control level and the level of SDMA is substantially unchanged relative to a control level, or when the ratio of L-Arginine to ADMA is decreased relative to a control ratio. In specific aspects, diastolic dysfunction is diagnosed, when the level of ADMA is above or about 0.65 µM and the level of SDMA is substantially unchanged relative to a control level, or when the ratio of L-Arginine to ADMA is below 170.

In alternative exemplary aspects, the inventive method is a method of diagnosing diastolic heart failure in a subject, and the method comprises determining a level of asymmetric dimethylarginine (ADMA) and a level of either or both of (a) symmetric dimethyl arginine (SDMA) and/or (b) L-Arginine, in a biological sample obtained from the subject. In exemplary aspects, the method further comprises diagnosing diastolic heart failure, when the level of ADMA is increased relative to a control level and the level of SDMA is substantially unchanged relative to a control level, or when the ratio of L-Arginine to ADMA is decreased relative to a control ratio. In specific aspects, diastolic heart failure is diagnosed, when the level of ADMA is above or about 0.65 µM and the level of SDMA is substantially unchanged relative to a control level, or when the ratio of L-Arginine to ADMA is below 170.

Also disclosed is a method of differentiating between diastolic dysfunction and systolic dysfunction in a subject suffering from heart failure, wherein, in exemplary embodiments, the method comprises determining a level of asymmetric dimethylarginine (ADMA) and a level of either or both of (a) symmetric dimethyl arginine (SDMA) and/or (b) L-Arginine, in a biological sample obtained from the subject. In exemplary aspects, the method differentiates diastolic dysfunction from systolic dysfunction, and the method identifies the subject as a subject suffering from diastolic dysfunction, when the level of ADMA is increased relative to a control level and the level of SDMA is substantially unchanged relative to a control level, or when the ratio of L-Arginine to ADMA is decreased relative to a control ratio. In specific aspects, the subject is identified as suffering from diastolic dysfunction, when the level of ADMA is above or about 0.65 µM and the level of SDMA is substantially unchanged relative to a control level, or when the ratio of L-Arginine to ADMA is below 170.

Also disclosed herein is a method of characterizing heart failure as diastolic heart failure or systolic heart failure in a subject, wherein, in exemplary embodiments, the method comprises determining a level of asymmetric dimethylarginine (ADMA) and a level of either or both of (a) symmetric dimethyl arginine (SDMA) and/or (b) L-Arginine, in a biological sample obtained from the subject. In exemplary aspects, the method comprises characterizing the heart failure in the subject as diastolic heart failure, when the level of ADMA is increased relative to a control level and the level of SDMA is substantially unchanged relative to a control level, or when the ratio of L-Arginine to ADMA is decreased relative to a control ratio. In exemplary aspects, the method comprises characterizing the heart failure in the subject as systolic heart failure when the ratio of the level of L-Arginine to the level of ADMA, is not reduced, relative to a level from a subject not suffering from heart failure. In specific aspects, the heart failure is characterized as diastolic heart failure, when the level of ADMA is above or about 0.65 µM and the level of SDMA is substantially unchanged relative to a control level, or when the ratio of L-Arginine to ADMA is below 170.

Disclosed herein is also a method of determining a subject's need for treatment of diastolic heart failure. In exemplary embodiments, the method comprises determining a level of asymmetric dimethylarginine (ADMA) and a level of either or both of (a) symmetric dimethyl arginine (SDMA) and/or (b) L-Arginine, in a biological sample obtained from the subject. When the level of ADMA is increased relative to a control level and the level of SDMA is substantially unchanged relative to a control level, or when the ratio of L-Arginine to ADMA is decreased relative to a control ratio, the subject is determined to need treatment for diastolic heart failure. In specific aspects, the subject is determined to need treatment for diastolic heart failure, when the level of ADMA is above or about 0.65 µM and the level of SDMA is substantially unchanged relative to a control level, or when the ratio of L-Arginine to ADMA is below 170.

Also provided is a method of determining a subject's need for treatment of diastolic dysfunction. In exemplary embodiments, the method comprises determining a level of asymmetric dimethylarginine (ADMA) and a level of either or both of (a) symmetric dimethyl arginine (SDMA) and/or (b) L-Arginine, in a biological sample obtained from the subject. When the level of ADMA is increased relative to a control level and the level of SDMA is substantially unchanged relative to a control level, or when the ratio of L-Arginine to ADMA is decreased relative to a control ratio, the subject is determined to need treatment for diastolic dysfunction. In specific aspects, the subject is determined to need treatment for diastolic dysfunction, when the level of ADMA is above or about 0.65 µM and the level of SDMA is substantially unchanged relative to a control level, or when the ratio of L-Arginine to ADMA is below 170.

Also provided is a method of identifying a patient at risk for diastolic dysfunction or diastolic heart failure. In exemplary embodiments, the method comprises determining a level of asymmetric dimethylarginine (ADMA) and a level of either or both of (a) symmetric dimethyl arginine (SDMA) and/or (b) L-Arginine, in a biological sample obtained from the subject. When the level of ADMA is increased, relative to control level, and the level of SDMA is unchanged, relative to a control level, the subject is identified as a patient at risk for diastolic dysfunction or diastolic heart failure. Alternatively, the subject is identified as a patient at risk for diastolic dysfunction or diastolic heart failure, when the ratio of the level of L-Arginine to the level of ADMA is decreased, relative to a control ratio. In specific aspects, the patient is determined to be at risk for diastolic dysfunction or diastolic heart failure, when the level of ADMA is above or about 0.65 µM and the level of SDMA is substantially unchanged relative to a control level, or when the ratio of L-Arginine to ADMA is below 170.

Here disclosed is also a method of reducing a subject's risk for diastolic dysfunction or diastolic heart failure. In exemplary embodiments, the method comprises (i) determining a level of asymmetric dimethylarginine (ADMA) and a level of either or both of (a) symmetric dimethyl arginine (SDMA) and/or (b) L-Arginine, in a biological sample obtained from the subject and (ii) administering to the subject a therapeutic agent for the treatment of diastolic dysfunction or diastolic heart failure, when the level of ADMA is increased, relative to a control level, and the level of SDMA is unchanged, relative to a control level, or when the ratio of the level of L-Arginine to the level of ADMA is decreased, relative to a control ratio. In specific aspects, the therapeutic agent for the treatment of diastolic dysfunction or diastolic heart failure is administered to the subject, when the level of ADMA is above or about 0.65 µM and the level of SDMA is substantially unchanged relative to a control level, or when the ratio of L-Arginine to ADMA is below 170.

Disclosed herein is also a method of monitoring a subject's risk for diastolic dysfunction or diastolic heart failure. In exemplary aspects, the method comprises (i) determining a first level of asymmetric dimethylarginine (ADMA) and a first level of either or both of (a) symmetric dimethyl arginine (SDMA) and/or (b) L-Arginine, in a biological sample obtained from the subject at a first timepoint; and (ii) determining a second level of asymmetric dimethylarginine (ADMA) and a second level of either or both of (a) symmetric dimethyl arginine (SDMA) and/or (b) L-Arginine, in a biological sample obtained from the subject at a second timepoint which occurs after the first timepoint, wherein the subject's risk for diastolic dysfunction or diastolic heart failure is increased, when the second level of ADMA is greater than the first level of ADMA and the second level of SDMA is substantially unchanged relative to the first level, or the ratio of the second level of L-Arginine to the second level of ADMA is less than the ratio of the first level of L-Arginine to the first level of ADMA.

Also disclosed is a method of screening a compound for an ability to treat diastolic dysfunction or diastolic heart failure in a subject. In exemplary aspects, the method comprises (i) determining a first level of asymmetric dimethylarginine (ADMA) and a first level of either or both of (a) symmetric dimethyl arginine (SDMA) and/or (b) L-Arginine, in a biological sample obtained from the subject before administration of the compound; and (ii) determining a second level of asymmetric dimethylarginine (ADMA) and a second level of either or both of (a) symmetric dimethyl arginine (SDMA) and/or (b) L-Arginine, in a biological sample obtained from the subject after administration of the compound, wherein the compound is able to treat diastolic dysfunction or diastolic heart failure, when the second level of ADMA is less than the first level of ADMA and the second level of SDMA is substantially unchanged from the first level of SDMA, or the ratio of the second level of L-Arginine to the second level of ADMA is more than the ratio of the first level of L-Arginine to the first level of ADMA. In specific aspects, the compound is able to treat diastolic dysfunction or diastolic heart failure, when the first level of ADMA is above or about 0.65 µM and the second level of ADMA is below 0.65 µM, and the the second level of SDMA is substantially unchanged relative to the first level. In specific aspects, the compound is able to treat diastolic dysfunction or diastolic heart failure, when the ratio of the second level of L-Arginine to the second level of ADMA of L-Arginine to ADMA is below 170 and the ratio of the first level of L-Arginine to the first level of ADMA is above 170.

Also disclosed is a method of determining efficacy of a treatment of diastolic dysfunction or diastolic heart failure in a subject. In exemplary aspects, the method comprises (i) determining a first level of asymmetric dimethylarginine (ADMA) and a first level of either or both of (a) symmetric dimethyl arginine (SDMA) and/or (b) L-Arginine, in a biological sample obtained from the subject before treatment; and (ii) determining a second level of asymmetric dimethylarginine (ADMA) and a second level of either or both of (a) symmetric dimethyl arginine (SDMA) and/or (b) L-Arginine, in a biological sample obtained from the subject after treatment, wherein the treatment is effective for treating diastolic dysfunction or diastolic heart failure in the subject, when the second level of ADMA is less than the first level of ADMA and the second level of SDMA is substantially unchanged from the first level of SDMA, or when the ratio of the second level of L-Arginine to the second level of ADMA is greater than the ratio of the first level of L-Arginine to the first level of ADMA. In specific aspects, the compound is able to treat diastolic dysfunction or diastolic heart failure, when the first level of ADMA is above or about 0.65 µM and the second level of ADMA is below 0.65 µM, and the the second level of SDMA is substantially unchanged relative to the first level. In specific aspects, the treatment is effective for treating diastolic dysfunction or diastolic heart failure in the subject, when the first level of ADMA is above or about 0.65 µM and the second level of ADMA is below 0.65 µM, and the the second level of SDMA is substantially unchanged relative to the first level. In specific aspects, the treatment is effective, when the ratio of the second level of L-Arginine to the second level of ADMA of L-Arginine to ADMA is below 170 and the ratio of the first level of L-Arginine to the first level of ADMA is above 170.

Also disclosed is a method of treating a subject suffering from heart failure, wherein, in exemplary embodiments, the method comprises (a) determining a level of a asymmetric dimethylarginine (ADMA) and a level of either or both of (a) symmetric dimethyl arginine (SDMA) and/or (b) L-Arginine, in a biological sample obtained from the subject; and (b) administering to the subject a therapeutic agent for the treatment of diastolic dysfunction or diastolic heart failure, when the level of ADMA is increased relative to a control level and the level of SDMA is substantially unchanged relative to a control level, or when the ratio of L-Arginine to ADMA is decreased relative to a control ratio. In specific aspects, the therapeutic agent is administered to the subject, when the level of ADMA is over 0.65 µM and the level of SMDA is substantially unchanged, relative to a control, or when the the ratio of L-Arginine to ADMA is under 170.

Also disclosed is a method of treating a subject who has been assayed for a level of asymmetric dimethylarginine (ADMA) and a level of either or both of (a) symmetric dimethyl arginine (SDMA) and/or (b) L-Arginine. In exemplary embodiments, the method comprises administering to the subject a therapeutic agent for the treatment of diastolic dysfunction or diastolic heart failure, when the level of ADMA is increased relative to a control level and the level of SDMA is substantially unchanged relative to a control level, or when the ratio of L-Arginine to ADMA is decreased relative to a control ratio.. In specific aspects, the therapeutic agent is administered to the subject, when the level of ADMA is over 0.65 µM and the level of SMDA is substantially unchanged, relative to a control, or when the the ratio of L-Arginine to ADMA is under 170.

With regard to the inventive methods of treatment, suitable therapeutic agents for the treatment of diastolic dysfunction or diastolic heart failure known in the art some of which are described herein may be used. See the subsection entitled *Therapeutic agents for the Treatment of diastolic dysfunction or diastolic heart failure*. In exemplary aspects, the therapeutic is BH₄ or ranolazine.

As used herein, the term "asymmetric dimethylarginine" or "ADMA" refers to 2-Amino-5-[(amino-dimethylaminomethylene)amino]pentanoic acid. ADMA is a naturally occurring chemical found in blood plasma that interferes with L-Arginine in the production of nitric oxide. As used herein, the term "symmetric dimethylarginine" or "SDMA" refers to (2S)-2-amino-5-[(N,N'-dimethylcarbamimidoyl)amino]pentanoic acid. SDMA is endogenously produced and inhibits nitric oxide synthase. As used herein "L-Arginine" or "L-Arg" refers to the essential amino acid having the chemical name 2-Amino-5-guanidinopentanoic acid.

As used herein, the term "substantially unchanged" means that the level of SDMA of the biological sample is not statistically different from the level of SDMA of a control level or comparator level. In exemplary aspects, the level of SDMA is within one standard deviation ± the control level or comparator level. In exemplary aspects, the level of SDMA is within 10% or less ± the control level or comparator level. For example, if the control level or comparator level is 10, then a level of SDMA which is substantially unchanged from the control level or comparator level is a level within about 9 and about 11.

In exemplary aspects, the level of ADMA, SDMA, and/or L-Arg is an absolute level. In alternative aspects, the level of ADMA, SDMA, and/or L-Arg is a normalized or relative level. In exemplary aspects, the level of ADMA, SDMA, and/or L-Arg is relative to the level of ADMA, SDMA, and/or L-Arg of a control sample, wherein the control sample is obtained from a subject known to not have diastolic dysfunction or diastolic heart failure. In exemplary aspects, the level of ADMA, SDMA, and/or L-Arg of the control sample is set to 1, and the level of the subject (the test sample, the unknown sample, the non-control sample) is expressed relative to the control sample. An increased level in such aspects is determined when the the relative level is greater than 1, e.g., when the relative level is greater than one + one standard deviation.

### Levels of analytes

With regard to the inventive methods, the level(s) of MyBP-C, post-translationally modified forms of MyBP-C, fragments of MyBP-C, ADMA, SDMA, and/or L-Arginine (collectively referred to herein as "analytes") may be determined in the biological sample through any suitable means known in the art.

In exemplary aspects, the levels of the analytes are determined by measuring the levels from a biological sample. Suitable methods of determining levels of proteins are known in the art and include immunoassays (e.g., Western blotting, an enzyme-linked immunosorbent assay (ELISA), a radioimmunoassay (RIA), and immunohistochemical assay.

For example, the level(s) of the analytes may be determined through any known protein based detection method, including, but not limited to an immunoassay (e.g., radioimmunoassay, Western blotting, immunoprecipitation, immunostaining, immunofluorescence, enzyme-linked immunosorbent assay (ELISA) (e.g., sandwich ELISA, indirect ELISA, competitive ELISA), magnetic immunoassay, and the like). Antibodies to MyBP-C, glutathione, ADMA, and SDMA are commercially available from vendors, such as United States Biological (Marblehead, MA), Acris Antibodies (San Diego, CA), Antibodies-online.com (Atlanta, GA), and Abcam (Cambridge, MA). In exemplary embodiments, antibodies used in the step of determining the level(s) of MyBP-C, post-translationally modified forms of MyBP-C, fragments of MyBP-C, are the inventive antibodies described herein, which specifically bind to (a) S-glutathionylated MyBP-C, (b) an epitope within MyBP-C, which epitope comprises one of more of Cys 239, Cys249, Cys 426, Cys 436, Cys 443, Cys 475 , Cys528, Cys566, Cys 623, Cys 651, and/or Cys 719 of MyBP-C, optionally, wherein the Cys residue is S-glutationylated, or (c) one or more of Cys 239, Cys249, Cys 426, Cys 436, Cys 443, Cys 475 , Cys528, Cys566, Cys 623, Cys 651, and/orCys 719 of MyBP-C, wherein the Cys residue is S-glutationylated. In exemplary aspects, the antibodies used in the determining step is one of the binding agents of the invention conjugated to a binding partner of a high affinity binding interaction between two binding partners. In exemplary aspects, the antibodies used in the determining step is one of the binding agents of the invention conjugated to avidin. The level of MyBP-C, or a post-translationally modified form thereof, or a fragment thereof, may be determined, for example, by a combination of immunoprecipitation followed by Western blotting. For example, the level of an S-glutationylated MyBP-C or a S-glutathionylated fragment of MyBP-C may be determined, for example, by immunoprecipitation with an anti-glutathione antibody, followed by a MyBP-C specific antibody, or by immunoprecipitation with an anti-MyBP-C antibody, followed by a glutathione-specific antibody. The level of intact MyBP-C and/or MyBP-C fragments may be determined by immunoprecipitation with an anti-MyBP-C antibody followed by gel electrophoresis of the immunoprecipitates and staining of the resulting gel. Several kits for protein staining of gels are commercially available from vendors such as Promega.

Analytical techniques such as mass spectrometry also may be used to determine the level of the analytes in the biological sample. Tandem mass spectrometry utilized for the determination of levels of ADMA, SDMA, and L-Arginine is described in the art. See, e.g., Tang et al., Euro Heart J 29: 2506-2513 (2008). Mass spectrometry also may be used to determine the level of MyBP-C, or a post-translationally modified form thereof, or a fragment thereof.

In exemplary alternative aspects, the levels are determined by obtaining the levels from a medical record. For example, the levels may have been measured and recorded at a time prior to when the steps of the methods of the invention are carried out. In exemplary aspects, the levels are obtained from a medical record of a subject containing levels that were measured and recorded no more than 6 months prior to the time at which the steps of the method of the invention are carried out. In exemplary aspects, the levels are obtained from a medical record of a subject containing levels that were measured and recorded no more than 5 months or 4 months or 3 months or 2 months prior to the time at which the steps of the method of the invention are carried out. In exemplary aspects, the levels are obtained from a medical record of a subject containing levels that were measured and recorded no more than 1 month or 3 weeks or 2 weeks or 1 week prior to the time at which the steps of the method of the invention are carried out. In exemplary aspects, some of the levels are determined by obtaining the levels from a medical record and some are measured. In exemplary aspects, levels are determined by both measuring the level of a biological sample and obtaining from a medical record. In exemplary aspects, wherein a level of an analyte is determined by obtaining the level from a medical record, the method further comprises determining a level of the same or different analyte by measuring the level(s) from a biological sample, as described herein.

### Additional steps and aspects

In some embodiments of the methods provided herein, the method comprises additional steps or comprises a combination of the steps disclosed herein. In some embodiments, steps of the inventive method are repeated one or more times.

In exemplary aspects, the method comprises determining a level of MyBP-C and a level of a post-translationally modified form of MyBP-C, e.g., S-glutathionylated MyBP-C. In exemplary aspects, the method comprises determining a level of MyBP-C and one or more fragments of MyBP-C, optionally, wherein the MyBP-C and/or the one or more fragments of MyBP-C are S-glutathionylated.

In exemplary aspects, the method comprises a combination of determining a level of MyBP-C, a post-translationally modified form thereof, and/or a fragment thereof, in combination with determining a level of one or more of BNP, ADMA, SDMA, and L-Arginine. In exemplary aspects, the method further comprises determining a level of ADMA and L-Arginine. In exemplary aspects, the method further comprises determining a level of ADMA and SDMA. In exemplary aspects, the method further comprises determining a level of ADMA, SDMA, and L-Arginine.

In exemplary aspects, the method comprises one or more additional diagnostic steps. In exemplary aspects, the method comprises the measurement of one or more biomarkers of diastolic dysfunction as taught in International Patent Application No. PCT/US2010/54096, which published on May 12, 2011, as International Patent Application Publication No. WO/2011/056572. For example, the methods provided herein may include measurement ofrenin, angiotensin II, aldosterone, angiotensin-converting enzyme (ACE), NADPH oxidase, reactive oxygen species, glutathione disulfide (GSSG), oxidized cystine (CysS), a lipid peroxidase, an isoprostane, nitrite, nitrate, plasminogen activator inhibitor 1 (PAI-1), dihydrobiopterin (BH₂), uncoupled nitric oxide synthse (uncoupled NOS), glutathione (GSH), cysteine (Cys), nitric oxide (NO), a coupled nitric oxide synthase (coupled NOS), tetrahydrobiopterin (BH₄), Cys, DROM, isoprostane, angiotensin I, angiotensinogen, anti-diuretic hormone (ADH), leptin, resistin, or a combination of two or more of the foregoing.

In some embodiments, the method comprises measuring left ventricle (LV) pressure, volume, and/or wall thickness. In some embodiments, the method comprises executing calculations that reflect the process of active relaxation (the rate of isovolumic LV pressure and LV filling) and calculations that reflect passive stiffness (chamber compliance and myocardial viscoelastic stiffness) (Zile et al., (2010), *supra*).

In some embodiments, the methods comprise performing an echocardiography, as described in Silberman et al., Circulation 121: 519-528 (2010). In some aspects, LV tissue Doppler and mitral valve in-flow velocity are measured by echocardiography. In some aspects, the method comprises assaying for a late diastolic velocity (A') which is higher than the early diastolic velocity (E'), by tissue Doppler imaging (TDI).

In alternative or additional embodiments, the method comprises performing a magnetic resonance imaging (MRI), cardiac catheterization, or measurement of LV end diastolic pressure and systolic function.

In some embodiments, the method comprises additional steps which further characterize the subject. In exemplary aspects, the method comprises determining a body mass index (BMI) of the subject, performing a physical examination, performing a chest X-ray, or a combination thereof.

In exemplary aspects, the methods of the invention comprise further steps of informing an individual (e.g., the subject from whom the biological sample was obtained, a medical practitioner, a medical insurance provider, etc.) of the determined level of MyBP-C, a post-translationally modified form thereof, or fragment thereof, or ADMA, SDMA, or L-Arginine. In exemplary aspects, a medical practitioner of the subject from whom the biological sample is obtained, is informed and the medical practitioner prescribes anti-diastolic heart failure or anti-diastolic dysfunction agents to the subject. In exemplary aspects, the subject from whom the biological sample is obtained, is informed, and the subject begins a routine of regular monitoring of risk for diastolic dysfunction or diastolic heart failure treatment and/or begins treatment for diastolic heart failure or diastolic dysfunction.

In exemplary aspects, the inventive systems, computer-readable storage media, methods implemented by a processor in a computer, kits, or a combination thereof, each of which described below, may be utilized in any of the methods of the invention. For example, the diagnosing step of the inventive methods of diagnosing diastolic dysfunction or diastolic heart failure may be achieved with use of one of the inventive systems, computer-readable storage media, methods implemented by a processor in a computer, kits, or a combination thereof.

In exemplary aspects of the inventive methods, the methods further comprise the step of administering to the subject a therapeutic agent for the treatment of diastolic heart failure or diastolic dysfunction, when the level of MyBP-C, or a post-translationally modified form or fragment thereof, of the biological sample is increased, relative to a control. In exemplary aspects of other methods comprising the step of determining a level of ADMA, SDMA, and, optionally, L-Arginine, the method further comprises the step of administering to the subject a therapeutic agent for the treatment of diastolic heart failure or diastolic dysfunction, when the level ADMA is increased relative to a control level and the level of SDMA is unchanged relative to a control level, or when the ratio of the level of L-Arginine to the level of ADMA is decreased, relative to a control ratio. Suitable therapeutic agents for the treatment of diastolic heart failure or diastolic dysfunction are known in the art, some of which are described below.

### Therapeutic agents for the treatment of diastolic dysfunction or diastolic heart failure

The therapeutic agent suitable for treating the diastolic dysfunction or for treating diastolic heart failure may be any medical standard of care for diastolic dysfunction in the absence of systolic dysfunction or diastolic heart failure. In some aspects, the therapeutic agent is tetrahydrobiopterin, or a derivative thereof, such as any of those disclosed in U.S. Patent Application Publication No. 2008-0075666A1. In some aspects, the therapeutic agent is an agent which increases the level of adiponectin in the subject, such as any of those described herein. In some aspects, the therapeutic agent is a cardiac metabolic modifier in accordance with co-pending International Patent Application No. PCT/US2010/048650. Accordingly, the therapeutic agent in some embodiments comprises a structure of Formula I: 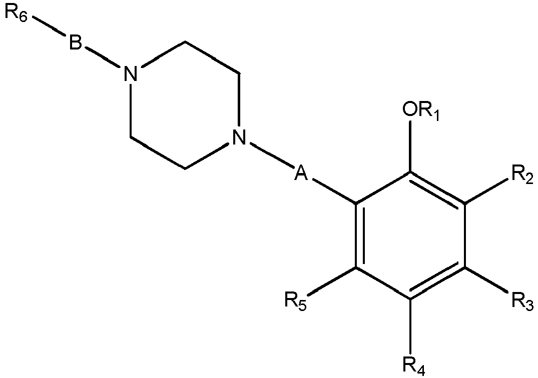 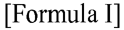
wherein A comprises a main chain of 1-8 atoms, each atom of which is independently C, O, N, or S, and each atom or which is optionally bound to an additional group selected from C1-C8 alkyl, C1-C8 alkoxy, OH, NH₂, NH(C1-C4 alkyl) and SH;
wherein R₁ is H or a C1-C8 alkyl;
wherein each of R₂, R₃, R₄, and R₅ independently is H, a C1-C8 alkyl, or a C1-C8 alkoxy;
wherein B is H or comprises a main chain of 1-8 atoms, each atom of which is independently C, O, N, or S, and each atom of which is optionally bound to an additional group; and,
wherein R₆ is absent or phenyl, which phenyl is optionally substituted with 1 to 5 groups, each group of which is independently C1-C8 alkyl, C1-C8 alkoxy, or OH.

As used herein, "alkyl" refers to straight chained and branched saturated hydrocarbon groups, nonlimiting examples of which include methyl, ethyl, and straight and branched propyl, butyl, pentyl, hexyl, heptyl, and octyl groups containing the indicated number of carbon atoms. The term Cn means the alkyl group has "n" carbon atoms. For example, C1-C7 alkyl refers to alkyl groups having a number of carbon atoms encompassing the entire range (i.e., 1 to 7 carbon atoms), as well as all subgroups (e.g., 1-6, 2-7, 1-5, 3-6, 1, 2, 3, 4, 5, 6, and 7 carbon atoms).. Accordingly, the C1-C8 alkyl can be a methyl, ethyl, propyl, butyl, C5 alkyl, C6 alkyl, C7 alkyl, or C8 alkyl, of which the propyl, butyl, C5 alkyl, C6 alkyl, C7 alkyl, or C8 alkyl is a straight chain alkyl or branched alkyl.

As used herein "alkoxy" refers to -OR, wherein R is alkyl (e.g., a straight or branched chain alkyl group). Examples of alkoxy groups include, but are not limited to, methoxy, ethoxy, propoxy, isopropoxy, butoxy, n-butoxy, sec-butoxy, t-butoxy and the like. Accordingly, the C1-C8 alkoxy can be methoxy, ethoxy, C3 alkoxy, C4 alkoxy, C5 alkoxy, C6 alkoxy, C7 alkoxy, or C8 alkoxy, or which the C3 alkoxy, C4 alkoxy, C5 alkoxy, C6 alkoxy, C7 alkoxy, or C8 alkoxy is a straight chain alkoxy or branched alkoxy.

As used herein "NH(C1-C4 alkyl)" refers to nitrogen bound to both H and a C1-C4 alkyl.

With regard to Formula I, A comprises a main chain of 1-8 atoms, each atom of which is independently C, O, N, or S. In some aspects, A comprises a main chain of a single atom selected from C, O, N, and S. In some aspects, A comprises a main chain of 2-8 atoms (e.g., 2, 3, 4, 5, 6, 7, or 8 atoms), each atom of which is independently C, O, N, or S.

Each atom of the main chain of A is optionally bound to an additional group. In some aspects, the additional group is selected from C1-C8 alkyl, C1-C8 alkoxy, OH, NH2, NH(C1-C4 alkyl) and SH. In some aspects, every atom of the main chain is bound to an additional group. In other aspects, one or more, but not all, atoms of the main chain are bound to an additional group. In some instances, one atom of the main chain is bound to an additional group. In some instances, 2, 3, 4, 5, 6, or 7 atoms of the main chain is bound to an additional group.

In some aspects, A is (CH₂)₁₋₈. In some aspects, A is (CH₂)₁₋₆. In some aspects, A is (CH₂)₁₋₄. In some aspects, A is (CH₂)₁ or (CH₂)₂. In some aspects, A is (CH₂)₁.

In other aspects, A comprises a structure of Formula IV: 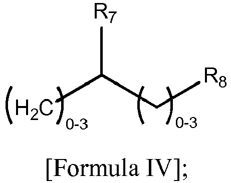 wherein R₇ is OH, C1-C4 alkyl, C1-C4 alkoxy, NH2, or NH(C1-C4 alkyl); and wherein R₈ is O or NH. In some aspects, when A comprises a structure of Formula IV, R₇ is OH and R₈ is O or NH. In some aspects, when A comprises a structure of Formula IV, R₇ is OH, C1-C4 alkyl, C1-C4 alkoxy, NH2, or NH(C1-C4 alkyl) and R₈ is O. In particular aspects, when A comprises a structure of Formula IV, R₇ is OH and R₈ is O. In some aspects, A comprises

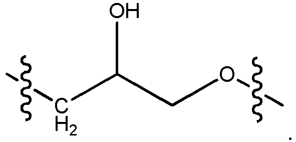

With regard to Formula I, R₁ is H or a C1-C8 alkyl. In particular aspects, R₁ is CH₃.

With regard to Formula I, each of R₂, R₃, R₄, and R₅ independently is H, a C1-C8 alkyl, or a C1-C8 alkoxy. In some aspects, each of R₂, R₃, R₄, and R₅ independently is H or methoxy. In alternative embodiments, each of R₂ and R₃ is a methoxy, and each of R₄ and R₅ is H.

With regard to Formula I, B is H or comprises a main chain of 1-8 atoms, each atom of which is independently C, O, N, or S. In some aspects, B comprises a main chain of a single atom selected from C, O, N, and S, while in other aspects, B comprises a main chain of 2-8 atoms (e.g., 2, 3, 4, 5, 6, 7, or 8 atoms), each atom of which is independently selected from C, O, N, and S.

Each atom of the main chain of B is optionally bound to an additional group. In some aspects, the additional group is selected from C1-C8 alkyl, C1-C8 alkoxy, OH, NH2, NH(C1-C4 alkyl) and SH. In some aspects, every atom of the main chain is bound to an additional group. In other aspects, one or more, but not all, atoms of the main chain are bound to an additional group. In some instances, one atom of the main chain is bound to an additional group. In some instances, 2, 3, 4, 5, 6, or 7 atoms of the main chain is bound to an additional group.

In some embodiments, B comprises H and R₆ is absent. In alternative embodiments, B comprises a structure of Formula V: 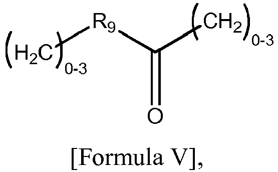 wherein R₉ is NH or O.

In some embodiments, when B comprises a structure of Formula V, R9 is NH. In further aspects, B comprises a structure of

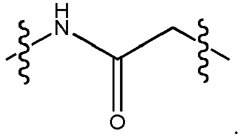

With regard to Formula I, R₆ is absent or phenyl, which phenyl is optionally substituted with 1 to 5 (e.g., 1, 2, 3, 4, 5) groups, each group of which is independently selected from C1-C8 alkyl, C1-C8 alkoxy, and OH. In some aspects, R₆ is absent. In alternative aspects, when R₆ is present and comprises phenyl substituted with 1 to 5 (e.g., 1, 2, 3, 4, 5) methyl groups. In specific aspects, R₆ comprises phenyl substituted with two methyl groups, one at each of the ortho positions.

In some aspects, the cardiac metabolic modifier comprises a compound of Formula II, or a pharmaceutically acceptable salt thereof or a conjugate thereof: 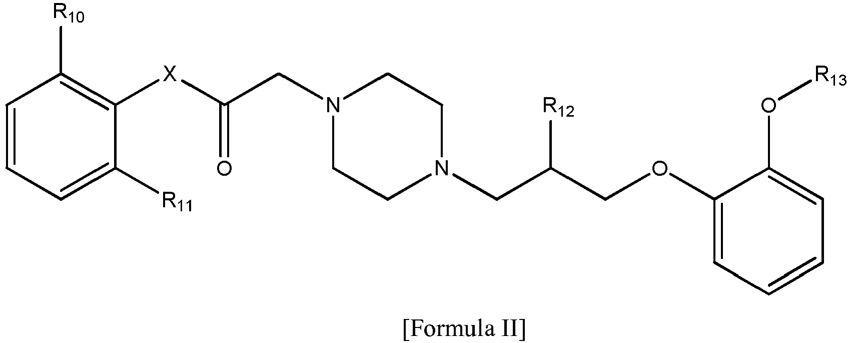
wherein each of R₁₀, R₁₁, and R₁₃ independently is C1-C3 alkyl,
wherein X is NH or O; and
wherein R₁₂ is OH or C1-C3 alkyl.

In some embodiments, the compound of Formula II comprises the following structure: 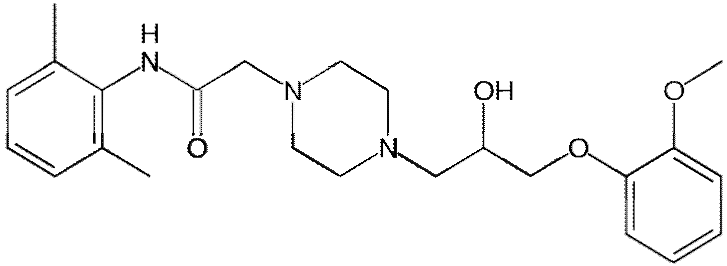 In some aspects, the compound of Formula II is ranolazine, or a pharmaceutically acceptable salt or a conjugate of ranolazine.

In some aspects, the cardiac metabolic modifier is a compound of Formula III, or a pharmaceutically acceptable salt thereof or a conjugate thereof: 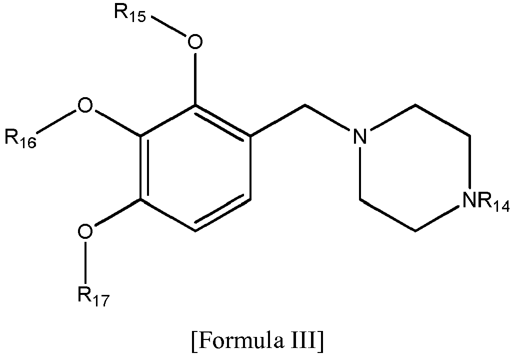 wherein each of R₁₄, R₁₅, R₁₆, and R₁₇ independently is H or C1-C3 alkyl.

In some embodiments, the compound of Formula II comprises the following structure:

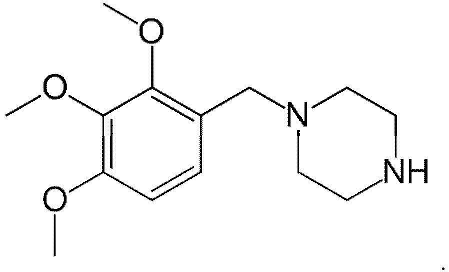

In some aspects, the compound of Formula III is trimetazidine, or a pharmaceutically acceptable salt or a conjugate of trimetazidine.

### Treatment and Prevention

The terms "treat," and "prevent" as well as words stemming therefrom, as used herein, do not necessarily imply 100% or complete treatment or prevention. Rather, there are varying degrees of treatment or prevention of which one of ordinary skill in the art recognizes as having a potential benefit or therapeutic effect. In this respect, the inventive methods can provide any amount of any level of treatment or prevention of diastolic dysfunction or heart failure in a mammal. Furthermore, the treatment or prevention provided by the inventive method can include treatment or prevention of one or more conditions or symptoms of the disease, e.g., cancer, being treated or prevented. Also, for purposes herein, "prevention" can encompass delaying the onset of the disease, or a symptom or condition thereof

### Biological Samples

With regard to the methods disclosed herein, in some embodiments, the biological sample comprises a bodily fluid, including, but not limited to, whole blood, or a fraction thereof (e.g., plasma, serum), lymph, breast milk, saliva, mucous, semen, vaginal secretions, cellular extracts, inflammatory fluids, cerebrospinal fluid, feces, vitreous humor, or urine obtained from the subject. In some aspects, the sample is a composite panel of at least two of the foregoing samples. In some aspects, the sample is a composite panel of at least two of a blood sample, a plasma sample, a serum sample, and a urine sample. In exemplary aspects, the biological sample is plasma.

### Subjects

In some embodiments of the invention, the subject is a mammal, including, but not limited to, mammals of the order Rodentia, such as mice and hamsters, and mammals of the order Logomorpha, such as rabbits, mammals from the order Carnivora, including Felines (cats) and Canines (dogs), mammals from the order Artiodactyla, including Bovines (cows) and Swines (pigs) or of the order Perssodactyla, including Equines (horses). In some aspects, the mammals are of the order Primates, Ceboids, or Simoids (monkeys) or of the order Anthropoids (humans and apes). In some aspects, the mammal is a human. In specific aspects, the human is a male human. In additional or alternative aspects, the human has a BMI of about 29 or higher.

In some embodiments, the subject exhibits or is presenting a sign or symptom of diastolic dysfunction. In exemplary embodiments, the subject exhibits an ejection fraction which is greater than or about 45%, e.g., greater than or about 50%, e.g., 50-75%.

In some aspects, the subject is suffering from diastolic dysfunction in the presence of systolic dysfunction. In some aspects, the subject is suffering from diastolic dysfunction in the absence of systolic dysfunction.

In some embodiments, the subject exhibits or is presenting a sign or symptom of heart failure. In some aspects, the sign or symptom of heart failure is one of those previously described herein. See the section entitled *Heart Failure.*

In some embodiments, the subject is suffering from heart failure, e.g., diastolic heart failure, any of the types of heart failure described herein. See the section entitled *Heart Failure.* In exemplary aspects, the subject is not suffering from myocardial infarction. In exemplary aspects, the subject is not suffering from systolic dysfunction or systolic heart failure.

In exemplary aspects, the subject suffers from shortness of breath.

In some embodiments, the subject suffers from hypertension. Hypertension is a chronic medical condition in which the systemic arterial blood pressure is elevated. The hypertension in some embodiments is classified as a primary hypertension for which no medical cause is found. In some embodiments, the hypertension is a secondary hypertension caused by another condition that affects the kidneys, arteries, heart, or endocrine system.

A systolic or the diastolic blood pressure measurement higher than the accepted normal values for the age of the individual is classified as prehypertension or hypertension.

| **Classification** | **Systolic pressure** | | **Diastolic pressure** | |
|---|---|---|---|---|
| | **mmHg** | **kPa** | **mmHg** | **kPa** |
| Normal | 90-119 | 12-15.9 | 60-79 | 8.0-10.5 |
| Prehypertension | 120-139 | 16.0-18.5 | 80-89 | 10.7-11.9 |
| Stage 1 | 140-159 | 18.7-21.2 | 90-99 | 12.0-13.2 |
| Stage 2 | ≥160 | ≥21.3 | ≥100 | ≥13.3 |
| Isolated systolic hypertension | ≥140 | ≥18.7 | <90 | <12.0 |
| *Source*: Chobanian et al. (2003) | | | | |

Hypertension has several sub-classifications including, hypertension stage I, hypertension stage II, and isolated systolic hypertension. Isolated systolic hypertension refers to elevated systolic pressure with normal diastolic pressure and is common in the elderly. These classifications are made after averaging a patient's resting blood pressure readings taken on two or more office visits. Individuals older than 50 years are classified as having hypertension if their blood pressure is consistently at least 140 mmHg systolic or 90 mmHg diastolic. Patients with blood pressures higher than 130/80 mmHg with concomitant presence of diabetes mellitus or kidney disease require further treatment (Chobanian et al. (December 2003). "Seventh report of the Joint National Committee on Prevention, Detection, Evaluation, and Treatment of High Blood Pressure". Hypertension 42(6): 1206-52.)

In some aspects, the subject suffers from a metabolic disease or metabolic syndrome. Metabolic Syndrome, also known as metabolic syndrome X, insulin resistance syndrome or Reaven's syndrome, is a disorder that affects over 50 million Americans. Metabolic Syndrome is typically characterized by a clustering of at least three or more of the following risk factors: (1) abdominal obesity (excessive fat tissue in and around the abdomen), (2) atherogenic dyslipidemia (blood fat disorders including high triglycerides, low HDL cholesterol and high LDL cholesterol that enhance the accumulation of plaque in the artery walls), (3) elevated blood pressure, (4) insulin resistance or glucose intolerance, (5) prothrombotic state (e.g.,high fibrinogen or plasminogen activator inhibitor-1 in blood), and (6) pro-inflammatory state (e.g.,elevated C-reactive protein in blood). Other risk factors may include aging, hormonal imbalance and genetic predisposition.

Metabolic Syndrome is associated with an increased the risk of coronary heart disease and other disorders related to the accumulation of vascular plaque, such as stroke and peripheral vascular disease, referred to as atherosclerotic cardiovascular disease (ASCVD). Patients with Metabolic Syndrome may progress from an insulin resistant state in its early stages to full blown type II diabetes with further increasing risk of ASCVD. Without intending to be bound by any particular theory, the relationship between insulin resistance, Metabolic Syndrome and vascular disease may involve one or more concurrent pathogenic mechanisms including impaired insulin-stimulated vasodilation, insulin resistance-associated reduction in NO availability due to enhanced oxidative stress, and abnormalities in adipocyte-derived hormones such as adiponectin (Lteif and Mather, Can. J. Cardiol. 20 (suppl. B):66B-76B (2004)).

According to the 2001 National Cholesterol Education Program Adult Treatment Panel (ATP III), any three of the following traits in the same individual meet the criteria for Metabolic Syndrome: (a) abdominal obesity (a waist circumference over 102 cm in men and over 88 cm in women); (b) serum triglycerides (150 mg/dl or above); (c) HDL cholesterol (40 mg/dl or lower in men and 50 mg/dl or lower in women); (d) blood pressure (130/85 or more); and (e) fasting blood glucose (110 mg/dl or above). According to the World Health Organization (WHO), an individual having high insulin levels (an elevated fasting blood glucose or an elevated post meal glucose alone) with at least two of the following criteria meets the criteria for Metabolic Syndrome: (a) abdominal obesity (waist to hip ratio of greater than 0.9, a body mass index of at least 30 kg/m2, or a waist measurement over 37 inches); (b) cholesterol panel showing a triglyceride level of at least 150 mg/dl or an HDL cholesterol lower than 35 mg/dl; (c) blood pressure of 140/90 or more, or on treatment for high blood pressure). (Mathur, Ruchi, "Metabolic Syndrome," ed. Shiel, Jr., William C., MedicineNet.com, May 11, 2009).

For purposes herein, if an individual meets the criteria of either or both of the criteria set forth by the 2001 National Cholesterol Education Program Adult Treatment Panel or the WHO, that individual is considered as afflicted with Metabolic Syndrome.

With regard to the methods of the invention, in some embodiments, the subject suffers from diabetes or obesity or suffers from both diabetes and obesity.

In some embodiments, the subject does not suffer from a cardiac injury or a structural heart disease other than the diastolic dysfunction or heart failure being treated or prevented or diagnosed by the inventive method. By "cardiac injury" is meant a disruption of normal cardiac myocyte membrane integrity resulting in the loss into the extracellular space or intracellular constituents including detectable levels of biologically active cytosolic and structure proteins (e.g., troponin, creatine kinase, myoglobin, heart-type fatty acid binding protein, lactate dehydrogenase). By "structural heart disease" is meant any disease that affects the heart muscle or changes the architecture of the heart. In some aspects, the subject does not suffer from ischemic heart disease, chronic stable angina, chronic angina. In some aspects, the subject does not suffer from ischemia, ischemia-reperfusion or coronary artery occlusion-reperfusion, ischemic heart disease, myocardial injury, myocardial toxicity, myocardial infarction, congenital heart lesion, valvular stenosis or valvular regurgitation, coronary artery disease, chronic angina, chronic stable angina, arrhythmias. In some aspects, the subject does not suffer from a myocardial trauma, a myocardial toxicity, a viral infection, a deficiency in nutrients. In some aspects, the subject does not suffer from myocarditis.

In regards to the methods of treatment provided herein, the subject in some embodiments is a subject in need thereof. In some aspects, the subject is a subject suffering from diastolic dysfunction, e.g., any of the forms of diastolic dysfunction described in the section set forth herein entitled "*Diastolic Dysfunction.*" In some aspects, the subject is a subject suffering from heart failure, e.g., diastolic heart failure, heart failure with preserved ejection fraction, any of the types of heart failure described in the section set forth herein entitled "*Heart Failure.*"

### Control Levels

With regard to the methods of the invention, levels of analytes (MyBP-C, the post-translationally modified form thereof or the fragment thereof, ADMA, SDMA, and/or L-Arginine) of the biological sample obtained from the subject are compared to one or more control levels. In exemplary aspects, the control level is the level of the same analyte(s) but it is the level of a biological sample obtained from a control subject.

In some embodiments, the control subject is a matched control of the same species, gender, ethnicity, age group, smoking status, BMI, current therapeutic regimen status, medical history, or a combination thereof, but differs from the subject being diagnosed in that the control does not suffer from diastolic dysfunction. In some embodiments, the control subject is a matched control of the same species, gender, ethnicity, age group, smoking status, BMI, current therapeutic regimen status, medical history, or a combination thereof, but differs from the subject being diagnosed in that the control subject suffers from neither diastolic dysfunction nor systolic dysfunction. In alternative aspects, the control subject is a matched control of the same species, gender, ethnicity, age group, smoking status, BMI, current therapeutic regimen status, medical history, or a combination thereof, but differs from the subject being diagnosed in that the control does not suffer from diastolic dysfunction but does suffer from systolic dysfunction. In alternative aspects, the control subject is a matched control of the same species, gender, ethnicity, age group, smoking status, BMI, current therapeutic regimen status, medical history, or a combination thereof, but differs from the subject being diagnosed in that the control does not suffer from systolic dysfunction but does suffer from diastolic dysfunction. In alternative aspects, the control subject is a matched control of the same species, gender, ethnicity, age group, smoking status, BMI, current therapeutic regimen status, medical history, or a combination thereof, but differs from the subject being diagnosed in that the control does not suffer from heart failure.

With regard to the method of diagnosing a type of heart failure, when the subject being diagnosed is a subject suffering from a heart failure, the control subject in some embodiments is a control subject suffering from heart failure. In some aspects, the control subject suffers from a systolic heart failure or a heart failure with reduced ejection fraction. In alternative aspects, the control subject is not suffering from heart failure.

With regard to the inventive methods of the invention, the control level, in exemplary aspects, is a level of the analyte in a patient not suffering from heart failure, unless otherwise indicated.

In some aspects, the control level of ADMA is a level which is under 0.65 µM, when the biological sample is plasma, such that a level of ADMA in plasma above this number is considered a state of diastolic dysfunction or diastolic heart failure, or risk therefor. In some aspects, the control ratio of L-Argining to ADMA is a ratio which is over 170, such that a ratio of L-Arg:ADMA under 170 is considered a state of diastolic dysfunction or diastolic heart failure, or risk therefor.

Relative to a control level, the level of the analyte that is determined may an increased level. As used herein, the term "increased" with respect to level refers to any % increase above a control level. The increased level may be at least or about a 5% increase, at least or about a 10% increase, at least or about a 15% increase, at least or about a 20% increase, at least or about a 25% increase, at least or about a 30% increase, at least or about a 35% increase, at least or about a 40% increase, at least or about a 45% increase, at least or about a 50% increase, at least or about a 55% increase, at least or about a 60% increase, at least or about a 65% increase, at least or about a 70% increase, at least or about a 75% increase, at least or about a 80% increase, at least or about a 85% increase, at least or about a 90% increase, at least or about a 95% increase, relative to a control level. In exemplary aspects, the increased level is at least a 50% increase over the control level. The increased level may be a 1.1-fold, a 1.2-fold, a 1.3-fold, a 1.4-fold, a 1.5-fold, a 1.6-fold, a 1.7-fold, a 2.0-fold, a 2.5-fold, a 3.0 fold, a 3.5-fold, a 4.0-fold, a 4.5-fold, a 5.0 fold, a 6.0 fold, a 7.0-fold, an 8.0-fold, a 9.0-fold, 10-fold, a 15-fold, a 20-fold, a 30-fold, a 40-fold, a 50-fold or more, increase, relative to the control level.

Relative to a control level, the level that is determined may be a decreased level. As used herein, the term "decreased" with respect to level (e.g., expression level, *biological* activity level) refers to any % decrease below a control level. The decreased level may be at least or about a 5% decrease, at least or about a 10% decrease, at least or about a 15% decrease, at least or about a 20% decrease, at least or about a 25% decrease, at least or about a 30% decrease, at least or about a 35% decrease, at least or about a 40% decrease, at least or about a 45% decrease, at least or about a 50% decrease, at least or about a 55% decrease, at least or about a 60% decrease, at least or about a 65% decrease, at least or about a 70% decrease, at least or about a 75% decrease, at least or about a 80% decrease, at least or about a 85% decrease, at least or about a 90% decrease, at least or about a 95% decrease, relative to a control level. In exemplary aspects, the decreased level is at least a 50% decrease below the control level. The decreased level may be a 1.1-fold, a 1.2-fold, a 1.3-fold, a 1.4-fold, a 1.5-fold, a 1.6-fold, a 1.7-fold, a 2.0-fold, a 2.5-fold, a 3.0 fold, a 3.5-fold, a 4.0-fold, a 4.5-fold, a 5.0 fold, a 6.0 fold, a 7.0-fold, an 8.0-fold, a 9.0-fold, 10-fold, a 15-fold, a 20-fold, a 30-fold, a 40-fold, a 50-fold or more, decrease, relative to the control level.

In exemplary aspects, the ratio of the level of L-Arginine of the biological sample to the level of ADMA of the biological sample is decreased, relative to a control level. In exemplary aspects, the ratio is at least a 50% decrease below the control level.

### Antibodies and other Binding Agents

Isolated binding agents which are useful in the inventive methods are also provided herein. In exemplary embodiments, the isolated binding agent specifically binds to S-glutathionylated MyBP-C. In exemplary aspects, the MyBP-C is S-glutathionylated at one or more of Cys 239, Cys249, Cys 426, Cys 436, Cys 443, Cys 475 , Cys528, Cys566, Cys 623, Cys 651, and/or Cys 719, according to the amino acid position numbering of SEQ ID NO: 21. In exemplary aspects, the MyBP-C is S-glutathionylated at one or more of Cys 475, 623, and/or 651, according to the amino acid position numbering of SEQ ID NO: 21. In exemplary aspects, the binding agent does not bind to a MyBP-C lacking one or more S-glutathionylatied Cys residues. In exemplary aspects, the binding agent does not bind to an un-S-glutathionylated MyBP-C. In exemplary aspects, the binding agent does not bind to one or more of Cys 239, Cys249, Cys 426, Cys 436, Cys 443, Cys 475 , Cys528, Cys566, Cys 623, Cys 651, Cys 719, according to the amino acid position numbering of SEQ ID NO: 21, when the Cys residue is not S-glutathionylated. In exemplary aspects, the binding agent does not bind to one or more of Cys475, Cys 623, and/or Cys651, according to the amino acid position numbering of SEQ ID NO: 21, when the Cys residue is not S-glutathionylated.

In exemplary embodiments, the isolated binding agent specifically binds to an epitope within MyBP-C, wherein the epitope comprises one or more of Cys 239, Cys249, Cys 426, Cys 436, Cys 443, Cys 475 , Cys528, Cys566, Cys 623, Cys 651, Cys 719, according to the amino acid position numbering of SEQ ID NO: 21. In exemplary aspects, the epitope comprises one or more of Cys 475, Cys 623, and/or Cys 651, according to the amino acid position numbering of SEQ ID NO: 21. In exemplary aspects, the one or more Cys residues is/are S-glutathionylated. In exemplary aspects, the binding agent does not bind to the one or more Cys residues when the one or more Cys residues is/are not S-glutathionylated. In alternative aspects, the one or more Cys residues is/are not S-glutathionylated and the binding agent binds to the one or more Cys residues only when it/they is/are not S-glutathionylated.

In exemplary embodiments, the isolated binding agent specifically binds to a Cys residue of MyBP-C, wherein the Cys residue is Cys 239, Cys249, Cys 426, Cys 436, Cys 443, Cys 475 , Cys528, Cys566, Cys 623, Cys 651, Cys 719, according to the amino acid position numbering of SEQ ID NO: 21. In exemplary aspects, the Cys residue is Cys 475, Cys 623, and/or Cys 651, according to the amino acid position numbering of SEQ ID NO: 21. In exemplary aspects, the Cys residue is S-glutathionylated. In exemplary aspects, the binding agent does not bind to the Cys residue when the Cys residue is not S-glutathionylated. In alternative aspects, the Cys residue is not S-glutathionylated and the binding agent binds to the Cys residue only when it is not S-glutathionylated.

As used herein, the term "binding agent" refers to a molecule comprising one or more binding units (directly or indirectly) associated with each other by covalent or non-covalent bonds. As used herein, the term "binding unit" refers to the portion of a binding agent which specifically binds to MyBP-C, e.g., S-glutathionylated MyBP-C, a Cys residue of MyBP-C, an epitope within MyBP-C. In exemplary aspects, the binding unit binds with high affinity. The term "high affinity" is used in a physiological context pertaining to the relative affinity of the binding agent for the MyBP-C, e.g., S-glutathionylated MyBP-C, a Cys residue of MyBP-C, an epitope within MyBP-C, *in vivo* in a mammal, e.g., a laboratory test animal, a domesticated farm or pet animal, or a human. In certain embodiments, the binding unit has a dissociation constant (K_{D}) for the MyBP-C, e.g., S-glutathionylated MyBP-C, a Cys residue of MyBP-C, an epitope within MyBP-C, which is in the sub-nanomolar (e.g., picomolar), nanomolar range, or micromolar range. In some embodiments, the K_{D} is between about 0.0001 nM and about 100 nM. In some embodiments, the K_{D} is at least or about 0.0001 nM, at least or about 0.001 nM, at least or about 0.01 nM, at least or about 0.1 nM, at least or about 1 nM, or at least or about 10 nM. In some embodiments, the K_{D} is no more than or about 100 nM, no more than or about 75 nM, no more than or about 50 nM, or no more than or about 25 nM.

In certain aspects, each binding unit or at least one of the binding units of the binding agent comprises at least one peptide or polypeptide. In other aspects, the binding unit comprises multiple peptides or polypeptides, covalently or non-covalently joined together. In specific aspects, the binding unit comprises at least one antibody, or antigen binding fragment thereof, such that the binding agent comprises at least one antibody, or antigen binding fragment thereof. In some embodiments, the binding agent comprises more than one binding unit, and, in specific aspects, some or all of the binding units of the binding agent are antibodies, or antigen binding fragments thereof. Further descriptions of such embodiments are described herein. See, e.g., the section entitled *Antibodies and Antigen Binding Fragments.*

In certain aspects, the binding agent comprises one or more binding units that are not polypeptides comprising an antibody or antigen binding fragment thereof. In some embodiments, the binding agent comprises at least one binding unit which is not an antibody or antigen binding fragment thereof. In alternative embodiments, the binding unit is neither a peptide nor a polypeptide. In exemplary specific aspects, the binding unit comprises one or more of: an organic small molecule, an aptamer, and combinations thereof.

In some embodiments, the binding units (e.g., peptide, polypeptide, or other) are directly joined together in the absence of a linker. In alternative aspects, the binding units of the binding agent are indirectly connected via one or more linkers. Whether directly joined together or indirectly joined together through a linker, the binding units may be connected through covalent bonds (e.g., a peptide, ester, amide, or sulfhydryl bond) or non-covalent bonds (e.g., via hydrophobic interaction, hydrogen bond, van der Waals bond, electrostatic or ionic interaction), or a combination thereof. The binding units may be connected via any means known in the art, including, but not limited to, any of those taught herein with regard to conjugation of a binding agent to a second moiety.

### Antibodies and Antigen Binding Fragments Thereof

In some aspects of the invention, the binding agent is an antibody, or antigen binding fragment thereof, which specifically binds to MyBP-C, e.g., S-glutathionylated MyBP-C, a Cys residue of MyBP-C, an epitope within MyBP-C, in accordance with the disclosures herein. The antibody can be any type of immunoglobulin that is known in the art. For instance, the antibody can be of any isotype, e.g., IgA, IgD, IgE, IgG, IgM, etc. The antibody can be monoclonal or polyclonal. The antibody can be a naturally-occurring antibody, e.g., an antibody isolated and/or purified from a mammal, e.g., mouse, rabbit, goat, horse, chicken, hamster, human, and the like. In this regard, the antibody may be considered as a mammalian or avian antibody, e.g., a mouse antibody, rabbit antibody, goat antibody, horse antibody, chicken antibody, hamster antibody, human antibody, and the like. The term "isolated" as used herein means having been removed from its natural environment. The term "purified," as used herein relates to the isolation of a molecule or compound in a form that is substantially free of contaminants normally associated with the molecule or compound in a native or natural environment and means having been increased in purity as a result of being separated from other components of the original composition. It is recognized that "purity" is a relative term, and not to be necessarily construed as absolute purity or absolute enrichment or absolute selection. In some aspects, the purity is at least or about 50%, is at least or about 60%, at least or about 70%, at least or about 80%, or at least or about 90% (e.g., at least or about 91%, at least or about 92%, at least or about 93%, at least or about 94%, at least or about 95%, at least or about 96%, at least or about 97%, at least or about 98%, at least or about 99% or is approximately 100%.

The antibody can have any level of affinity or avidity for the MyBP-C, e.g., S-glutathionylated MyBP-C, a Cys residue of MyBP-C, an epitope within MyBP-C. The dissociation constant (K_{D}) may be any of those exemplary dissociation constants described herein with regard to binding units. Binding constants, including dissociation constants, may be determined by methods known in the art, including, for example, methods which utilize the principles of surface plasmon resonance, e.g., methods utilizing a Biacore™ system. In accordance with the foregoing, in some embodiments, the antibody is in monomeric form, while in other embodiments, the antibody is in polymeric form. In certain embodiments in which the antibody comprises two or more distinct antigen binding regions fragments, the antibody is considered bispecific, trispecific, or multi-specific, or bivalent, trivalent, or multivalent, depending on the number of distinct epitopes that are recognized and bound by the binding agent.

If the binding agents reduce the interaction between MyBP-C, e.g., S-glutathionylated MyBP-C, a Cys residue of MyBP-C, an epitope within MyBP-C, and a binding partner, in some embodiments, the antibody is considered as a blocking antibody or neutralizing antibody. In some aspects, the K_{D} of the binding agent is about the same as the K_{D} of the binding partner of the MyBP-C, e.g., S-glutathionylated MyBP-C, a Cys residue of MyBP-C, an epitope within MyBP-C. In some aspects, the K_{D} of the binding agent is lower (e.g., at least 0.5-fold lower, at least 1-fold lower, at least 2-fold lower, at least 5-fold lower, at least 10-fold lower, at least 25-fold lower, at least 50-fold lower, at least 75-fold lower, at least 100-fold lower) than the K_{D} of the binding parnter of MyBP-C, e.g., S-glutathionylated MyBP-C, a Cys residue of MyBP-C, an epitope within MyBP-C.

In some embodiments, the antibody can be a genetically-engineered antibody, e.g., a single chain antibody, a humanized antibody, a chimeric antibody, a CDR-grafted antibody, an antibody which includes portions of CDR sequences specific for MyBP-C, e.g., S-glutathionylated MyBP-C, a Cys residue of MyBP-C, an epitope within MyBP-C, a humaneered antibody, a bispecific antibody, a trispecific antibody, and the like. Genetic engineering techniques also provide the ability to make fully human antibodies in a non-human source.

In some aspects, the antibody is a chimeric antibody. The term "chimeric antibody" is used herein to refer to an antibody containing constant domains from one species and the variable domains from a second, or more generally, containing stretches of amino acid sequence from at least two species.

In some aspects, the antibody is a humanized antibody. The term "humanized" when used in relation to antibodies is used to refer to antibodies having at least CDR regions from a nonhuman source which are engineered to have a structure and immunological function more similar to true human antibodies than the original source antibodies. For example, humanizing can involve grafting CDR from a non-human antibody, such as a mouse antibody, into a human antibody. Humanizing also can involve select amino acid substitutions to make a non-human sequence look more like a human sequence.

Use of the terms "chimeric or humanized" herein is not meant to be mutually exclusive, and rather, is meant to encompass chimeric antibodies, humanized antibodies, and chimeric antibodies that have been further humanized. Except where context otherwise indicates, statements about (properties of, uses of, testing, and so on) chimeric antibodies of the invention apply to humanized antibodies of the present disclosures, and statements about humanized antibodies of the present disclosures pertain also to chimeric antibodies. Likewise, except where context dictates, such statements also should be understood to be applicable to antibodies and antigen binding fragments of such antibodies of the invention.

In some aspects of the invention, the binding agent is an antigen binding fragment of an antibody, which specifically binds to MyBP-C, e.g., S-glutathionylated MyBP-C, a Cys residue of MyBP-C, an epitope within MyBP-C, in accordance with the disclosures herein. The antigen binding fragment (also referred to herein as "antigen binding portion") may be an antigen binding fragment of any of the antibodies described herein. The antigen binding fragment can be any part of an antibody that has at least one antigen binding site, including, but not limited to, Fab, F(ab')₂, dsFv, sFv, diabodies, triabodies, bis-scFvs, fragments expressed by a Fab expression library, domain antibodies, VhH domains, V-NAR domains, VH domains, VL domains, and the like. Antibody fragments of the invention, however, are not limited to these exemplary types of antibody fragments.

A domain antibody comprises a functional binding unit of an antibody, and can correspond to the variable regions of either the heavy (V_{H}) or light (V_{L}) chains of antibodies. A domain antibody can have a molecular weight of approximately 13 kDa, or approximately one-tenth of a full antibody. Domain antibodies may be derived from full antibodies such as those described herein. The antigen binding fragments in some embodiments are monomeric or polymeric, bispecific or trispecific, bivalent or trivalent.

Antibody fragments that contain the antigen binding, or idiotype, of the antibody molecule may be generated by techniques known in the art. For example, such fragments include, but are not limited to, the F(ab')₂ fragment which may be produced by pepsin digestion of the antibody molecule; the Fab' fragments which may be generated by reducing the disulfide bridges of the F(ab')₂ fragment, and the two Fab' fragments which may be generated by treating the antibody molecule with papain and a reducing agent.

A single-chain variable region fragment (sFv) antibody fragment, which consists of a truncated Fab fragment comprising the variable (V) domain of an antibody heavy chain linked to a V domain of a light antibody chain via a synthetic peptide, can be generated using routine recombinant DNA technology techniques (see, e.g., Janeway et al., supra). Similarly, disulfide-stabilized variable region fragments (dsFv) can be prepared by recombinant DNA technology (see, e.g., Reiter et al., Protein Engineering, 7, 697-704 (1994)).

Recombinant antibody fragments, e.g., scFvs, can also be engineered to assemble into stable multimeric oligomers of high binding avidity and specificity to different target antigens. Such diabodies (dimers), triabodies (trimers) or tetrabodies (tetramers) are well known in the art, see *e.g.,* Kortt et al., Biomol Eng. 2001 18:95-108, (2001) and Todorovska et al., J Immunol Methods. 248:47-66, (2001).

Bispecific antibodies (bscAb) are molecules comprising two single-chain Fv fragments joined via a glycine-serine linker using recombinant methods. The V light-chain (V_{L}) and V heavy-chain (V_{H}) domains of two antibodies of interest in exemplary embodiments are isolated using standard PCR methods. The V_{L} and V_{H} cDNA's obtained from each hybridoma are then joined to form a single-chain fragment in a two-step fusion PCR. Bispecific fusion proteins are prepared in a similar manner. Bispecific single-chain antibodies and bispecific fusion proteins are antibody substances included within the scope of the present invention. Exemplary bispecific antibodies are taught in U.S. Patent Application Publication No. 2005-0282233A1 and International Patent Application Publication No. WO 2005/087812.

Suitable methods of making antibodies are known in the art. For instance, standard hybridoma methods are described in, e.g., Harlow and Lane (eds.), Antibodies: A Laboratory Manual, CSH Press (1988), and CA. Janeway et al. (eds.), Immunobiology, 5th Ed., Garland Publishing, New York, NY (2001)).

Briefly, a polyclonal antibody is prepared by immunizing an animal with an immunogen comprising a polypeptide of the present invention and collecting antisera from that immunized animal. A wide range of animal species can be used for the production of antisera. In some aspects, an animal used for production of anti-antisera is a non-human animal including rabbits, mice, rats, hamsters, goat, sheep, pigs or horses. Because of the relatively large blood volume of rabbits, a rabbit is a preferred choice for production of polyclonal antibodies. In an exemplary method for generating a polyclonal antisera immunoreactive with the chosen MyBP-C, e.g., S-glutathionylated MyBP-C, a Cys residue of MyBP-C, an epitope within MyBP-C, a 20-30 amino acid portion of MyBP-C antigen is emulsified in Freund's Complete Adjuvant for immunization of rabbits. At intervals of, for example, 21 days, the epitope is emulsified in Freund's Incomplete Adjuvant for boosts. Polyclonal antisera may be obtained, after allowing time for antibody generation, simply by bleeding the animal and preparing serum samples from the whole blood.

Monoclonal antibodies for use in the invention may be prepared using any technique which provides for the production of antibody molecules by continuous cell lines in culture. These include but are not limited to the hybridoma technique originally described by Koehler and Milstein (Nature 256: 495-497, 1975), the human B-cell hybridoma technique (Kosbor et al., Immunol Today 4:72, 1983; Cote et al., Proc Natl Acad Sci 80: 2026-2030, 1983) and the EBV-hybridoma technique (Cole et al., Monoclonal Antibodies and Cancer Therapy, Alan R Liss Inc, New York N.Y., pp 77-96, (1985).

Briefly, in exemplary embodiments, to generate monoclonal antibodies, a mouse is injected periodically with MyBP-C, e.g., S-glutathionylated MyBP-C, a Cys residue of MyBP-C, an epitope within MyBP-C, against which the antibody is to be raised (e.g., 10-20 µg emulsified in Freund's Complete Adjuvant). The mouse is given a final pre-fusion boost of MyBP-C, e.g., S-glutathionylated MyBP-C, a Cys residue of MyBP-C, an epitope within MyBP-C containing the epitope that allows specific recognition of lymphatic endothelial cell in PBS, and four days later the mouse is sacrificed and its spleen removed. The spleen is placed in 10 ml serum-free RPMI 1640, and a single cell suspension is formed by grinding the spleen between the frosted ends of two glass microscope slides submerged in serum-free RPMI 1640, supplemented with 2 mM L-glutamine, 1 mM sodium pyruvate, 100 units/ml penicillin, and 100 µg/ml streptomycin (RPMI) (Gibco, Canada). The cell suspension is filtered through sterile 70-mesh Nitex cell strainer (Becton Dickinson, Parsippany, N.J.), and is washed twice by centrifuging at 200 g for 5 minutes and resuspending the pellet in 20 ml serum-free RPMI. Splenocytes taken from three naive Balb/c mice are prepared in a similar manner and used as a control. NS-1 myeloma cells, kept in log phase in RPMI with 11% fetal bovine serum (FBS) (Hyclone Laboratories, Inc., Logan, Utah) for three days prior to fusion, are centrifuged at 200 g for 5 minutes, and the pellet is washed twice.

Spleen cells (1 x 10⁸) are combined with 2.0 x 10⁷ NS-1 cells and centrifuged, and the supernatant is aspirated. The cell pellet is dislodged by tapping the tube, and 1 ml of 37 °C. PEG 1500 (50% in 75 mM Hepes, pH 8.0) (Boehringer Mannheim) is added with stirring over the course of 1 minute, followed by the addition of 7 ml of serum-free RPMI over 7 minutes. An additional 8 ml RPMI is added and the cells are centrifuged at 200 g for 10 minutes. After discarding the supernatant, the pellet is resuspended in 200 ml RPMI containing 15% FBS, 100 µM sodium hypoxanthine, 0.4 µM aminopterin, 16 µM thymidine (HAT) (Gibco), 25 units/ml IL-6 (Boehringer Mannheim) and 1.5 x 10⁶ splenocytes/ml and plated into 10 Corning flat-bottom 96-well tissue culture plates (Corning, Corning N.Y.).

On days 2, 4, and 6, after the fusion, 100 µl of medium is removed from the wells of the fusion plates and replaced with fresh medium. On day 8, the fusion is screened by ELISA, testing for the presence of mouse IgG binding to MyBP-C, e.g., S-glutathionylated MyBP-C, a Cys residue of MyBP-C, an epitope within MyBP-C.

Selected fusion wells are cloned twice by dilution into 96-well plates and visual scoring of the number of colonies/well after 5 days. The monoclonal antibodies produced by hybridomas are isotyped using the Isostrip system (Boehringer Mannheim, Indianapolis, Ind.).

When the hybridoma technique is employed, myeloma cell lines may be used. Such cell lines suited for use in hybridoma-producing fusion procedures preferably are non-antibody-producing, have high fusion efficiency, and enzyme deficiencies that render them incapable of growing in certain selective media which support the growth of only the desired fused cells (hybridomas). For example, where the immunized animal is a mouse, one may use P3-X63/Ag8, P3-X63-Ag8.653, NS1/1.Ag 4 1, Sp210-Ag14, FO, NSO/U, MPC-11, MPC11-X45-GTG 1.7 and S194/15XX0 Bul; for rats, one may use R210.RCY3, Y3-Ag 1.2.3, IR983F and 4B210; and U-266, GM1500-GRG2, LICR-LON-HMy2 and UC729-6 are all useful in connection with cell fusions. It should be noted that the hybridomas and cell lines produced by such techniques for producing the monoclonal antibodies are contemplated to be novel compositions of the present disclosures.

Depending on the host species, various adjuvants may be used to increase immunological response. Such adjuvants include but are not limited to Freund's, mineral gels such as aluminum hydroxide, and surface active substances such as lysolecithin, pluronic polyols, polyanions, peptides, oil emulsions, keyhole limpet hemocyanin, and dinitrophenol. BCG (bacilli Calmette-Guerin) and Corynebacterium parvum are potentially useful human adjuvants.

Alternatively, other methods, such as EBV-hybridoma methods (Haskard and Archer, J. Immunol. Methods, 74(2), 361-67 (1984),and Roder et al.5 Methods Enzymol., 121, 140-67 (1986)), and bacteriophage vector expression systems (see, e.g., Huse et al., Science, 246, 1275-81 (1989)) are known in the art. Further, methods of producing antibodies in non-human animals are described in, e.g., U.S. Patents 5,545,806, 5,569,825, and 5,714,352, and U.S. Patent Application Publication No. 2002/0197266 A1).

Antibodies may also be produced by inducing in vivo production in the lymphocyte population or by screening recombinant immunoglobulin libraries or panels of highly specific binding reagents as disclosed in Orlandi et al (Proc Natl Acad Sci 86: 3833-3837; 1989), and Winter G and Milstein C (Nature 349: 293-299, 1991).

Phage display furthermore can be used to generate the antibody of the present disclosures. In this regard, phage libraries encoding antigen-binding variable (V) domains of antibodies can be generated using standard molecular biology and recombinant DNA techniques (see, e.g., Sambrook et al. (eds.), Molecular Cloning, A Laboratory Manual, 3rd Edition, Cold Spring Harbor Laboratory Press, New York (2001)), Phage encoding a variable region with the desired specificity are selected for specific binding to the desired antigen, and a complete or partial antibody is reconstituted comprising the selected variable domain. Nucleic acid sequences encoding the reconstituted antibody are introduced into a suitable cell line, such as a myeloma cell used for hybridoma production, such that antibodies having the characteristics of monoclonal antibodies are secreted by the cell (see, e.g., Janeway et al., supra, Huse et al., supra, and U.S. Patent 6,265,150). Related methods also are described in U.S. Pat. No. 5,403,484; U.S. Pat. No. 5,571,698; U.S. Pat. No. 5,837,500; U.S. Pat. No. 5,702,892. The techniques described in U.S. Pat. No. 5,780,279; U.S. Pat. No. 5,821,047; U.S. Pat. No. 5,824,520; U.S. Pat. No. 5,855,885; U.S. Pat. No. 5,858,657; U.S. Pat. No. 5,871,907; U.S. Pat. No. 5,969,108; U.S. Pat. No. 6,057,098; U.S. Pat. No. 6,225,447,

Antibodies can be produced by transgenic mice that are transgenic for specific heavy and light chain immunoglobulin genes. Such methods are known in the art and described in, for example U.S. Patents 5,545,806 and 5,569,825, and Janeway et al., supra.

Methods for generating humanized antibodies are well known in the art and are described in detail in, for example, Janeway et al., supra, U.S. Patents 5,225,539, 5,585,089 and 5,693,761, European Patent No. 0239400 B1, and United Kingdom Patent No. 2188638. Humanized antibodies can also be generated using the antibody resurfacing technology described in U.S. Patent 5,639,641 and Pedersen et al., J. MoI. Biol, 235, 959-973 (1994).

Techniques developed for the production of "chimeric antibodies", the splicing of mouse antibody genes to human antibody genes to obtain a molecule with appropriate antigen specificity and biological activity, can be used (Morrison et al., Proc Natl Acad Sci 81: 6851-6855, 1984; Neuberger et al., Nature 312: 604-608, 1984; Takeda et al., Nature 314: 452-454; 1985). Alternatively, techniques described for the production of single chain antibodies (U.S. Pat. No. 4,946,778) can be adapted to produce VEGFR-3-specific single chain antibodies.

A preferred chimeric or humanized antibody has a human constant region, while the variable region, or at least a CDR, of the antibody is derived from a non-human species. Methods for humanizing non-human antibodies are well known in the art. (see U.S. Patent Nos. 5,585,089, and 5,693,762). Generally, a humanized antibody has one or more amino acid residues introduced into its framework region from a source which is non-human. Humanization can be performed, for example, using methods described in Jones et al. (Nature 321: 522-525, 1986), Riechmann et al., (Nature, 332: 323-327, 1988) and Verhoeyen et al. (Science 239:1534-1536, 1988), by substituting at least a portion of a rodent complementarity-determining region (CDRs) for the corresponding regions of a human antibody. Numerous techniques for preparing engineered antibodies are described, e.g., in Owens and Young, J. Immunol. Meth., 168:149-165 (1994). Further changes can then be introduced into the antibody framework to modulate affinity or immunogenicity.

Likewise, using techniques known in the art to isolate CDRs, compositions comprising CDRs are generated. Complementarity determining regions are characterized by six polypeptide loops, three loops for each of the heavy or light chain variable regions. The amino acid position in a CDR is defined by Kabat et al., "Sequences of Proteins of Immunological Interest," U.S. Department of Health and Human Services, (1983). For example, hypervariable regions of human antibodies are roughly defined to be found at residues 28 to 35, from 49-59 and from residues 92-103 of the heavy and light chain variable regions (Janeway and Travers, Immunobiology, 2nd Edition, Garland Publishing, New York, (1996)). The murine CDR also are found at approximately these amino acid residues. It is understood in the art that CDR regions may be found within several amino acids of these approximated residues set forth above. An immunoglobulin variable region also consists of four "framework" regions surrounding the CDRs (FR1-4). The sequences of the framework regions of different light or heavy chains are highly conserved within a species, and are also conserved between human and murine sequences.

Framework regions (FR) of a murine antibody are humanized by substituting compatible human framework regions chosen from a large database of human antibody variable sequences, including over twelve hundred human V_{H} sequences and over one thousand V_{L} sequences. The database of antibody sequences used for comparison is downloaded from Andrew C. R. Martin's KabatMan web page (http://www.rubic.rdg.ac.uk/abs/). The Kabat method for identifying CDR provides a means for delineating the approximate CDR and framework regions from any human antibody and comparing the sequence of a murine antibody for similarity to determine the CDRs and FRs. Best matched human V_{H} and V_{L} sequences are chosen on the basis of high overall framework matching, similar CDR length, and minimal mismatching of canonical and V_{H} / V_{L} contact residues. Human framework regions most similar to the murine sequence are inserted between the murine CDR. Alternatively, the murine framework region may be modified by making amino acid substitutions of all or part of the native framework region that more closely resemble a framework region of a human antibody.

"Conservative" amino acid substitutions are made on the basis of similarity in polarity, charge, solubility, hydrophobicity, hydrophilicity, and/or the amphipathic nature of the residues involved. For example, nonpolar (hydrophobic) amino acids include alanine (Ala, A), leucine (Leu, L), isoleucine (Ile, I), valine (Val, V), proline (Pro, P), phenylalanine (Phe, F), tryptophan (Trp, W), and methionine (Met, M); polar neutral amino acids include glycine (Gly, G), serine (Ser, S), threonine (Thr, T), cysteine (Cys, C), tyrosine (Tyr, Y), asparagine (Asn, N), and glutamine (Gln, Q); positively charged (basic) amino acids include arginine (Arg, R), lysine (Lys, K), and histidine (His, H); and negatively charged (acidic) amino acids include aspartic acid (Asp, D) and glutamic acid (Glu, E). "Insertions" or "deletions" are preferably in the range of about 1 to 20 amino acids, more preferably 1 to 10 amino acids. The variation may be introduced by systematically making substitutions of amino acids in a polypeptide molecule using recombinant DNA techniques and assaying the resulting recombinant variants for activity. Nucleic acid alterations can be made at sites that differ in the nucleic acids from different species (variable positions) or in highly conserved regions (constant regions). Methods for expressing polypeptide compositions useful in the invention are described in greater detail below.

Additionally, another useful technique for generating antibodies for use in the present invention may be one which uses a rational design type approach. The goal of rational design is to produce structural analogs of biologically active polypeptides or compounds with which they interact (agonists, antagonists, inhibitors, peptidomimetics, binding partners, etc.). In one approach, one would generate a three-dimensional structure for the antibodies or an epitope binding fragment thereof. This could be accomplished by x-ray crystallography, computer modeling or by a combination of both approaches. An alternative approach, "alanine scan," involves the random replacement of residues throughout molecule with alanine, and the resulting affect on function determined.

It also is possible to solve the crystal structure of the specific antibodies. In principle, this approach yields a pharmacore upon which subsequent drug design can be based. It is possible to bypass protein crystallography altogether by generating anti-idiotypic antibodies to a functional, pharmacologically active antibody. As a mirror image of a mirror image, the binding site of anti-idiotype would be expected to be an analog of the original antigen. The anti-idiotype could then be used to identify and isolate additional antibodies from banks of chemically- or biologically-produced peptides.

Chemically constructed bispecific antibodies may be prepared by chemically cross-linking heterologous Fab or F(ab')₂ fragments by means of chemicals such as heterobifunctional reagent succinimidyl-3-(2-pyridyldithiol)-propionate (SPDP, Pierce Chemicals, Rockford, Ill.). The Fab and F(ab')₂ fragments can be obtained from intact antibody by digesting it with papain or pepsin, respectively (Karpovsky et al., J. Exp. Med. 160:1686-701, 1984; Titus et al., J. Immunol., 138:4018-22, 1987).

Methods of testing antibodies for the ability to bind to the epitope of the MyBP-C, e.g., S-glutathionylated MyBP-C, a Cys residue of MyBP-C, an epitope within MyBP-C, regardless of how the antibodies are produced are known in the art and include any antibody-antigen binding assay, such as, for example, radioimmunoassay (RIA), ELISA, Western blot, immunoprecipitation, and competitive inhibition assays (see, e.g., Janeway et al., infra, and U.S. Patent Application Publication No. 2002/0197266 A1).

### Aptamers

In exemplary aspects, the binding agent of the invention is an aptamer. Recent advances in the field of combinatorial sciences have identified short polymer sequences (e.g., oligonucleic acid or peptide molecules) with high affinity and specificity to a given target. For example, SELEX technology has been used to identify DNA and RNA aptamers with binding properties that rival mammalian antibodies, the field of immunology has generated and isolated antibodies or antibody fragments which bind to a myriad of compounds and phage display has been utilized to discover new peptide sequences with very favorable binding properties. Based on the success of these molecular evolution techniques, it is certain that molecules can be created which bind to any target molecule. A loop structure is often involved with providing the desired binding attributes as in the case of: aptamers which often utilize hairpin loops created from short regions without complimentary base pairing, naturally derived antibodies that utilize combinatorial arrangement of looped hyper-variable regions and new phage display libraries utilizing cyclic peptides that have shown improved results when compared to linear peptide phage display results. Thus, sufficient evidence has been generated to suggest that high affinity ligands can be created and identified by combinatorial molecular evolution techniques. For the present disclosures, molecular evolution techniques can be used to isolate binding agents specific for MyBP-C, e.g., S-glutathionylated MyBP-C, a Cys residue of MyBP-C, an epitope within MyBP-C, described herein. For more on aptamers, see, generally, Gold, L., Singer, B., He, Y. Y., Brody. E., "Aptamers As Therapeutic And Diagnostic Agents," J. Biotechnol. 74:5-13 (2000). Relevant techniques for generating aptamers may be found in U.S. Pat. No. 6,699,843.

In some embodiments, the aptamer may be generated by preparing a library of nucleic acids; contacting the library of nucleic acids with a growth factor, wherein nucleic acids having greater binding affinity for the growth factor (relative to other library nucleic acids) are selected and amplified to yield a mixture of nucleic acids enriched for nucleic acids with relatively higher affinity and specificity for binding to the growth factor. The processes may be repeated, and the selected nucleic acids mutated and rescreened, whereby a growth factor aptamer is be identified. Nucleic acids may be screened to select for molecules that bind to more than target MyBP-C, e.g., S-glutathionylated MyBP-C, a Cys residue of MyBP-C, an epitope within MyBP-C. Binding more than one target MyBP-C, e.g., S-glutathionylated MyBP-C, a Cys residue of MyBP-C, an epitope within MyBP-C, can refer to binding more than one MyBP-C, e.g., S-glutathionylated MyBP-C, a Cys residue of MyBP-C, an epitope within MyBP-C, simultaneously or competitively.

### Epitopes

In exemplary embodiments, the binding agent of invention binds to an epitope of an MyBP-C, e.g., S-glutathionylated MyBP-C, a Cys residue of MyBP-C, an epitope within MyBP-C. By "epitope of an MyBP-C" as used herein is meant the region of or within the MyBP-C which is bound by the binding unit(s) of the binding agent. In some embodiments, the epitope is a linear epitope. By "linear epitope" as used herein refers to the region of or within the MyBP-C, which is bound by the binding unit(s) of the binding agent, which region is composed of contiguous amino acids of the amino acid sequence of the MyBP-C, e.g., S-glutathionylated MyBP-C, a Cys residue of MyBP-C, an epitope within MyBP-C. The amino acids of a linear epitope are located in close promity to each other in the primary structure of the antigen and the secondary and/or tertiary structure(s) of the antigen. For example, when the antigen, e.g., MyBP-C, e.g., S-glutathionylated MyBP-C, a Cys residue of MyBP-C, an epitope within MyBP-C, is in its properly folded state (e.g., its native conformation), the contiguous amino acids of the linear epitope are located in close proximity to one another.

In other exemplary aspects, the epitope of the binding agent is a conformational epitope. By "conformational epitope" is meant an epitope which is composed of amino acids which are located in close proximity to one another only when the MyBP-C is in its properly folded state, but are not contiguous amino acids of the amino acid sequence of the MyBP-C.

In exemplary aspects, the binding agent specifically binds to an epitope within MyBP-C and the epitope comprises one or more of Cys 239, Cys249, Cys 426, Cys 436, Cys 443, Cys 475 , Cys528, Cys566, Cys 623, Cys 651, and/or Cys 719, according to the amino acid position numbering of SEQ ID NO: 21. In exemplary aspects, one or more of Cys 239, Cys249, Cys 426, Cys 436, Cys 443, Cys 475 , Cys528, Cys566, Cys 623, Cys 651, and/or Cys 719 is/are S-glutathionylated, and the binding agent specifically binds to the S-glutathionylated form of MyBP-C comprising the one or more Cys residues, or an S-glutathionylated fragment thereof

In exemplary aspects, the binding agent specifically binds to an epitope within MyBP-C and the epitope comprises one or more of Cys 475, Cys 623, and/or Cys 651, according to the amino acid position numbering of SEQ ID NO: 21. In exemplary aspects, one or more of Cys 475, Cys 623, and/or Cys 651 is/are S-glutathionylated, and the binding agent specifically binds to the S-glutathionylated form of MyBP-C comprising the one or more Cys residues, or an S-glutathionylated fragment thereof

In exemplary aspects, the binding agent binds to an S-glutathionylated MyBP-C. In some aspects, the binding agent specifically binds to the S-glutathionylated form of MyBP-C, and not the un-glutathionylated form of MyBP-C. In some aspects, the S-glutathionylation occurs at one or more of Cys 239, Cys249, Cys 426, Cys 436, Cys 443, Cys 475 , Cys528, Cys566, Cys 623, Cys 651, and/or Cys 719 of MyBP-C, wherein the positions of the Cys residues are in accordance with the amino acid position numbering of SEQ ID NO: 21. In some aspects, the S-glutathionylation occurs at one or more of Cys 475, Cys 623, and/or Cys 651 of MyBP-C, wherein the positions of the Cys residues are in accordance with the amino acid position numbering of SEQ ID NO: 21.

In exemplary aspects, the binding agent specifically binds to one of Cys 239, Cys249, Cys 426, Cys 436, Cys 443, Cys 475 , Cys528, Cys566, Cys 623, Cys 651, and/or Cys 719 of MyBP-C, wherein the positions of the Cys residues are in accordance with the amino acid position numbering of SEQ ID NO: 21. In exemplary aspects, the binding agent specifically binds to one of Cys 475, Cys 623, and/or Cys 651 of MyBP-C, wherein the positions of the Cys residues are in accordance with the amino acid position numbering of SEQ ID NO: 21. In some aspects, the binding agent is considered a "site-specific antibody." In exemplary aspects, one or more of Cys 239, Cys249, Cys 426, Cys 436, Cys 443, Cys 475, Cys528, Cys566, Cys 623, Cys 651, and/or Cys 719 of MyBP-C are S-glutathionylated, and the binding agent specifically binds to the S-glutathionylated form of MyBP-C comprising the one or more Cys residues, or an S-glutathionylated fragment thereof. In exemplary aspects, one or more of Cys 475, Cys 623, and/or Cys 651 of MyBP-C are S-glutathionylated, and the binding agent specifically binds to the S-glutathionylated form of MyBP-C comprising the one or more Cys residues, or an S-glutathionylated fragment thereof. In some aspects, the binding agent is a site- and glutathionylated-specific antibody of MyBP-C, which does not recognize other sites on MyBP-C, and does not recognize the un-glutationylated site.

In exemplary aspects, the binding agent is an antibody, antigen binding fragment of an antibody, or an aptamer, such as any of those described herein.

### Conjugates

In exemplary embodiments, the binding agent is conjugated to a second moiety or a heterologous moiety. As used herein, the term "heterologous moiety" is synonymous with the term "conjugate moiety" and refers to any molecule (chemical or biochemical, naturally-occurring or non-coded) which is different from the binding agents of the invention described herein. Exemplary conjugate moieties that can be linked to any of the binding agents described herein include but are not limited to a heterologous peptide or polypeptide (including for example, an enzyme, an enzyme substrate, a binding partner of a high affinity binding interaction between two binding partners), a targeting agent, a heterologous immunoglobulin or portion thereof (e.g.,variable region, CDR, or Fc region), a diagnostic or detectable label such as a radioisotope, fluorophore or enzymatic label, a polymer including water soluble polymers, or other therapeutic or diagnostic agents. In some embodiments, the invention provides a conjugate comprising an inventive binding agent linked to a polypeptide, a nucleic acid molecule, an antibody or fragment thereof, a polymer, a quantum dot, a small molecule, a toxin, a diagnostic agent, a carbohydrate, an amino acid.

In some embodiments, the heterologous moiety is a polymer. In some embodiments, the polymer is selected from the group consisting of: polyamides, polycarbonates, polyalkylenes and derivatives thereof including, polyalkylene glycols, polyalkylene oxides, polyalkylene terepthalates, polymers of acrylic and methacrylic esters, including poly(methyl methacrylate), poly(ethyl methacrylate), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate), polyvinyl polymers including polyvinyl alcohols, polyvinyl ethers, polyvinyl esters, polyvinyl halides, poly(vinyl acetate), and polyvinylpyrrolidone, polyglycolides, polysiloxanes, polyurethanes and co-polymers thereof, celluloses including alkyl cellulose, hydroxyalkyl celluloses, cellulose ethers, cellulose esters, nitro celluloses, methyl cellulose, ethyl cellulose, hydroxypropyl cellulose, hydroxy-propyl methyl cellulose, hydroxybutyl methyl cellulose, cellulose acetate, cellulose propionate, cellulose acetate butyrate, cellulose acetate phthalate, carboxylethyl cellulose, cellulose triacetate, and cellulose sulphate sodium salt, polypropylene, polyethylenes including poly(ethylene glycol), poly(ethylene oxide), and poly(ethylene terephthalate), and polystyrene.

In some aspects, the polymer is a biodegradable polymer, including a synthetic biodegradable polymer (e.g., polymers of lactic acid and glycolic acid, polyanhydrides, poly(ortho)esters, polyurethanes, poly(butic acid), poly(valeric acid), and poly(lactide-cocaprolactone)), and a natural biodegradable polymer (e.g., alginate and other polysaccharides including dextran and cellulose, collagen, chemical derivatives thereof (substitutions, additions of chemical groups, for example, alkyl, alkylene, hydroxylations, oxidations, and other modifications routinely made by those skilled in the art), albumin and other hydrophilic proteins (e.g., zein and other prolamines and hydrophobic proteins)), as well as any copolymer or mixture thereof. In general, these materials degrade either by enzymatic hydrolysis or exposure to water *in vivo,* by surface or bulk erosion.

In some aspects, the polymer is a bioadhesive polymer, such as a bioerodible hydrogel described by H. S. Sawhney, C. P. Pathak and J. A. Hubbell in Macromolecules, 1993, 26, 581-587 polyhyaluronic acids, casein, gelatin, glutin, polyanhydrides, polyacrylic acid, alginate, chitosan, poly(methyl methacrylates), poly(ethyl methacrylates), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate).

In some embodiments, the polymer is a water-soluble polymer or a hydrophilic polymer. Hydrophilic polymers are further described herein under "Hydrophilic Moieties." Suitable water-soluble polymers are known in the art and include, for example, polyvinylpyrrolidone, hydroxypropyl cellulose (HPC; Klucel), hydroxypropyl methylcellulose (HPMC; Methocel), nitrocellulose, hydroxypropyl ethylcellulose, hydroxypropyl butylcellulose, hydroxypropyl pentylcellulose, methyl cellulose, ethylcellulose (Ethocel), hydroxyethyl cellulose, various alkyl celluloses and hydroxyalkyl celluloses, various cellulose ethers, cellulose acetate, carboxymethyl cellulose, sodium carboxymethyl cellulose, calcium carboxymethyl cellulose, vinyl acetate/crotonic acid copolymers, poly-hydroxyalkyl methacrylate, hydroxymethyl methacrylate, methacrylic acid copolymers, polymethacrylic acid, polymethylmethacrylate, maleic anhydride/methyl vinyl ether copolymers, poly vinyl alcohol, sodium and calcium polyacrylic acid, polyacrylic acid, acidic carboxy polymers, carboxypolymethylene, carboxyvinyl polymers, polyoxyethylene polyoxypropylene copolymer, polymethylvinylether co-maleic anhydride, carboxymethylamide, potassium methacrylate divinylbenzene co-polymer, polyoxyethyleneglycols, polyethylene oxide, and derivatives, salts, and combinations thereof.

In specific embodiments, the polymer is a polyalkylene glycol, including, for example, polyethylene glycol (PEG).

In some embodiments, the heterologous moiety is a carbohydrate. In some embodiments, the carbohydrate is a monosaccharide (e.g., glucose, galactose, fructose), a disaccharide (e.g., sucrose, lactose, maltose), an oligosaccharide (e.g., raffinose, stachyose), or a polysaccharide (e.g., starch, amylase, amylopectin, cellulose, chitin, callose, laminarin, xylan, mannan, fucoidan, or galactomannan).

In some embodiments, the heterologous moiety is a lipid. The lipid, in some embodiments, is a fatty acid, eicosanoid, prostaglandin, leukotriene, thromboxane, N-acyl ethanolamine), glycerolipid (e.g., mono-, di-, tri-substituted glycerols), glycerophospholipid (e.g., phosphatidylcholine, phosphatidylinositol, phosphatidylethanolamine, phosphatidylserine), sphingolipid (e.g., sphingosine, ceramide), sterol lipid (e.g., steroid, cholesterol), prenol lipid, saccharolipid, or a polyketide, oil, wax, cholesterol, sterol, fat-soluble vitamin, monoglyceride, diglyceride, triglyceride, a phospholipid.

In some embodiments, the heterologous moiety is a polypeptide or peptide, optionally, an enzyme, enzyme substrate, or a binding protein of a known binding interaction. In exemplary aspects, the heterologous moiety is horseradish peroxidase, the substrate thereof, avidin, biotin, a radioactive label or a fluorescent label. The fluorescent label in some aspects is isothiocyanate, fluorescein, rhodamine, alexa fluors, dylight fluors, ATTO dyes, BODIPY dyes, and the like.

In some embodiments, the heterologous moiety is attached via non-covalent or covalent bonding to the binding agent of the invention. In exemplary aspects, the heterologous moiety is attached to the binding agent of the invention via a linker. Linkage can be accomplished by covalent chemical bonds, physical forces such electrostatic, hydrogen, ionic, van der Waals, or hydrophobic or hydrophilic interactions. A variety of non-covalent coupling systems may be used, including biotin-avidin, ligand/receptor, enzyme/substrate, nucleic acid/nucleic acid binding protein, lipid/lipid binding protein, cellular adhesion molecule partners; or any binding partners or fragments thereof which have affinity for each other.

The binding agent in some embodiments is linked to conjugate moieties via direct covalent linkage by reacting targeted amino acid residues of the binding agent with an organic derivatizing agent that is capable of reacting with selected side chains or the N- or C-terminal residues of these targeted amino acids. Reactive groups on the binding agent or conjugate moiety include, e.g., an aldehyde, amino, ester, thiol, α-haloacetyl, maleimido or hydrazino group. Derivatizing agents include, for example, maleimidobenzoyl sulfosuccinimide ester (conjugation through cysteine residues), N-hydroxysuccinimide (through lysine residues), glutaraldehyde, succinic anhydride or other agents known in the art. Alternatively, the conjugate moieties can be linked to the binding agent indirectly through intermediate carriers, such as polysaccharide or polypeptide carriers. Examples of polysaccharide carriers include aminodextran. Examples of suitable polypeptide carriers include polylysine, polyglutamic acid, polyaspartic acid, co-polymers thereof, and mixed polymers of these amino acids and others, e.g., serines, to confer desirable solubility properties on the resultant loaded carrier.

Cysteinyl residues are most commonly reacted with α-haloacetates (and corresponding amines), such as chloroacetic acid, chloroacetamide to give carboxymethyl or carboxyamidomethyl derivatives. Cysteinyl residues also are derivatized by reaction with bromotrifluoroacetone, alpha-bromo-β-(5-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylpyrocarbonate at pH 5.5-7.0 because this agent is relatively specific for the histidyl side chain. Para-bromophenacyl bromide also is useful; the reaction is preferably performed in 0.1 M sodium cacodylate at pH 6.0.

Lysinyl and amino-terminal residues are reacted with succinic or other carboxylic acid anhydrides. Derivatization with these agents has the effect of reversing the charge of the lysinyl residues. Other suitable reagents for derivatizing alpha-amino-containing residues include imidoesters such as methyl picolinimidate, pyridoxal phosphate, pyridoxal, chloroborohydride, trinitrobenzenesulfonic acid, O-methylisourea, 2,4-pentanedione, and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high pKa of the guanidine functional group. Furthermore, these reagents may react with the groups of lysine as well as the arginine epsilon-amino group.

The specific modification of tyrosyl residues may be made, with particular interest in introducing spectral labels into tyrosyl residues by reaction with aromatic diazonium compounds or tetranitromethane. Most commonly, N-acetylimidizole and tetranitromethane are used to form O-acetyl tyrosyl species and 3-nitro derivatives, respectively.

Carboxyl side groups (aspartyl or glutamyl) are selectively modified by reaction with carbodiimides (R-N=C=N-R'), where R and R' are different alkyl groups, such as 1-cyclohexyl-3-(2-morpholinyl-4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.

Other modifications include hydroxylation of proline and lysine, phosphorylation of hydroxyl groups of seryl or threonyl residues, methylation of the alpha-amino groups of lysine, arginine, and histidine side chains (T. E. Creighton, Proteins: Structure and Molecular Properties, W.H. Freeman & Co., San Francisco, pp. 79-86 (1983)), deamidation of asparagine or glutamine, acetylation of the N-terminal amine, and/or amidation or esterification of the C-terminal carboxylic acid group.

Another type of covalent modification involves chemically or enzymatically coupling glycosides to the binding agent. Sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of tyrosine, or tryptophan, or (f) the amide group of glutamine. These methods are described in WO87/05330 published 11 Sep. 1987, and in Aplin and Wriston, CRC Crit. Rev. Biochem., pp. 259-306 (1981).

### Multimers

The invention further provides multimers or dimers of the binding agents disclosed herein, including homo- or hetero- multimers or homo- or hetero- dimers. Two or more of the binding agents can be linked together using standard linking agents and procedures known to those skilled in the art. For example, dimers can be formed between two binding agents through the use of bifunctional thiol crosslinkers and bi-functional amine crosslinkers, particularly for the binding agents that have been substituted with cysteine, lysine ornithine, homocysteine or acetyl phenylalanine residues. The dimer can be a homodimer or alternatively can be a heterodimer. In some aspects, the monomers are connected via terminal amino acids (e.g., N-terminal or C-terminal), via internal amino acids, or via a terminal amino acid of at least one monomer and an internal amino acid of at least one other monomer. In specific aspects, the monomers are not connected via an N-terminal amino acid. In some aspects, the monomers of the multimer are attached together in a "tail-to-tail" orientation in which the C-terminal amino acids of each monomer are attached together.

### Systems, Computer-Readable Storage Media, and Methods Implemented by a Computer Processor

Fig. 12 illustrates an exemplary embodiment 101 of a system 100 for assessing a subject's need for a therapeutic agent for diastolic dysfunction of a diastolic heart failure. Generally, the system 100 may include one or more client devices 102, a network 104, and a database 108. Each client device 102 may be communicatively coupled to the network 104 by one or more wired or wireless network connections 112, which may be, for example, a connection complying with a standard such as one of the IEEE 802.11 standards ("Wi-Fi"), the Ethernet standard, or any other appropriate network connection. Similarly, the database 108 may be communicatively coupled to the network 104 via one or more connections 114. (Of course, the database could alternatively be internal to one or more of the client devices 102.) The database 108 may store data related to the determination of a subject's need for an a therapeutic agent for diastolic dysfunction of a diastolic heart failure including, but not limited to, data of a biological sample obtained from the subject, data of a biological sample obtained from a control or test population, data of a Gaussian distribution associated with the data of the biological samples, data of one or more threshold values associated with the data of the biological sample(s) and/or Gaussian distribution, etc. The data of the biological samples may be, for example, related to one or more of a level of MyBP-C, a post-translationally modified form thereof, or a fragment thereof, or a level of ADMA, SDMA, or L-Arginine, etc., as described in greater detail below.

As will be understood, the network 104 may be a local area network (LAN) or a wide-area network (WAN). That is, network 104 may include only local (e.g., intra-organization) connections or, alternatively, the network 104 may include connections extending beyond the organization and onto one or more public networks (e.g., the Internet). In some embodiments, for example, the client device 102 and the database 108 may be within the network operated by a single company (Company A). In other embodiments, for example, the client device(s) 102 may be on a network operated by Company A, while the database 108 may be on a network operated by a second company (Company B), and the networks of Company A and Company B may be coupled by a third network such as, for example, the Internet.

Referring still to Fig. 12, the client device 102 includes a processor 128 (CPU), a RAM 130, and a non-volatile memory 132. The non-volatile memory 132 may be any appropriate memory device including, by way of example and not limitation, a magnetic disk (e.g., a hard disk drive), a solid state drive (e.g., a flash memory), etc. Additionally, it will be understood that, at least with regard to Fig. 12, the database 108 need not be separate from the client device 102. Instead, in some embodiments, the database 108 is part of the non-volatile memory 132 and the data 122, 124, 126 may be stored as data within the memory 132. For example, the data 122 may be included as data in a spreadsheet file stored in the memory 132, instead of as data in the database 108. In addition to storing the records of the database 108 (in some embodiments), the memory 132 stores program data and other data necessary to analyze data of one or more sample and/or control populations, determine a Gaussian fit for the data, determine a threshold against which data of the subject may be compared, and/or determine a subject's need for a therapeutic agent for diastolic dysfunction of a diastolic heart failure. For example, in an embodiment, the memory 132 stores a first routine 134, a second routine 136, and a third routine 138. The first routine 134 may receive data values related to one or more sample and/or control populations, and may fit the data values received by the routine 134 to a Gaussian distribution. The second routine 136 may computer one or more statistical parameters of the data collected by the first routine 134, such as determining a mean value, a standard deviation value, etc. of the Gaussian distribution. Additionally and/or alternatively, the second routine 136 may set a first threshold against which data from one or more subjects may be compared in order to determine whether each subject should receive an a therapeutic agent for diastolic dysfunction of a diastolic heart failure. The third routine 138 may, for example, receive data for one or more subjects, compare the data of the one or more subjects to the threshold value(s) determined by the second routine 136, and/or determine whether each subject should receive a therapeutic agent for diastolic dysfunction of a diastolic heart failure according to the comparison of the subject's data to the threshold value. Regardless, each of the routines is executable by the processor 128 and comprises a series of compiled or compilable machine-readable instructions stored in the memory 132. Additionally, the memory 132 may store generated reports or records of data output by one of the routines 134 or 136. Alternatively, the reports or records may be output to the database 108. One or more display/output devices 140 (e.g., printer, display, etc.) and one or more input devices 142 (e.g., mouse, keyboard, tablet, touch-sensitive interface, etc.) may also be coupled to the client device 102, as is generally known.

As will be understood, although individual operations of one or more methods are illustrated and described as separate operations, one or more of the individual operations may be performed concurrently, and nothing requires that the operations be performed in the order illustrated. Structures and functionality presented as separate components in example configurations may be implemented as a combined structure or component. Similarly, structures and functionality presented as a single component may be implemented as separate components. These and other variations, modifications, additions, and improvements fall within the scope of the subject matter herein.

For example, the network 104 may include but is not limited to any combination of a LAN, a MAN, a WAN, a mobile, a wired or wireless network, a private network, or a virtual private network. Moreover, while only two clients 102 are illustrated in Fig. 12 to simplify and clarify the description, it is understood that any number of client computers are supported and can be in communication with one or more servers (not shown).

Additionally, certain embodiments are described herein as including logic or a number of routines. Routines may constitute either software routines (e.g., code embodied on a machine-readable medium or in a transmission signal) or hardware routines. A hardware routine is tangible unit capable of performing certain operations and may be configured or arranged in a certain manner. In example embodiments, one or more computer systems (e.g., a standalone, client or server computer system) or one or more hardware routines of a computer system (e.g., a processor or a group of processors) may be configured by software (e.g., an application or application portion) as a hardware routine that operates to perform certain operations as described herein.

Similarly, the methods or routines described herein may be at least partially processor-implemented. For example, at least some of the operations of a method may be performed by one or processors or processor-implemented hardware modules. The performance of certain of the operations may be distributed among the one or more processors, not only residing within a single machine, but deployed across a number of machines. In some example embodiments, the processor or processors may be located in a single location (e.g., within a home environment, an office environment or as a server farm), while in other embodiments the processors may be distributed across a number of locations.

The performance of certain of the operations may be distributed among the one or more processors, not only residing within a single machine, but deployed across a number of machines. In some example embodiments, the one or more processors or processor-implemented modules may be located in a single geographic location (e.g., within a home environment, an office environment, or a server farm). In other example embodiments, the one or more processors or processor-implemented modules may be distributed across a number of geographic locations.

Some embodiments may be described using the expression "coupled" and "connected" along with their derivatives. For example, some embodiments may be described using the term "coupled" to indicate that two or more elements are in direct physical or electrical contact. The term "coupled," however, may also mean that two or more elements are not in direct contact with each other, but yet still co-operate or interact with each other. The embodiments are not limited in this context.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In addition, use of the "a" or "an" are employed to describe elements and components of the embodiments herein. This is done merely for convenience and to give a general sense of the description. This description should be read to include one or at least one and the singular also includes the plural unless it is obvious that it is meant otherwise.

Still further, the figures depict preferred embodiments of a map editor system for purposes of illustration only. One skilled in the art will readily recognize from the following discussion that alternative embodiments of the structures and methods illustrated herein may be employed without departing from the principles described herein

Upon reading this disclosure, those of skill in the art will appreciate still additional alternative structural and functional designs for a system and a process for identifying terminal road segments through the disclosed principles herein. Thus, while particular embodiments and applications have been illustrated and described, it is to be understood that the disclosed embodiments are not limited to the precise construction and components disclosed herein. Various modifications, changes and variations, which will be apparent to those skilled in the art, may be made in the arrangement, operation and details of the method and apparatus disclosed herein without departing from the spirit and scope defined in the appended claims.

The invention provides systems comprising: a processor; a memory device coupled to the processor, and machine readable instructions stored on the memory device. In exemplary embodiments, the machine readable instructions, when executed by the processor, cause the processor to
(i) receive a data value selected from the group consisting of: (a) a level of a MyBP-C, (b) a level of a post-translationally modified form of MyBP-C, or (c) a level of a MyBP-C fragment, determined from a biological sample obtained from a subject,
(ii) compare the data value of (i) to a corresponding control data value, wherein the corresponding control data value is the mean of a plurality of control data values, each of which is a level of (a) a MyBP-C, (b) a post-translationally modified form of MyBP-C, or (c) a MyBP-C fragment, determined from a biological sample obtained from a subject known to not suffer from heart failure, and
(iii) provide an output diagnosis of diastolic dysfunction or diastolic heart failure in the subject, when the data value of (i) is greater than the corresponding control data value.

In exemplary aspects, the processor is able to receive a plurality of control data values, each of which is a level of (a) a MyBP-C, (b) a post-translationally modified form of MyBP-C, or (c) a MyBP-C fragment, determined from a biological sample obtained from a subject known to not suffer from heart failure. The processor may then determine a mean value of the plurality of control data values, and assign the mean value as the corresponding control data value. In exemplary aspects, the processor assigns each control data value of the plurality as a control data value. In exemplary aspects, the processor calculates or re-calculates the mean value each time a new control data value is received. In exemplary aspects, the processor stores each control data value and each mean value on the memory device.

In exemplary aspects, the processor assigns the data value of (i) as a diastolic dysfunction data value, when an output diagnosis of diastolic dysfunction in the subject is provided, and stores the diastolic dysfunction data value on the memory device.

In alternative or additional aspects, the system comprises machine readable instructions that, when executed by the processor, cause the processor to:
(i) receive a first data value and a second data value, wherein the first data value is a level of asymmetric dimethylarginine (ADMA) determined from a biological sample obtained from a subject, and the second data value is a level of symmetric dimethylarginine (SDMA) determined from the biological sample obtained from the subject,
(ii) compare the first data value of (i) to a control ADMA data value, wherein the control ADMA data value is the mean of a plurality of control data values, each of which is a level of ADMA determined from a biological sample obtained from a subject known to not suffer from heart failure,
(iii) compare the second data value of (i) to a control SDMA data value, wherein the control SDMA data value is the mean of a plurality of control data values, each of which is a level of SDMA determined from a biological sample obtained from a subject known to not suffer from heart failure.

In exemplary aspects, (i) the processor is able to receive a plurality of control data values, each of which is a level of ADMA determined from a biological sample obtained from a subject known to not suffer from heart failure, determine a mean value of the plurality of control data values, and assign the mean value as the control ADMA data value, (ii) the processor is able to receive a plurality of control data values, each of which is a level of SDMA determined from a biological sample obtained from a subject known to not suffer from heart failure, determine a mean value of the plurality of control data values, and assign the mean value as the control SDMA data value, or (iii) a combination thereof

In exemplary aspects, the processor assigns each control data value (which is a level of ADMA determined from a biological sample obtained from a subject known to not suffer from heart failure) of the plurality as a control data value (ADMA). In exemplary aspects, the processor calculates or re-calculates the mean value as the control ADMA data value each time a new control data value (ADMA) is received. In exemplary aspects, the processor stores each control data value (ADMA) and each mean value (control ADMA data value) on the memory device.

In exemplary aspects, the processor assigns each control data value (which is a level of SDMA determined from a biological sample obtained from a subject known to not suffer from heart failure) of the plurality as a control data value (SDMA). In exemplary aspects, the processor calculates or re-calculates the mean value as the control ADMA data value each time a new control data value (SDMA) is received. In exemplary aspects, the processor stores each control data value (SDMA) and each mean value (control SDMA data value) on the memory device.

In alternative or additional aspects, the system comprises machine readable instructions that, when executed by the processor, cause the processor to:
(i) receive a first data value and a second data value, wherein the first data value is a level of asymmetric dimethylarginine (ADMA) determined from a biological sample obtained from a subject, and the second data value is a level of L-Arginine (L-Arg) determined from the biological sample obtained from the subject,
(ii) compare the ratio of the second data value to the first data value (Rt) to a control ratio (Rc), wherein Rc is the mean of a plurality of control ratios, each of which is a ratio of a level of L-Arginine determined from a biological sample obtained from a subject known to not suffer from heart failure to a level of ADMA determined from a biological sample obtained from a subject known to not suffer from heart failure, and
(iii) provide an output diagnosis of diastolic dysfunction or diastolic heart failure in the subject, when Rₜ is less than R_{c}.

In exemplary aspects, the processor is able to receive a plurality of control ratios, each of which is a ratio of a level of L-Arginine determined from a biological sample obtained from a subject known to not suffer from heart failure to a level of ADMA determined from a biological sample obtained from a subject known to not suffer from heart failure. In exemplary aspects, the processor assigns each control ratio as a control ratio. In exemplary aspects, the processor calculates or re-calculates the mean control ratio each time a new control ratio is received. In exemplary aspects, the processor stores each control ratio and each mean control ratio on the memory device.

In exemplary aspects, the processor assigns Rₜ as a diastolic dysfunction ratio, when an output diagnosis of diastolic dysfunction in the subject is provided, and stores the diastolic dysfunction ratio on the memory device.

Also provided herein are computer-readable storage media having stored thereon machine-readable instructions executable by a processor. In exemplary embodiments, the machine-readable instructions are any of the aforementioned instructions of the inventive system. In exemplary embodiments, the instructions comprise:
(i) instructions for causing the processor to receive a data value selected from the group consisting of: (a) a level of a MyBP-C, (b) a level of a post-translationally modified form of MyBP-C, or (c) a level of a MyBP-C fragment, determined from a biological sample obtained from a subject;
(ii) instructions for causing the processor to compare the data value of (i) to a corresponding control data value, wherein the corresponding control data value is the mean of a plurality of control data values, each of which is a level of (a) a MyBP-C, (b) a post-translationally modified form of MyBP-C, or (c) a MyBP-C fragment, determined from a biological sample obtained from a subject known to not suffer from heart failure, and
(iii) instructions for causing the processor to provide an output diagnosis of diastolic dysfunction or diastolic heart failure in the subject, when the data value of (i) is greater than the corresponding control data value.

In exemplary aspects, the computer-readable storage medium comprises instructions for causing the processor to receive a plurality of control data values, each of which is a level of (a) a MyBP-C, (b) a post-translationally modified form of MyBP-C, or (c) a MyBP-C fragment, determined from a biological sample obtained from a subject known to not suffer from heart failure, determine a mean value of the plurality of control data values, and assign the mean value as the corresponding control data value. In exemplary aspects, the computer-readable storage medium comprises instructions for causing the processor to assign the data value of (i) as a diastolic dysfunction data value, when an output diagnosis of diastolic dysfunction in the subject is provided, and stores the diastolic dysfunction data value on the memory device.

In alternative or additional embodiments, the computer-readable storage medium comprises instructions for causing the processor to:
(i) receive a first data value and a second data value, wherein the first data value is a level of asymmetric dimethylarginine (ADMA) determined from a biological sample obtained from a subject, and the second data value is a level of symmetric dimethylarginine (SDMA) determined from the biological sample obtained from the subject;
(ii) compare the first data value of (i) to a control ADMA data value, wherein the control ADMA data value is the mean of a plurality of control data values, each of which is a level of ADMA determined from a biological sample obtained from a subject known to not suffer from heart failure,
(iii) compare the second data value of (i) to a control SDMA data value, wherein the control SDMA data value is the mean of a plurality of control data values, each of which is a level of SDMA determined from a biological sample obtained from a subject known to not suffer from heart failure, and
(iv) provide an output diagnosis of diastolic dysfunction or diastolic heart failure in the subject, when the first data value of (i) is greater than the control ADMA data value and when the second data value of (i) is approximately the same as the control SDMA data value.

In exemplary aspects, the computer-readable storage medium comprises instructions for causing the processor to receive a plurality of control data values, each of which is a level of ADMA determined from a biological sample obtained from a subject known to not suffer from heart failure, determine a mean value of the plurality of control data values, and assign the mean value as the control ADMA data value, (ii) the processor is able to receive a plurality of control data values, each of which is a level of SDMA determined from a biological sample obtained from a subject known to not suffer from heart failure, determine a mean value of the plurality of control data values, and assign the mean value as the control SDMA data value, or (iii) a combination thereof.

In alternative or additional embodiments, the computer-readable storage medium comprises instructions for causing the processor to:
(i) receive a third data value which is a level of L-Arginine (L-Arg) determined from the biological sample obtained from the subject,
(ii) compare the ratio of the third data value to the first data value (Rt) to a control ratio (R_{c}), wherein R_{c} is the mean of a plurality of control ratios, each of which is a ratio of a level of L-Arginine determined from a biological sample obtained from a subject known to not suffer from heart failure to a level of ADMA determined from a biological sample obtained from a subject known to not suffer from heart failure, and
(iii) provide an output diagnosis of diastolic dysfunction or diastolic heart failure in the subject, when Rₜ is less than R_{c}.

In exemplary aspects, the computer-readable storage medium comprises instructions for causing the processor to assign Rₜ as a diastolic dysfunction ratio, when an output diagnosis of diastolic dysfunction in the subject is provided, and stores the diastolic dysfunction ratio on the memory device.

Further provided herein are methods implemented by a processor in a computer. In exemplary embodiments, the method comprises the steps of:
(i) receiving a data value selected from the group consisting of: (a) a level of a MyBP-C, (b) a level of a post-translationally modified form of MyBP-C, or (c) a level of a MyBP-C fragment, determined from a biological sample obtained from a subject,
(ii) comparing the data value of (i) to a corresponding control data value, wherein the corresponding control data value is the mean of a plurality of control data values, each of which is a level of (a) a MyBP-C, (b) a post-translationally modified form of MyBP-C, or (c) a MyBP-C fragment, determined from a biological sample obtained from a subject known to not suffer from heart failure, and
(iii) providing an output diagnosis of diastolic dysfunction or diastolic heart failure in the subject, when the data value of (i) is greater than the corresponding control data value.

In exemplary embodiments, the method comprises the steps of:
(i) receiving a first data value and a second data value, wherein the first data value is a level of asymmetric dimethylarginine (ADMA) determined from a biological sample obtained from a subject, and the second data value is a level of symmetric dimethylarginine (SDMA) determined from the biological sample obtained from the subject,
(ii) comparing the first data value of (i) to a control ADMA data value, wherein the control ADMA data value is the mean of a plurality of control data values, each of which is a level of ADMA determined from a biological sample obtained from a subject known to not suffer from heart failure,
(iii) comparing the second data value of (i) to a control SDMA data value, wherein the control SDMA data value is the mean of a plurality of control data values, each of which is a level of SDMA determined from a biological sample obtained from a subject known to not suffer from heart failure, and
(iv) providing an output diagnosis of diastolic dysfunction or diastolic heart failure in the subject, when the first data value of (i) is greater than the control ADMA data value and when the second data value of (i) is approximately the same as the control SDMA data value.

In alternative or additional embodiments, the method comprises the steps of:
(i) receiving a first data value and a second data value, wherein the first data value is a level of asymmetric dimethylarginine (ADMA) determined from a biological sample obtained from a subject, and the second data value is a level of L-Arginine (L-Arg) determined from the biological sample obtained from the subject,
(ii) comparing the ratio of the second data value to the first data value (Rt) to a control ratio (Rc), wherein Rc is the mean of a plurality of control ratios, each of which is a ratio of a level of L-Arginine determined from a biological sample obtained from a subject known to not suffer from heart failure to a level of ADMA determined from a biological sample obtained from a subject known to not suffer from heart failure, and
(iii) providing an output diagnosis of diastolic dysfunction or diastolic heart failure in the subject, when Rt is less than Rc.

In exemplary aspects of the inventive systems, computer-readable storage medium, and methods implemented by a processor in a computer, the post-translationally modified form of MyBP-C is S-glutathionylated MyBP-C or an S-glutathionylated fragment of MyBP-C.

### Kits

Provided herein are kits, e.g., diagnostic kits, comprising a binding agent or substrate specific for MyBP-C, a post-translationally modified form or fragment thereof. In exemplary aspects, the post-translationally modified form of MyBP-C is S-glutathionylated MyBP-C or an S-glutathionylated fragment of MyBP-C. In some aspects, wherein the binding agent does not specifically recognize the S-glutationylated form of MyBP-C or an S-glutationylated MyBP-C fragment, the kit may comprise a glutathione specific binding agent. In some aspects, the binding agent is an antibody, antigen binding fragment, an aptamer, a peptide, or a nucleic acid probe. In exemplary aspects, the binding agent is one of the binding agents of the invention described herein. In exemplary aspects, the binding agent specifically binds to S-glutathionylated MyBP-C. In exemplary aspects, the MyBP-C is S-glutathionylated at one or more of Cys 239, Cys249, Cys 426, Cys 436, Cys 443, Cys 475 , Cys528, Cys566, Cys 623, Cys 651, and/or Cys 719, according to the amino acid position numbering of SEQ ID NO: 21. In exemplary aspects, the MyBP-C is S-glutathionylated at one or more of Cys 475, Cys 623, and/or Cys 651, according to the amino acid position numbering of SEQ ID NO: 21. In exemplary aspects, the binding agent does not bind to a MyBP-C lacking one or more S-glutathionylatied Cys residues. In exemplary aspects, the binding agent does not bind to an un-S-glutathionylated MyBP-C. In exemplary aspects, the binding agent does not bind to one or more of Cys 239, Cys249, Cys 426, Cys 436, Cys 443, Cys 475 , Cys528, Cys566, Cys 623, Cys 651, and/or Cys 719, according to the amino acid position numbering of SEQ ID NO: 21, when the Cys residue is not S-glutathionylated. In exemplary aspects, the binding agent does not bind to one or more of Cys 475, Cys 623, and/or Cys 651, according to the amino acid position numbering of SEQ ID NO: 21, when the Cys residue is not S-glutathionylated.

In exemplary embodiments, the isolated binding agent specifically binds to an epitope within MyBP-C, wherein the epitope comprises one or more of Cys 239, Cys249, Cys 426, Cys 436, Cys 443, Cys 475 , Cys528, Cys566, Cys 623, Cys 651, and/or Cys 719, according to the amino acid position numbering of SEQ ID NO: 21. In exemplary embodiments, the isolated binding agent specifically binds to an epitope within MyBP-C, wherein the epitope comprises one or more of Cys 475, Cys 623, and/or Cys 651, according to the amino acid position numbering of SEQ ID NO: 21. In exemplary aspects, the one or more Cys residues is/are S-glutathionylated. In exemplary aspects, the binding agent does not bind to the one or more Cys residues when the one or more Cys residues is/are not S-glutathionylated. In alternative aspects, the one or more Cys residues is/are not S-glutathionylated and the binding agent binds to the one or more Cys residues only when it/they is/are not S-glutathionylated.

In exemplary embodiments, the isolated binding agent specifically binds to a Cys residue of MyBP-C, wherein the Cys residue is Cys 239, Cys249, Cys 426, Cys 436, Cys 443, Cys 475 , Cys528, Cys566, Cys 623, Cys 651, and/or Cys 719, according to the amino acid position numbering of SEQ ID NO: 21. In exemplary embodiments, the isolated binding agent specifically binds to a Cys residue of MyBP-C, wherein the Cys residue is Cys 475, Cys 623, and/or Cys 651, according to the amino acid position numbering of SEQ ID NO: 21. In exemplary aspects, the Cys residue is S-glutathionylated. In exemplary aspects, the binding agent does not bind to the Cys residue when the Cys residue is not S-glutathionylated. In alternative aspects, the Cys residue is not S-glutathionylated and the binding agent binds to the Cys residue only when it is not S-glutathionylated.

In exemplary aspects, the binding agent is conjugated to a detectable label, e.g., a fluorophore, a chromophore, a radioisotope, and the like. In exemplary aspects, the binding agent is conjugated to an enzyme or enzyme substrate. In exemplary aspects, the binding agent is conjugated to a binding partner of a high affinity binding interaction between two binding partners. In exemplary aspects, the binding agent is conjugated to avidin or biotin.

In exemplary aspects, the kit comprises secondary antibodies that bind to a primary antibody, which in exemplary aspects is the binding agent for MyBP-C, a post-translationally modified form thereof, or a fragment thereof. The secondary antibody in some aspects is conjugated to a detectable label (e.g., a fluorophore, a chromophore, a radioisotope, and the like), an enzyme, an enzyme substrate, a binding partner of a high affinity binding interaction between two binding partners (e.g., avidin, biotin).

In exemplary aspects, the kit comprises additional components for an immunoassay, e.g., a microtiter plate, a bead, a blocking solution, a tube, a flask, membrane, a filter, a washing solution, an enzyme, enzyme substrate, a detectable label.

In exemplary aspects, the kit comprises components for an ELISA, e.g., a sandwich ELISA. For example, the kit may comprise a microtiter plate, a MyBP-C specific antibody, an anti-glutathione specific antibody, a secondary antibody, a washing solution, a blocking solution, and a enhanced chemiluminecent (ECL) kit.

In alternative or additional embodiments of the inventive kits, the kit comprises a binding agent for ADMA and/or a binding agent for SDMA. In exemplary aspects, the binding agent specific for ADMA and/or the binding agent for SDMA is an antibody.

In some aspects, the binding agent(s) is/are bound to a solid support, e.g., a bead, a well, a dish, a tube, a filter paper, a membrane, and the like. In other aspects, the binding agent is provided in solution. In yet further aspects, the binding agent is provided in a freeze-dried form.

In some aspects, the kit comprises a collection of nucleic acid probes which specifically bind to genes or mRNA encoding MyBP-C, or a fragment thereof. In some aspects, the collection of nucleic acid probes is formatted in an array on a solid support, e.g., a gene chip.

In some aspects, the kits further comprises instructions for use, e.g., instructions for carrying out the methods of the invention. In some aspects, the instructions are provided as a paper copy of instructions, an electronic copy of instructions, e.g., a compact disc, a flash drive, or other electronic medium. In some aspects, the instructions are provided by way of providing directions to an internet site at which the instructions may be accessed by the user.

In some aspects, the instructions comprise a step in which the user compares data relating to a level of MyBP-C, or a post-translationally modified form or fragment thereof, to a database containing correlation data between the data and to a diagnosis of diastolic heart failure. In some aspects, the kit comprises an electronic copy of a database containing correlation data between the data relating to a level of MyBP-C, or a post-translationally modified form or fragment thereof, and to a diagnosis of diastolic heart failure. In some aspects, the kit comprises an electronic copy of a computer software program which allows the user to compare the level of MyBP-C, or a post-translationally modified form or fragment thereof with that of a control subject. In exemplary aspects, the kit comprises a system or a computer-readable storage medium of the invention.

In alternative aspects, the instructions comprise a step in which the user provides data relating to a level of MyBP-C, or a post-translationally modified form or fragment thereof, to a provider and the provider, after analyzing the data, provides diagnostic information to the user.

In some aspects, the kits further comprise a unit for a collecting a sample, e.g., any of the samples described herein, of the subject. In some aspects, the unit for collecting a sample is a vial, a beaker, a tube, a microtiter plate, a petri dish, a bag, a cup, and the like.

The following examples are given merely to illustrate the present invention and not in any way to limit its scope.

### EXAMPLES

### EXAMPLE 1

The following example demonstrates a significant increase in glutathionylation of myosin binding protein C in animals with diastolic dysfunction.

Generation of DOCA-salt mouse model: Previously, we have shown that this model leads to mild hypertension, myocardial oxidative stress, and diastolic dysfunction. A gradual and mild elevation in blood pressure was induced by unilateral nephrectomy, subcutaneous implantation of a controlled release deoxycorticosterone acetate (DOCA) pellet (0.7 mg/d; Innovative Research of America, Sarasota, FL), and substituting drinking water with 1.05% saline. Control animals underwent a sham operation, had placebo pellet implantation, and received water without salt.

Invasive hemodynamic studies, noninvasive echocardiography, and myocyte isolation were done on postoperative day 14-18 for DOCA-salt and control mice. All experiments were approved by the University of Illinois at Chicago Animal Care and Use Committee.

Chronic Administration of Ranolazine: A special diet contained 5 mg ranolazine, 0.3 mg P450 inhibitor, and 0.25 mg red food color was pressed into a 1-g nutritionally complete grain-based tablet (Harlan, Madison, WI). The control diet contained 0.3mg P450 inhibitor and 0.25 mg yellow food color pressed into the same type of 1-g tablets. DOCA-salt and sham mice consumed daily ∼1g of the special diet from postoperative day 11 to 18.

Analysis ofPost-Translational Modifications: Skinned fibers with 1% (v/v) Triton X-100 were solubilized in 15 µL sample buffer (8 M urea, 2 M thiourea, 0.05 M tris pH 6.8, 75 mM DTT, 3% SDS, and 0.05% bromophenol blue) by incubation on a shaker for 30 min followed by two cycles of 10 min incubations in a sonicating bath with 30 seconds vortexing between the incubations. Samples were heated at 100°C for three min and after 10 min spin clarification, all of the supernatant was loaded on to a 12% resolving 1D SDS-PAGE gel. The gels were stained and destained with Pro-Q Diamond (Invitrogen) according to the manufacturer's recommendations preceding imaging with a Typhoon 9410 scanner (GE Healthcare). Optical density of the proteins was determined using ImageQuant TL (GE Healthcare) software and Commassie R-250 stained gel was used to normalize protein load to both actin the whole lane. Results were exported to Excel and analyzed with JMP statistical software (Cary, NC).

Western blot analysis was used to detect for glutathionylated proteins. Myofibrils were prepared from DOCA and sham mouse hearts, and pellets were solubilized in a non-reducing 2X Laemmli buffer (4% SDS, 20% glycerol, 0.004% bromophenol blue, and 0.125 M Tris HCl pH 6.8). 25 mM N-ethylmaleimide (NEM) was added to the standard rigor buffer with Triton X-100, the standard rigor wash buffer and the 2X Laemmli buffer. Using the protein concentration determined from an RC-DC (Bio-Rad) assay, 40 µg of total protein was applied to 1D 12% resolving SDS-PAGE gel and transferred onto a 0.2 µM Polyvinylidene fluoride (PVDF) membrane. The blot was blocked in 5% nonfat dry milk with 2.5 mM NEM for 1 hour. Anti-glutathione mouse monoclonal primary antibody (Virogen, Watertown, MA) was used at 1:1000 dilution along with anti-mouse HRP-conjugated secondary antibody (Sigma) at 1:100,000 dilution to detect for S-glutathionylation. 10 Optical density of the bands was measured with ImageQuant TL (GE Healthcare) and exported to Excel for further analysis. An antibody to myosin binding protein C was used for identification of the band indentified as being modified.

As reported in U.S. Provisional Patent Application 61/544,030 (filed October 6, 2011), U.S. Provisional Patent Application No. 61/618,900 (filed April 2, 2012), and Lovelock et al., Circ Res 110(6): 841-850 (e-pub Feb 16, 2012), we demonstrated a significant increase in glutathionylation of myosin binding protein C in animals with diastolic dysfunction. Figure 1 represents data from this publication demonstrating the post-translational modifications of the myofilaments from sham and DOCA-salt hearts. Figure 1A: Western blot analysis demonstrating detection of glutathionylated proteins when comparing myofilament samples from sham and DOCA-salt treated hearts. The blots were also analyzed for myosin binding protein C, indicating that this protein was the major modification. Note that one pair of comparisons was removed from the gel. Figure 1B: Quantification of the difference between glutathionylated proteins (GSH) normalized to myosin binding protein C (MyBP-C) in sham and DOCA-salt myofilaments. (*p < 0.05, n=3).

### EXAMPLE 2

### Generation of DOCA-Salt Mouse Model:

Previously, we have shown that the DOCA-salt mouse model leads to mild hypertension, NOS uncoupling, myocardial oxidative stress, and diastolic dysfunction [10]. A gradual and mild elevation in blood pressure was induced by unilateral nephrectomy, subcutaneous implantation of a controlled release deoxycorticosterone acetate (DOCA) pellet (0.7 mg/d; Innovative Research of America, Sarasota, FL), and substituting drinking water with 1.05% saline. Control animals underwent a sham operation, had placebo pellet implantation, and received water without salt.

### Administration of BH₄:

Mice were divided into two groups which received a control diet (sham N = 7; DOCA-salt N = 10) and two groups which received a BH₄ supplemental diet of 5 mg BH₄/day (Research Diets Inc, New Brunswick, NJ; sham + BH₄ N = 8; DOCA-salt + BH₄ N = 8). The supplemental diet began on day 11 after surgery, and continued until day 18, when the mice were analyzed and sacrificed.

### Analysis of Sarcomeric Protein Glutathionylation by Western Immunoblotting:

Myofibrils were prepared from DOCA-salt and sham model hearts, and pellets were solubilized in a non-reducing 2X Laemmli buffer (4% SDS, 20% glycerol, 0.004% bromophenol blue, and 0.125 M Tris HCI pH 6.8). 25 mM N-ethylmaleimide (NEM) was added to the standard rigor buffer with Triton X-100, the standard rigor wash buffer and the 2X Laemmli buffer. [21] Using the protein concentration determined from an RC-DC (Bio-Rad) assay, 40 pg of total protein was applied to a 12% SDS-PAGE gel and transferred onto a 0.2 pm PVDF membrane. The blot was blocked in 5% nonfat dry milk with 2.5 mM NEM for 1 h. Anti-glutathione mouse monoclonal primary antibody (Virogen) was used at 1:1000 dilution along with anti-mouse HRPconjugated secondary antibody (Sigma) at 1:100,000 dilution to detect for S-glutathionylation. Optical density of the bands was measured with ImageQuant TL (GE Healthcare) and exported to Excel for further analysis.

### Statistical Analysis:

Echocardiography, isolated cardiomyocyte, skinned fiber tension, ATPase measurements, and post-translational modifications of myofilament protein data were statistically analyzed using either GraphPad Prism or JMP statistical software, using one- or two-way ANOVA followed by Student's West. Analysis of the relation between Ca²⁺ and tension or ATPase activity was fitted using a modified Hill equation as described previously. Analysis of the relation between MyBP-C glutathionylation and echocardiographic, E/E' ratio was correlated in linear regression analysis. A value of *P* < 0.05 was considered significantly different. Data are presented as means ± SEM.

### Myosin Binding Protein C Post-Translational Modifications

In view of our earlier findings indicating an increase in MyBP-C S-glutathiolation in cardiac myofilaments from DOCA-salt mouse, we determined whether the BH₄ diet could reverse this modification. Representative gels and plotted data normalized to total protein loadings are shown in Figure 2. MyBP-C glutathionylation was significantly increased in the DOCA-salt group compared to all other groups, which were not significantly different from each other.

In the data shown in Figures 3A to 3D, we plotted diastolic function parameters (E/E' or E'/A' ratio) as a function of normalized MyBP-C glutathionylation. As shown in Figure 3A and B, the E/E' ratio was significantly, positively correlated with MyBP-C glutathionylation (Slope = 3.23 ± 0.90, R² = 0.305, **P < 0.01). Moreover, TDI E'/A' ratio was negatively correlated with MyBP-C glutathionylation (Slope = -0.08 ± 0.02, R² = 0.257, ***P* < 0.01). Myofilament tension cost was also inversely correlated with both MyBP-C glutathionylation and E/E', echocardiographic data.

Ten days after surgery, we employed echocardiography to characterize the diastolic dysfunction. Mitral pulse wave Doppler flow and tissue Doppler imaging (TDI) were perfomed using the Vevo 770 high-resolution *in vivo* imaging system (Visual Sonics, Toronto, Canada). Mice were anesthetized with 1-1.5% isoflurane until a heart rate of around 350-390 beats/min was achieved because measure of diastolic function are sensitive to heart rate and loading conditions. M-mode images in the parasternal long axis and the left ventricle (LV) short-axis views at the mid-papillary level were taken. Measurements were averaged from five consecutive beats during expiration. The images for each mouse were recorded for at least 5s (30-40 cardiac cycles) from which three to five representative cycles with the highest quality imaging were selected. Percent fractional shortenin (%FS) were calculated as 100 x (LVEDd)-(LVESd)/(LVEDd) and percent LV ejection fraction (%EF) was calculated as 100x [(7/2.4 + LVEDd) x LEDd³]-[(7/2.4+LVESd)xLVESd³] / [(7/2.4+LVEDd)xLEDd³]. Doppler measurements were made at the tips of the mitral leaflets for diastolic filling profiles in the apical four-chamber view. Mitral inflow velocities, peak early (E) and late (A) were measured by conventional pulsed-wave Doppler. TDI was used to determine the mitral annulus longitudinal velocities (Sm, E', and A'). Baseline images before treatment were acquired to confirm diastolic dysfunction in DOCA-salt mice. Subsequently, the mice were fed with BH₄, followed by echocardiography at day 18.

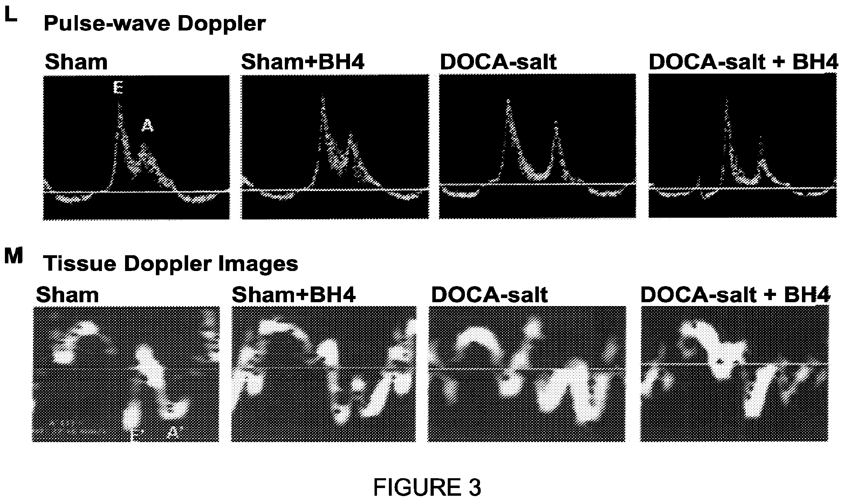

Treatment with BH₄ supplemental diet was begun on postoperative day 11, and echocardiography was repeated on postoperative day 18. The results can be seen in Figure 3 and Table 1. Seven days of BH₄ administration in sham and DOCA-salt did not affect LV ejection fraction (Figure 3G) or fractional shortening (Figure 3H).

Mitral Doppler flow was measured at comparable heart rates (∼ average 370 beats/min) in all mice. As we have reported in this model, mitral E velocity, A velocity, and the E/A ratio were not significantly changed in all groups (Figure 3I and 3L). Nevertheless, mitral tissue Doppler E' was significantly decreased in the DOCA-salt mice indicating a pseudo-normal diastolic dysfunction stage. The ratio of E'A' was significantly decreased in DOCA-salt mice and restored with BH₄ treatment (DOCA-salt+ BH₄, 1.12 ± 1.10 vs. DOCA-salt, 0.74 ± 0.05, *P* < 0.001). The sham and sham + BH₄ groups did not show any significant differences in E'/A' (Figures 3J and 3M). The E/E' ratio, a measure of left atrial pressure, was significantly increased in DOCA-salt mice, and restored to the control level after BH₄ administration (Figure 3K, DOCA-salt+ BH₄, 34.5 ± 2.2 vs. DOCA-salt, 43.7 ± 2.7, *P* < 0.01).

### EXAMPLE 3

The plasma of DOCA-salt mice or sham control mice were collected and the levels of glutathionylated MyBP-C and glutathionylated fragments thereof were measured by immunoprecipitating the samples with a glutathione-specific antibody followed by Western blotting with a MyBP-C-specific antibody. Figure 4 shows that total serum levels of MyBP-C are increased, and that the intact or full length glutathionylated MyBP-C (144kD) was increased in plasma of DOCA-salt DD mice (N=4). A glutathionylated 40 kD fragment of MyBP-C and a glutathionylated 15-20 kDa fragment were also elevated in plasma of DOCA-salt mice compared to sham control mice.

### EXAMPLE 4

The plasma from a total of N=120 subjects, with N=40 in each of the three groups: systolic HF, diastolic HF and healthy control patients; is collected. An intermediate analysis is preformed when samples are available on 20 patients in each group to determine whether to continue with the larger cohort. Potential subjects come from the cohort of several thousand patients followed by University of Illinois at Chicago and the Jesse Brown Veterans Affairs Medical Center. Eligibility criteria include: 1) greater ≥ 18 years of age and 2) have SD or DD heart failure documented by an echocardiogram in the last year. Ineligibility criteria include: history of congenital heart disease, congenital arrhythmic disorders, patients undergoing cancer treatment. All patients enrolled give written consent.

Data are collected from subject interviews and review of hospital and clinic charts. Demographic data obtained include: age, race, body mass index, New York Heart Association (NYHA) functional class, and a history of previous myocardial infarction, hypertension, diabetes, smoking, or alcohol use. Additionally, all medications being taken at the time of enrollment and the date and method of EF determination are recorded.

A single blood draw is performed at the time of enrollment. Glutathionylation of MyBP-C is measured under non-reducing conditions. All patient samples are processed in a similar fashion to our animal studies.

A power analysis indicates that with a two-tailed alpha level of 0.05 and a test power of 0.80, the sample size needed based upon the mean values and the standard deviations of the preliminary animal data is ∼30 subjects per group. To have sufficient numbers to correct for up to 6 covariates, 40 patients are in each group.

### Analysis of plasma MyBP-C glutathionylation:

Plasma samples from the groups are collected and Western blot analysis are used to detect MyBP-C and S-glutathionylation as follows:

### Analysis of plasma MyBP-C glutathionylation:

Plasma samples from the groups are collected and divided into reducing and non-reducing aliquots. Reducing samples are prepared in a reducing 2X Laemmli sample buffer (2% β-mercaptoethanol, 4% SDS, 20% glycerol, 0.004% bromophenol blue, and 0.125 M Tris HCl pH 6.8) and boiled for 5 min at 95 °C. Non-reducing samples for glutathionylation are prepared in 2X Laemmli buffer (25 mM N-ethylmaleimide, 4% SDS, 20% glycerol, 0.004% bromophenol blue, and 0.125 M Tris HCl pH 6.8).⁵ Plasma samples are analyzed with 4-20% gradient SDS-PAGE gel and transferred to nitrocellulose blot. The blot is blocked in 5% nonfat dry milk with 2.5 mM NEM for 1 h. Anti-glutathione monoclonal primary antibody (Virogen) or anti-MyBP-C antibody is used at 1:1000 ∼ 1:5000 dilution along with anti-mouse HRP-conjugated secondary antibody (Sigma) at 1:20,000 dilution to detect for MyBP-C or S-glutathionylation.⁵ Optical density of the bands is measured with Image J.

### Data analysis and anticipated results:

The level of glutathionylated MyBP-C is compared between controls, DD, and SD patients. It is expected that glutathionylated MyBP-C will be highest in DD patients. Baseline data are expressed as mean ± SD for continuous variables, and frequencies for categorical variables. Differences in baseline characteristics between the groups are examined by use of Fisher exact and Mann-Whitney tests for categorical and continuous variables, respectively. Regression is used to adjust for independent variables in which the distribution is not balanced between the groups. A stepwise backward procedure is used. Results are compared to stepwise forward procedures. The possibility of multicolinearity is evaluated. Linear and non-linear terms are considered. Discrimination of the model is evaluated by an overall C index and validated by bootstrap methods. If nonlinearity is detected, transformations of the variables or fitting a nonlinear model are attempted, and terms are evaluated for their improvement of the model. Normality of the variable distributions is tested by a normal probability plot and by a Shapiro-Wilk test. While regression is fairly tolerant of violations in this regard, transformations are investigated as necessary. Homoscedasticity is evaluated by plot of residuals versus predicted values. If the assumptions appear to be violated, non-parametric methods are considered.

### Data analysis and anticipated results:

The level of glutathionylated MyBP-C are compared between controls, DD, and SD patients using statistical analysis as described in above. It is anticipated that glutathionylated MyBP-C are highest in DD patients.

### EXAMPLE 5

This example demonstrates an increase of S-glutathionylated MyBP-C in plasma samples of DOCA-salt mice.

Plasma samples were collected from sham or DOCA-salt mice under non-reducing conditions. Samples were immunoprecipitated with a glutathione-specific antibody and Western blotted with a MyBP-C-specific antibody.

Plasma levels of glutathionylated intact MyBP-C (144 kDa), and glutathionylated fragments thereof (75 kDa and 25 kDa) were significantly increased in DOCA-salt mice. As shown in Figure 5, the glutathionylated 75 kDa fragment of MyBP-C was significantly increased in DOCA-salt mice (3.7 ± 0.6 fold, *P* = 0.011, N=3), compared to sham control mice. Data were represented as mean ± SEM. P<0.05 in a *Student's unpaired t-test.*

### EXAMPLE 6

Plasma from diastolic dysfunction patients who have preserved left ventricular function was collected. The samples were Western blotted with a MyBP-C-specific antibody. As shown in Figure 6A, a 75kDa fragment of MyBP-C was significantly elevated (2.6 ± 0.6-fold, P<0.05, N=8) in the plasma from heart failure with preserved ejection fraction (HFpEF) patients (i.e., diastolic dysfunction patients who have preserved left ventricular function).

Equivalent volumes of plasma samples from the healthy patient group or the HFpEF group were immunoprecipitated with a glutathione-specific antibody, and then immunoblotted with anti-MyBP-C antibody. As shown in Figure 6B, the glutathionylated 75 kDa fragment of MyBP-C from the plasma of HFpEF patients were significantly increased (2.5 ± 0.3-fold, P<0.05, N=8), as compared to healthy patients. Densitometry data acquired from image J were represented as mean ± SEM. P < 0.05 in a Student's unpaired t-test.

### EXAMPLE 7

This example demonstrates that an increase in S-glutathionylation of MyBP-C is representative of diastolic dysfunction and suggests that treatment of diastolic dysfunction results in regression of S-glutathionylated MyBP-C levels.

S-glutathionylation levels in DOCA-salt and sham hearts from mice were determined after or without chronic administration of ranolazine. The administration of ranolazine to mice and the analysis of post translational modifications were analyzed as essentially described in Example 1.

Representative gels showing changes in S-glutathionylation of MyBP-C are shown in Figure 7A. MyBP-C glutathionylation was significantly increased in the DOCA-salt group compared to all other groups. A regressed level of MyBP-C glutathionylation was observed in the DOCA-salt animals when administered ranolazine.

### EXAMPLE 8

This example demonstrates that the increase of S-glutathionylated MyBP-C in DOCA-salt mice is specific to this type of post-translational modification and also specific to this protein.

DOCA-salt or sham mice were administered ranolazine or BH4 as essentially described in Examples 1 and 2, respectively. Pro-Q Diamond (Invitrogen) gel stain was used to detect changes in phosphorylation states of MyBP-C. Myofibrils were prepared from DOCA-salt and sham models of the mice hearts, and pellets were solubilized in a non-reducing 2X laemmli buffer (4% SDS, 20% glycerol, 0.004% bromophenol blue, and 0.125 M Tris HCl pH 6.8). 25 mM N-ethylmaleimide (NEM) was added to the standard rigor buffer with Triton X-100, the standard rigor wash buffer, and the 2X Laemmli buffer. An RC-DC assay (BioRad) was used to determine protein concentrations. Samples were diluted at 1:1 ratio in reducing sample buffer (8 M urea, 2 M thiourea, 0.05 M tris pH 6.8, 75 mM DTT, 3% SDS, and 0.05% bromophenol blue and approximately 10 µg of protein was loaded on to a 12% resolving 1D SDS-PAGE gel. The gels were stained and destained with Pro-Q Diamond according to the manufacturer's recommendations prior to the imaging with a Typhoon 9410 scanner (GE Healthcare). Coomassie R-250 staining was used to normalize protein load to both actin and the whole lane. Optical density of the proteins was determined using ImageQuant TL (GE Healthcare) software and results were exported to Excel for further analysis.

As shown in Figure 8A, there were no changes in the levels of phosphorylation of MyBP-C among sham and DOCA-salt mice. Treatment of mice with either Ranolazine of BH4 also did not affect the phosphorylation levels of MyBP-C (Figures 8A and 8B). Therefore, in contrast to the increase in S-glutathionylated MyBP-C levels observed in DOCA-salt mice, phosphorylation of MyBP-C does not change among healthy animals and animal models of diastolic dysfunction.

### EXAMPLE 9

This example demonstrates the sites of S-glutathionylation on MyBP-C.

Using tandem mass spectrometry, a 140 kDa band was identified as cMyBP-C and the sites of glutathionylation were determined as the cysteines at positions 655, 479, and 627. 8% non-reducing 1D SDS-PAGE gel was run as mentioned above and stained with Imperial Protein Stain (Thermo) according to manufacturer's protocol. The band around 140 kDa (MyBP-C) was cut from the gel and subjected to in-gel digest with Trypsin Gold (Promega). Reduction and alkylation steps were omitted to preserve the S-glutathionylation of the proteins. Pooled digestion extracts were concentrated via Speed Vac to less than 20 µl and brought up to 40 µl with mobile phase solution (5% ACN and 0.1% formic acid). Peptides were filtered with 0.22 µm PVDF Millipore Ultrafree-MC spin filter and 35 µl of sample was analyzed with Thermo Finnigan LTQ FT hybrid linear ion trap - Fourier Transform ICR mass spectrometer coupled to Dionex U3000 nano LC. Dionex acclaim PepMap100 C18 trapping column (500 µm × 5 mm column packed with 5 µm, 100 A Symmetry C18 material) was used to concentrate the samples at flow rate of 50 µl/min and Agilent Zorbax 300SB-C18 Nanoflow column (75 µm × 150 mm column packed with 3.5 µm, C18 material) was used for separation at flow rate of 0.250 µl/min. Peptides were eluted with a linear gradient of 5-45% solution B (95% ACN, 0.1% formic acid) through New Objective uncoated SilicaTips™ (5 cm long, tubing OD 360/75 µm, tip ID 8 µm). Peptides were ionized via electrospray ionization with LTQ source voltage set to 1.0 kV and capillary temperature set to 200°C. Mass spectra were obtained in positive ion mode over m/z range of 400 to 1800 at a resolution of 50,000 and top ten most intense ions were selected for tandem MS. MS/MS were obtained in collision induced dissociation mode with minimum signal required 5000, isolation width 3, and normalized collision energy.

Mass spectrometry data were collected with Xcalibur 2.0.7 software as .Raw file which were converted to mzXML and MGF files using MassMatrix MS Data File Conversion tool. Mascot search engine was used to analyze the MS/MS data. Searches were performed using NCBInr Mus musculus and a decoy (automatically generated reversed protein sequences) database with peptide tolerance of ± 15 ppm. Variable modifications were set to glutathione (C). The results are representative of three similar and separate mass spectrometry runs.

Tandem mass spectrometry was used to identify glutathionylated proteins and determine the sites of cMyBP-C glutathionylation. Mass spectra resulting from the LC/MS/MS of the -140 kDa band identified myosin-binding protein C, cardiac-type (gi|134031947), with 43-50% sequence coverage as the number one hit. Tryptic peptides of cMyBP-C were analyzed for a mass shift of 305 Da, indicating covalent attachment of GSH to one of the peptide residue. Three separate peptides, ⁶⁵¹IHLD**C**PGSTPDTIVVVAGNK⁶⁷⁰, ⁴⁷⁵VEFE**C**EVSEEGAQVK⁴⁸⁹, and ⁶⁰⁵LTIDDVTPADEADYSFVPEGFA**C**NLSAK⁶³², were found to be glutathionylated (Table 2). When the myofibrils were treated with 1 mM GSSG, only peptide ⁶⁵¹IHLD**C**PGSTPDTIVVVAGNK⁶⁷⁰ was found to be glutathionylated at cys655. The precursor ion for the triply charged glutathionylated peptide was observed at m/z 781.04³⁺ compared to the unmodified precursor peptide observed at m/z 679.35³⁺. Comparison between select b and y product ions of the glutathionylated and unmodified peptide show mass shift of 305 Da (singly charged ions) or 152.5 Da (doubly charged ions). With treatment of 5 mM GSSG, we identified all three of the above peptides to be glutathionylated at cys655, cys479, and cys627. ⁴⁷⁵VEFE**C**EVSEEGAQVK⁴⁸⁹ triply charged glutathionylated peptide was observed at m/z 663.28³⁺ and ⁶⁰⁵LTIDDVTPADEADYSFVPEGFA**C**NLSAK⁶³² glutathionylated precursor peptide was observed at m/z 1098.49³⁺. Expect value of less than 0.1 was considered to be significant identification of each individual peptide. Significant identification of glutathionylated cMyBP-C peptides was not made in the control non-treated myofibril samples.

**TABLE 2**

| **GSSG** | **Accession Number** | **Description** | **Score** | **Percent Coverage** | **Glutathionylated Peptide** | **Charge** | **Observed Mass** | **Peptide Mass Tolerance (ppm)** | **Miss Cleavages** | **Ions Score** | **Expect** |
|---|---|---|---|---|---|---|---|---|---|---|---|
| 1 mM | gi\|134031947 | myosin-binding protein C, cardiac-type [Mus musculus] | 7820 | 42.9 | K.IHLDC*PGSTPDTIVVVAGNK.L | 3+ | 781.04 | -5 | 0 | 41 | 0.0033 |
| 5 mM | gi\|134031947 | myosin-binding protein C, cardiac-type [Mus musculus] | 8033 | 49.7 | K.IHLDC*PGSTPDTIVVVAGNK.L | 3+ | 781.04 | -5 | 0 | 51 | 0.00033 |
| | | | | | K.IHLDC*PGSTPDTIVVVAGNK.L | 3+ | 781.04 | -5 | 0 | 48 | 0.00071 |
| | | | | | K.IHLDC*PGSTPDTIVVVAGNK.L | 3+ | 781.04 | -2 | 0 | 51 | 0.00041 |
| | | | | | K.IHLDC*PGSTPDTIVVVAGNK.L | 3+ | 781.04 | -2 | 0 | 50 | 0.00049 |
| | | | | | R.VEFEC*EVSEEGAQVK.W | 3+ | 663.28 | 0 | 0 | 45 | 0.00066 |
| | | | | | R.VEFEC*EVSEEGAQVK.W | 3+ | 663.28 | 0 | 0 | 36 | 0.0051 |
| | | | | | R.VEFEC*EVSEEGAQVK.W | 3+ | 663.28 | 1 | 0 | 30 | 0.018 |
| | | | | | K.LTIDDVTPADEADYSFVPEGFAC* NLSAK.L | 3+ | 1098.49 | 5 | 0 | 40 | 0.0034 |
| | | | | | K.LTIDDVTPADEADYSFVPEGFAC* NLSAK.L | 3+ | 1098.49 | 3 | 0 | 40 | 0.0031 |

Alignment of the sequences ⁶⁵¹IHLD**C**PGSTPDTIVVVAGNK⁶⁷⁰, ⁴⁷⁵VEFE**C**EVSEEGAQVK⁴⁸⁹, and ⁶⁰⁵LTIDDVTPADEADYSFVPEGFA**C**NLSAK⁶³² to the human sequence of cardiac MyBP-C (NCBI reference sequence CAA58882.1; SEQ ID NO: 21) suggested that the S-glutathionylated Cys residues in human cardiac MyBP-C are Cys475, Cys 623, and Cys 651, according to the amino acid position numbering of SEQ ID NO: 21.

### EXAMPLE 10

This example demonstrates an increased level of asymmetric dimethylarginine (ADMA) in patients of diastolic dysfunction.

NOS uncoupling is enhanced by asymmetric dimethylarginine (ADMA). We hypothesized that an elevation of plasma ADMA could contribute to NOS dysfunction and diastolic heart failure (DHF).

Plasma ADMA, L-arginine, and symmetric dimethyl arginine (SDMA) levels were determined from human plasma samples from patients with DHF, patients with systolic HF (SHF), and from a healthy control group. The levels were determined via ELISA. As shown in Figure 9A, plasma ADMA levels were elevated in both DHF patients (0.77 ± 0.04 µM, P<0.05) and SHF patients (0.68 ± 0.03 µM, P<0.05) compared to control group (0.57 ± 0.02 µM). Plasma SDMA and L-arginine levels did not show changes in any group (Figures 9B and 9C). As shown in Figure 9D, the ratio of L-arginine/ADMA was significantly reduced in the DHF group (139.5 ± 9.5, P<0.05) but not in the SHF group.

The plasma of acute HF patients and chronic HF patients were analyzed. The ADMA level was increased in the plasma of both patient groups (acute and chronic HF patients) (0.71 ± 0.03 µM, P<0.05, Acute; 0.74 ± 0.03 µM, P<0.05, Chronic) compared to control (Figure 10A), but there was no difference based on the acutely of the illness. Plasma SDMA, L-Arginine, and L-arginine/ADMA ratio was not changed among the acute and chronic groups (Figures 10B-10D).

Plasma ADMA level was significantly correlated with BMI (Slope = 24.27 ± 7.84, R² = 0.205, *P* = 0.0038) (Figure 11A). Moreover, as shown in Figure 11B, L-Arginine/ADMA ratio was inversely correlated with BMI (Slope = -2.38 ± 1.16, R² = 0.099, *P* <0.048).

These data suggest that ADMA may contribute to the pathology of DHF, and that an elevated plasma ADMA level or the ratio of L-arginine/ADMA may be useful as biomarkers for diastolic dysfunction.

### EXAMPLE 11

Human plasma from healthy patients without heart failure, patients with diastolic dysfunction (DD), or patients with systolic dysfunction (SD) was collected as essentially described in Example 4. Equal volumes of plasma samples were loaded into an electrophoresis gel, transferred to a membrane, and Western blotted by MyBP-C. Total plasma levels of MyBP-C were greatest in DD patients, lowest for healthy control patients, and intermediate for SD patients. (Figure 13, lower right graph). Plasma levels of a 75 kDa fragment of MyBP-C was greatest in DD patients, relative to control (Figure 13, lower left graph).

Equal volumes of plasma samples were immunoprecipitated with S-glutathione-specific antibodies and the immunoprecipitates were western blotted using a MyBP-C-specific antibody. As shown in Figure 14, lower graph, the level of an S-glutathionylated 75 kDa fragment of MyBP-C was greatest in DD patients, lowest in control patients, and intermediate in SD patients.

Ratio of S-glutathionylated MyBP-C fragment, 75kD, and protein amount of MyBP-C 75 kD from human plasma of DD and SD patients were calculated and represented in Figure 15. Immunoprecipitated data of S-glutathionylation and MyBP-C, 75kD, were normalized with immunoblotting data detecting MyBP-C, 75kD. The ratio of S-glutathionylation and protein amount of MyBP-C 75kD fragment were significantly increased in DD patients, and intermediate in SD patients compared with control group.

### EXAMPLE 12

This example demonstrates a method of detecting glutathionylated cMyBP-C levels through a sandwich ELISA.

A surface of a multi-well microtiter plate is pre-coated with a cardiac MyBP-C-specific antibody. This antibody is considered the capture antibody. The surface is then blocked to prevent non-specific binding to the surface. The test or control sample is then applied to the plate and the plate is washed to remove any unbound MyBP-C. An antibody specific to glutathionylated cardiac MyBP-C, which antibody is conjugated to biotin, (the detection antibody) is then added to the well. Avidin-conjugated horseradish peroxidase (HRP) is subsequently is subsequently added to the wells. Chromogenic substrates of HRP are added and color development is stopped. The intensity of the color is measured based enhanced chemiluminescence.

### REFERENCES:

(1) Zile MR, Baicu CF, Bonnema DD. Diastolic heart failure: definitions and terminology. Prog Cardiovasc Dis 2005 March;47(5):307-13.
(2) Baynes JW. Role of oxidative stress in development of complications in diabetes. Diabetes 1991 April;40(4):405-12.
(3) Baynes JW, Thorpe SR. Role of oxidative stress in diabetic complications: a new perspective on an old paradigm. Diabetes 1999 January;48(1):1-9.
(4) Silberman GA, Fan TH, Liu H, Jiao Z, Xiao HD, Lovelock JD, Boulden BM, Widder J, Fredd S, Bernstein KE, Wolska BM, Dikalov S, Harrison DG, Dudley SC Jr. Uncoupled cardiac nitric oxide synthase mediates diastolic dysfunction. Circulation 2010 February 2;121(4):519-28.
(5) Lovelock JD, Monasky MM, Jeong EM, Lardin HA, Liu H, Patel BG, Taglieri DM, Gu L, Kumar P, Pokhrel N, Zeng D, Belardinelli L, Sorescu D, Solaro RJ, Dudley SC Jr. Ranolazine Improves Cardiac Diastolic Dysfunction Through Modulation of Myofilament Calcium Sensitivity. Circ Res 2012 February 16.
(6) Jacquet S, Yin X, Sicard P, Clark J, Kanaganayagam GS, Mayr M, Marber MS. Identification of cardiac myosin-binding protein C as a candidate biomarker of myocardial infarction by proteomics analysis. Mol Cell Proteomics 2009 December;8(12):2687-99.
(7) Barefield D, Sadayappan S. Phosphorylation and function of cardiac myosin binding protein-C in health and disease. J Mol Cell Cardiol 2010 May: 48(5)866-75.
(8) Govindan S, McElligott A, Muthusamy S, Nair N, Barefield D, Martin JL, Gongora E, Greis KD, Luther PK, Winegrad S, Henderson KK, Sadayappan S. Cardiac myosin binding protein-C is a potential diagnostic biomarker for myocardial infarction. J Mol Cell Cardiol 2012 Jan; 52(1):154-64.
(9) McConnell BK, Jones KA, Fatkin D, Arroyo LH, Lee RT, Aristizabal O, Turnbull DH, Georgakopoulos D, Kass D, Bond M, Niimura H, Schoen FJ, Conner D, Fischman DA, Seidman CE, Seidman JG. Dilated cardiomyopathy in homozygous myosin-binding protein-C mutant mice. J Clin Invest 1999 December; 104(12):1771.
(10) Poutanen T, Tikanoja T, Jääskeläinen P, Jokinen E, Silvast A, Laakso M, Kuusisto J. Diastolic dysfunction without left ventricular hypertrophy is an early finding in children with hypertrophic cardiomyopathy-causing mutations in the beta-myosin heavy chain, alpha-tropomyosin, and myosin-binding protein C genes. Mol Cell Proteomics American Heart Journal 2006 February; 151(3)725.e1-725.e9.
(11) Mayr M, Jacquet S, Marber M, Gautel M. CMYBP-C AND MLC2 AS DIAGNOSTIC MARKERS OF CARDIAC INJURY. PCT/GB2010/000935 filed 5/11/2010; published as WO/2010/130985 on 11/18/2010; nationalized in Europe 11/15/2011 as application #10718654.6.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted.

Recitation of ranges of values herein are merely intended to serve as a shorthand method of referring individually to each separate value falling within the range and each endpoint, unless otherwise indicated herein, and each separate value and endpoint is incorporated into the specification as if it were individually recited herein.

All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A method comprising (i) determining a level of a myosin binding protein-C (MyBP-C), or fragment of MyBP-C having a molecular weight of 75 kDa, 40 kDa, or 25 kDa, wherein the MyBP-C or fragment is optionally S-glutathionylated, in a biological sample obtained from the subject, wherein the method is for (A) diagnosing diastolic dysfunction or diastolic heart failure, (B) differentiating between diastolic dysfunction and systolic dysfunction, or for characterizing heart failure as diastolic heart failure or systolic heart failure, (C) determining a subject's need for treatment for diastolic dysfunction or diastolic heart failure, or (D) identifying a patient at risk for diastolic dysfunction or diastolic heart failure,
wherein, when the method is for determining a subject's need for treatment for diastolic dysfunction or diastolic heart failure, the method comprises determining the subject as a subject who needs treatment for diastolic dysfunction or diastolic heart failure when the level is increased, as compared to a control level,
wherein, when the method is for identifying a patient at risk for diastolic dysfunction or diastolic heart failure, the method comprises identifying the subject as a subject at risk for diastolic dysfunction or diastolic heart failure, when the level of MyBP-C or fragment thereof, or a S-glutathionylated MyBP-C or fragment thereof, is increased, relative to a control level.

2. The method of claim 1, wherein the method is for diagnosing diastolic dysfunction or diastolic heart failure, wherein the method comprises diagnosing diastolic dysfunction or diastolic heart failure when the level is increased, relative to a control level.

3. The method of claim 1, wherein the method is for characterizing heart failure as diastolic heart failure or systolic heart failure wherein the method comprises characterizing the heart failure in the subject as (i) diastolic heart failure when the level is increased, relative to a control level, or (ii) systolic heart failure when the level is increased relative to a level from a subject not suffering from heart failure and decreased relative to a level from a subject suffering from diastolic heart failure.

4. A method, comprising (a) determining a first level of a myosin binding protein-C (MyBP-C) or fragment of MycBP-C having a molecular weight of 75 kDa, 40 kDa, or 25 kDa, wherein the MyBP-C or fragment is optionallyS-glutathionylated, in a biological sample obtained from the subject; and (b) determining a second level of the MyBP-C, or fragment thereof, in a biological sample obtained from the subject,
wherein the method is for monitoring a subject's risk for diastolic dysfunction or diastolic heart failure, wherein the first level is determined at a first timepoint and the second level is determined at a second timepoint which occurs after the first timepoint, and wherein the subject's risk for diastolic dysfunction or diastolic heart failure is decreased, when the second level is less than the first level; or
wherein the method is for determining efficacy of a treatment of diastolic dysfunction or diastolic heart failure in a subject, wherein the first level is determined before treatment and the second level is determined after treatment, and wherein the treatment is effective for treating diastolic dysfunction or diastolic heart failure in the subject, when the second level is less than the first level.

5. The method of any one of claims 1 to 4, wherein the MyBP-C has a molecular weight of 144 kDa or is a full-length MyBP-C, optionally, wherein the MyBP-C is S-glutathionylated.

6. The method of any one of claims 1 to 5, wherein the level of MyBP-C is (i) a level of MyBP-C normalized to a level of one or more fragments of MyBP-C or (ii) a ratio of [concentration of a 144 kDa MyBP-C]:[sum concentration of MyBP-C fragments], or wherein the level of MyBP-C or the level of MyBP-C fragment is normalized to a total level of MyBP-C in the sample,
and wherein the level of S-glutathionylated MyBP-C comprises (i) a level of S-glutathionylated MyBP-C normalized to a level of MyBP-C not comprising the S-glutathionylation or (ii) a level S-glutathionylated MyBP-C normalized to a total level of MyBP-C.

7. The method of any one of claims 1 to 6, wherein the MyBP-C or the MyBP-C fragment is S-glutathionylated at one or more of
i) Cys 239, Cys249, Cys 426, Cys 436, Cys 443, Cys 475 , Cys528, Cys566, Cys 623, Cys 651, and/or Cys 719, according to the amino acid position numbering of SEQ ID NO: 21, or
ii) Cys 475, Cys 623, and/or Cys 651, according to the amino acid position numbering of SEO ID NO: 21.

8. The method of any one of claims 1 to 7, wherein the level of the MyBP-C, or S-glutathionylated MyBP-C or fragment thereof, is determined via mass spectroscopy or an immunoassay, optionally wherein the immnunoassay comprises an radioimmunoassay, an immunohisto chemical assay, an immuno fluorescence assay, an ELISA, optionally a sandwich ELISA, a Western blot, affinity chromatography, or a combination thereof, optionally wherein the immunoassay comprises the use of an antibody, an antigen binding fragment of an antibody, or an aptamer that specifically binds to an epitope within MyBP-C, wherein the epitope comprises one or more of Cys 239, Cys249, Cys 426, Cys 436, Cys 443, Cys 475 , Cys528, Cys566, Cys 623, Cys 651, and/or Cys 719, according to the amino acid position numbering of SEQ ID NO: 21, optionally wherein the epitope comprises one or more of Cys 475, Cys 623, and/or Cys 651, according to the amino acid position numbering of SEQ ID NO: 21
optionally wherein the one or more Cys residues is/are S-glutathionylated,
optionally wherein the antibody, antigen binding fragment of an antibody or the aptamer does not bind to the one or more Cys residues when not S-glutathionylated.

9. The method of any one of claims 1 to 8, wherein the biological sample is blood or a fraction thereof, optionally plasma.

10. The method of any one of claims 1 to 9, wherein the subject is:
a) a human, optionally wherein the subject suffers from shortness of breath, optionally wherein the subject suffers from heart failure; and/or
b) not suffering from myocardial infarction; and/or
c) not suffering from systolic dysfunction, or
c) suffering from systolic dysfunction.

11. A therapeutic agent suitable for the treatment of diastolic dysfunction or diastolic heart failure for use in a method of treating said conditions in a subject that has been:
a) diagnosed with diastolic dysfunction or diastolic heart failure, or
b) determined as needing treatment for diastolic dysfunction or diastolic heart failure, or
c) identified as at risk for diastolic dysfunction or diastolic heart failure,
wherein said diagnosing, determining or identifying is performed using the method of any of claims 1-10 and wherein said therapeutic agent is BH₄ or ranolazine; or an agent as set out in the section entitled Therapeutic agents for the treatment of diastolic dysfunction or diastolic heart failure".

12. The method of any one of claims 1 to 11, further comprising determining (i) a level of asymmetric dimethylarginine (ADMA) in the biological sample obtained from the subject, (ii) a level of symmetric dimethylarginine (SDMA) in the biological sample obtained from the subject, (iii) a level of L-Arginine in the biological sample obtained from the subject, or (iv) a combination thereof,
optionally wherein the method further comprises determining a ratio of [the level of L-Arginine in the biological sample] to [the level of ADMA in the biological sample].

13. An isolated antibody, antigen binding fragment of an antibody or an aptamer that specifically binds to an epitope within MyBP-C, wherein the epitope comprises one or more of Cys 239, Cys249, Cys 426, Cys 436, Cys 443, Cys 475 , Cys528, Cys566, Cys 623, Cys 651, and/or Cys 719, according to the amino acid position numbering of SEQ ID NO: 21, optionally wherein the epitope comprises one or more of Cys 475, Cys 623, and/or Cys 651, according to the amino acid position numbering of SEQ ID NO: 21
wherein the one or more Cys residues is/are S-glutathionylated,
and wherein the antibody, antigen binding fragment of an antibody or the aptamer does not bind to the one or more Cys residues when not S-glutathionylated.

14. The isolated antibody, antigen binding fragment of an antibody or an aptamer of claim 13, conjugated to a heterologous moiety, optionally wherein the heterologous moiety is a detectable label, an enzyme, an enzyme substrate, or a binding partner of a high affinity binding interaction between two binding partners, optionally wherein the heterologous moiety is avidin or biotin.

15. A kit comprising
(A) the isolated antibody, antigen binding fragment of an antibody or the aptamer of any one of claims 13 or 14; optionally comprising
(B) a binding agent for BNP, and/or
(C) a binding agent for asymmetric dimethylarginine (ADMA), a binding agent for symmetric dirnethylarginine (SDMA), a binding agent for L-Arginine, or a combination thereof, and/or
(D) a unit for collecting plasma.

16. Use of an antibody, an antigen binding fragment of an antibody, or an aptamer that specifically binds to an epitope within MyBP-C, wherein the epitope comprises one or more of Cys 239, Cys249, Cys 426, Cys 436, Cys 443, Cys 475 , Cys528, Cys566, Cys 623, Cys 651, and/or Cys 719, according to the amino acid position numbering of SEQ ID NO: 21, optionally wherein the epitope comprises one or more of Cys 475, Cys 623, and/or Cys 651, according to the amino acid position numbering of SEQ ID NO: 21
optionally wherein the one or more Cys residues is/are S-glutathionylated
optionally wherein the antibody, antigen binding fragment of an antibody or the aptamer does not bind to the one or more Cys residues when not S-glutathionylated
in a method according to any one of claims 1-8.

## Patentansprüche

1. Verfahren, Folgendes umfassend: (i) Bestimmen eines Wertes eines Myosin bindenden Proteins C (MyBP-C) oder Fragments von MyBP-C mit einer Molekülmasse von 75 kDa, 40 kDa oder 25 kDa, wobei das MyBP-C oder Fragment optional S-glutathionyliert ist, in einer vom Subjekt gewonnenen biologischen Probe, wobei das Verfahren zu Folgendem dient: (A) Diagnostizieren von diastolischer Dysfunktion oder diastolischem Herzversagen, (B) Differenzieren zwischen diastolischer Dysfunktion und systolischer Dysfunktion oder zum Kennzeichnen von Herzversagen als diastolisches Herzversagen oder systolisches Herzversagen, (C) Bestimmen des Behandlungsbedarfs eines Subjekts wegen diastolischer Dysfunktion oder diastolischen Herzversagens oder (D) Erkennen eines Patienten mit einem Risiko für diastolische Dysfunktion oder diastolisches Herzversagen,
wobei, wenn das Verfahren dem Bestimmen des Behandlungsbedarfs eines Subjekts wegen diastolischer Dysfunktion oder diastolischen Herzversagens dient, das Verfahren umfasst, das Subjekt als ein Subjekt, das einer Behandlung wegen diastolischer Dysfunktion oder diastolischen Herzversagens bedarf, zu bestimmen, wenn der Wert verglichen mit einem Kontrollwert erhöht ist,
wobei, wenn das Verfahren dem Erkennen eines Patienten mit einem Risiko für diastolische Dysfunktion oder diastolisches Herzversagen dient, das Verfahren umfasst, das Subjekt als ein Subjekt mit einem Risiko für diastolische Dysfunktion oder diastolisches Herzversagen zu erkennen, wenn der Wert des MyBP-C oder Fragments davon oder eines S-glutathionylierten MyBP-C oder Fragments davon bezogen auf einen Kontrollwert erhöht ist.

2. Verfahren nach Anspruch 1, wobei das Verfahren dem Diagnostizieren von diastolischer Dysfunktion oder diastolischem Herzversagen dient, wobei das Verfahren umfasst, diastolische Dysfunktion oder diastolisches Herzversagen zu diagnostizieren, wenn der Wert bezogen auf einen Kontrollwert erhöht ist.

3. Verfahren nach Anspruch 1, wobei das Verfahren dem Kennzeichnen von Herzversagen als diastolisches Herzversagen oder systolisches Herzversagen dient, wobei das Verfahren umfasst, das Herzversagen bei dem Subjekt wie folgt zu kennzeichnen: (i) als diastolisches Herzversagen, wenn der Wert bezogen auf einen Kontrollwert erhöht ist, oder (ii) als systolisches Herzversagen, wenn der Wert bezogen auf einen Wert von einem Subjekt, das nicht an Herzversagen leidet, erhöht ist und bezogen auf einen Wert von einem Subjekt, das an diastolischem Herzversagen leidet, erniedrigt ist.

4. Verfahren, Folgendes umfassend: (a) Bestimmen eines ersten Wertes eines Myosin bindenden Proteins C (MyBP-C) oder Fragments von MyBP-C mit einer Molekülmasse von 75 kDa, 40 kDa oder 25 kDa, wobei das MyBP-C oder Fragment optional S-glutathionyliert ist, in einer vom Subjekt gewonnenen biologischen Probe; und (b) Bestimmen eines zweiten Wertes des MyBP-C oder Fragments davon in einer vom Subjekt gewonnenen Probe, wobei das Verfahren dem Überwachen des Risikos eines Subjekts für diastolische Dysfunktion oder diastolisches Herzversagen dient, wobei der erste Wert zu einem ersten Zeitpunkt bestimmt wird und der zweite Wert zu einem nach dem ersten Zeitpunkt eintretenden zweiten Zeitpunkt bestimmt wird und wobei das Risiko des Subjekts für diastolische Dysfunktion oder diastolisches Herzversagen vermindert ist, wenn der zweite Wert kleiner ist als der erste Wert; oder
wobei das Verfahren dem Bestimmen der Wirksamkeit einer Behandlung von diastolischer Dysfunktion oder diastolischem Herzversagen bei einem Subjekt dient, wobei der erste Wert vor der Behandlung bestimmt wird und der zweite Wert nach der Behandlung bestimmt wird und wobei die Behandlung zum Behandeln von diastolischer Dysfunktion oder diastolischem Herzversagen bei dem Subjekt wirksam ist, wenn der zweite Wert kleiner ist als der erste Wert.

5. Verfahren nach einem der Ansprüche 1 bis 4, wobei das MyBP-C eine Molekülmasse von 144 kDa aufweist oder ein MyBP-C mit voller Länge ist, wobei das MyBP-C optional S-glutathionyliert ist.

6. Verfahren nach einem der Ansprüche 1 bis 5, wobei der Wert des MyBP-C Folgendes ist: (i) ein Wert von MyBP-C, der auf einen Wert eines oder mehrerer Fragmente von MyBP-C normalisiert ist, oder (ii) ein Verhältnis von [Konzentration eines MyBP-C mit 144 kDa] : [Summenkonzentration von MyBP-C-Fragmenten], oder wobei der Wert von MyBP-C oder der Wert von MyBP-C-Fragment auf einen Gesamtwert von MyBP-C in der Probe normalisiert ist,
und wobei der Wert des S-glutathionylierten MyBP-C Folgendes umfasst: (i) einen Wert von S-glutathionyliertem MyBP-C, der auf einen Wert von MyBP-C, das die S-Glutathionylierung nicht umfasst, normalisiert ist, oder (ii) einen Wert von S-glutathionyliertem MyBP-C, der auf einen Gesamtwert von MyBP-C normalisiert ist.

7. Verfahren nach einem der Ansprüche 1 bis 6, wobei das MyBP-C oder das MyBP-C-Fragment an einer oder mehreren der Folgenden S-glutathionyliert ist:
i) Cys 239, Cys 249, Cys 426, Cys 436, Cys 443, Cys 475, Cys 528, Cys 566, Cys 623, Cys 651 und/oder Cys 719 gemäß der Aminosäurepositionsnummerierung von SEQ ID NR. 21 oder
ii) Cys 475, Cys 623 und/oder Cys 651 gemäß der Aminosäurepositionsnummerierung von SEQ ID NR. 21.

8. Verfahren nach einem der Ansprüche 1 bis 7, wobei der Wert des MyBP-C oder S-glutathionylierten MyBP-C oder Fragments davon mittels Massenspektroskopie oder eines Immunassays bestimmt wird, wobei der Immunassay optional einen Radioimmunassay, einen immunhistochemischen Assay, einen Immunfluoreszenzassay, einen ELISA, optional einen Sandwich-ELISA, einen Western Blot, Affinitätschromatographie oder eine Kombination derselben umfasst, wobei der Immunassay optional die Verwendung eines Antikörpers, eines antigenbindenden Fragments eines Antikörpers oder eines Aptamers, das spezifisch an ein Epitop innerhalb von MyBP-C bindet, umfasst, wobei das Epitop eine oder mehrere von Cys 239, Cys 249, Cys 426, Cys 436, Cys 443, Cys 475, Cys 528, Cys 566, Cys 623, Cys 651 und/oder Cys 719 gemäß der Aminosäurepositionsnummerierung von SEQ ID NR. 21 umfasst, wobei das Epitop optional eine oder mehrere von Cys 475, Cys 623 und/oder Cys 651 gemäß der Aminosäurepositionsnummerierung von SEQ ID NR. 21 umfasst,
wobei der eine oder die mehreren Cys-Rest(e) optional S-glutathionyliert ist/sind,
wobei der Antikörper, das antigenbindende Fragment eines Antikörpers oder das Aptamer optional nicht an den einen oder die mehreren Cys-Rest(e) bindet, wenn diese(r) nicht S-glutathionyliert ist/sind.

9. Verfahren nach einem der Ansprüche 1 bis 8, wobei die biologische Probe Blut oder ein Anteil davon, optional Plasma, ist.

10. Verfahren nach einem der Ansprüche 1 bis 9, wobei das Subjekt Folgendes ist:
a) ein Mensch, wobei das Subjekt optional an Atemnot leidet, wobei das Subjekt optional an Herzversagen leidet; und/oder
b) nicht an einem Myokardinfarkt leidet; und/oder
c) nicht an systolischer Dysfunktion leidet oder
c) an systolischer Dysfunktion leidet.

11. Therapeutikum, das zur Behandlung von diastolischer Dysfunktion oder diastolischem Herzversagen geeignet ist, zur Verwendung in einem Verfahren zum Behandeln dieser Zustände bei einem Subjekt, für das Folgendes zutrifft:
a) es wurde diastolische Dysfunktion oder diastolisches Herzversagen diagnostiziert, oder
b) es wurde bestimmt, dass Behandlungsbedarf wegen diastolischer Dysfunktion oder diastolischen Herzversagens besteht, oder
c) es wurde ein Risiko für diastolische Dysfunktion oder diastolisches Herzversagen erkannt,
wobei das Diagnostizieren, Bestimmen oder Erkennen unter Verwendung des Verfahrens nach einem der Ansprüche 1-10 erfolgt und wobei das Therapeutikum BH₄ oder Ranolazin; oder ein im Abschnitt mit der Überschrift "Therapeutika zur Behandlung von diastolischer Dysfunktion oder diastolischem Herzversagen" beschriebener Wirkstoff ist.

12. Verfahren nach einem der Ansprüche 1 bis 11, das ferner umfasst, Folgendes zu bestimmen: (i) einen Wert von asymmetrischem Dimethylarginin (ADMA) in der vom Subjekt gewonnenen biologischen Probe, (ii) einen Wert von symmetrischem Dimethylarginin (SDMA) in der vom Subjekt gewonnenen biologischen Probe, (iii) einen Wert von L-Arginin in der vom Subjekt gewonnenen biologischen Probe oder (iv) eine Kombination derselben, wobei das Verfahren optional ferner umfasst, ein Verhältnis von [dem Wert von L-Arginin in der biologischen Probe] zu [dem Wert von ADMA in der biologischen Probe] zu bestimmen.

13. Isolierter/s Antikörper, antigenbindendes Fragment eines Antikörpers oder Aptamer, das spezifisch an ein Epitop innerhalb von MyBP-C bindet, wobei das Epitop eine oder mehrere von Cys 239, Cys 249, Cys 426, Cys 436, Cys 443, Cys 475, Cys 528, Cys 566, Cys 623, Cys 651 und/oder Cys 719 gemäß der Aminosäurepositionsnummerierung von SEQ ID NR. 21 umfasst, wobei das Epitop optional eine oder mehrere von Cys 475, Cys 623 und/oder Cys 651 gemäß der Aminosäurepositionsnummerierung von SEQ ID NR. 21 umfasst,
wobei der eine oder die mehreren Cys-Rest(e) S-glutathionyliert ist/sind,
und wobei der Antikörper, das antigenbindende Fragment eines Antikörpers oder das Aptamer nicht an den einen oder die mehreren Cys-Rest(e) bindet, wenn diese(r) nicht S-glutathionyliert ist/sind.

14. Isolierter/s Antikörper, antigenbindendes Fragment eines Antikörpers oder Aptamer nach Anspruch 13, der/das mit einem heterologen Molekülteil konjugiert ist, wobei das heterologe Molekülteil optional eine nachweisbare Markierung, ein Enzym, ein Enzymsubstrat oder ein Bindungspartner mit Hochaffinitäts-Bindungsinteraktion zwischen zwei Bindungspartnern ist, wobei das heterologe Molekülteil optional Avidin oder Biotin ist.

15. Set, Folgendes umfassend:
(A) den/das isolierte(n) Antikörper, antigenbindende Fragment eines Antikörpers oder Aptamer nach einem der Ansprüche 13 oder 14; optional Folgendes umfassend:
(B) ein Bindemittel für BNP und/oder
(C) ein Bindemittel für asymmetrisches Dimethylarginin (ADMA), ein Bindemittel für symmetrisches Dimethylarginin (SDMA), ein Bindemittel für L-Arginin oder eine Kombination derselben und/oder
(D) eine Einheit zum Sammeln von Plasma.

16. Verwendung eines Antikörpers, eines antigenbindenden Fragments eines Antikörpers oder eines Aptamers, das spezifisch an ein Epitop innerhalb von MyBP-C bindet, wobei das Epitop eine oder mehrere von Cys 239, Cys 249, Cys 426, Cys 436, Cys 443, Cys 475, Cys 528, Cys 566, Cys 623, Cys 651 und/oder Cys 719 gemäß der Aminosäurepositionsnummerierung von SEQ ID NR. 21 umfasst, wobei das Epitop optional eine oder mehrere von Cys 475, Cys 623 und/oder Cys 651 gemäß der Aminosäurepositionsnummerierung von SEQ ID NR. 21 umfasst,
wobei der eine oder die mehreren Cys-Rest(e) optional S-glutathionyliert ist/sind,
wobei der Antikörper, das antigenbindende Fragment eines Antikörpers oder das Aptamer optional nicht an den einen oder die mehreren Cys-Rest(e) bindet, wenn diese(r) nicht S-glutathionyliert ist/sind,
in einem Verfahren nach einem der Ansprüche 1-8.

## Revendications

1. Procédé comprenant (i) la détermination d'un taux de protéine C de liaison à la myosine (MyBP-C) ou de fragment de MyBP-C ayant un poids moléculaire de 75 kDa, 40 kDa ou 25 kDa, dans lequel la MyBP-C ou le fragment est facultativement S-glutathionylé(e), dans un échantillon biologique prélevé sur le patient, lequel procédé est pour (A) le diagnostic d'une dysfonction diastolique ou d'une insuffisance cardiaque diastolique, (B) la différenciation entre dysfonction diastolique et dysfonction systolique ou pour la caractérisation de l'insuffisance cardiaque en tant qu'insuffisance cardiaque diastolique ou insuffisance cardiaque systolique, (C) la détermination d'une nécessité de traitement du patient pour la dysfonction diastolique ou l'insuffisance cardiaque diastolique, ou (D) l'identification d'un patient présentant un risque de dysfonction diastolique ou d'insuffisance cardiaque diastolique,
où, lorsque le procédé est pour la détermination d'une nécessité de traitement du patient pour la dysfonction diastolique ou l'insuffisance cardiaque diastolique, le procédé comprend la détermination du patient en tant que patient nécessitant un traitement pour la dysfonction diastolique ou l'insuffisance cardiaque diastolique lorsque le taux est augmenté, par rapport à un taux de contrôle,
où, lorsque le procédé est pour l'identification d'un patient présentant un risque de dysfonction diastolique ou d'insuffisance cardiaque diastolique, le procédé comprend l'identification du patient en tant que patient présentant un risque de dysfonction diastolique ou d'insuffisance cardiaque diastolique, lorsque le taux de MyBP-C ou de fragment de celle-ci, ou de MyBP-C S-glutathionylée ou d'un fragment de celle-ci, est augmenté, par rapport à un taux de contrôle.

2. Procédé selon la revendication 1, lequel procédé est pour le diagnostic d'une dysfonction diastolique ou d'une insuffisance cardiaque diastolique, lequel procédé comprend le diagnostic d'une dysfonction diastolique ou d'une insuffisance cardiaque diastolique lorsque le taux est augmenté, par rapport à un taux de contrôle.

3. Procédé selon la revendication 1, lequel procédé est pour la caractérisation de l'insuffisance cardiaque en tant qu'insuffisance cardiaque diastolique ou insuffisance cardiaque systolique lorsque le procédé comprend la caractérisation de l'insuffisance cardiaque chez le patient en tant que (i) insuffisance cardiaque diastolique lorsque le taux est augmenté, par rapport à un taux de contrôle, ou (ii) insuffisance cardiaque systolique lorsque le taux est augmenté, par rapport à un taux d'un patient ne souffrant pas d'insuffisance cardiaque et réduit par rapport à un taux d'un patient souffrant d'insuffisance cardiaque diastolique.

4. Procédé, comprenant (a) la détermination d'un premier taux de protéine C de liaison à la myosine (MyBP-C) ou de fragment de MyBP-C ayant un poids moléculaire de 75 kDa, 40 kDa ou 25 kDa, dans lequel la MyBP-C ou le fragment est facultativement S-glutathionylé(e), dans un échantillon biologique prélevé sur le patient ; et (b) la détermination d'un deuxième taux de MyBP-C ou de fragment de celle-ci, dans un échantillon biologique prélevé sur le patient,
lequel procédé est pour le contrôle du risque de dysfonction diastolique ou d'insuffisance cardiaque diastolique du patient, où le premier taux est déterminé à un premier moment et le deuxième taux est déterminé à un deuxième moment qui suit le premier moment, et où le risque de dysfonction diastolique ou d'insuffisance cardiaque diastolique du patient est réduit, lorsque le deuxième taux est inférieur au premier taux ; ou
lequel procédé est pour la détermination de l'efficacité d'un traitement de la dysfonction diastolique ou de l'insuffisance cardiaque diastolique chez un patient, où le premier taux est déterminé avant le traitement et le deuxième taux est déterminé après le traitement, et où le traitement est efficace pour traiter la dysfonction diastolique ou l'insuffisance cardiaque chez le patient, lorsque le deuxième taux est inférieur au premier taux.

5. Procédé selon l'une quelconque des revendications 1 à 4, dans lequel la MyBP-C a un poids moléculaire de 144 kDa ou est une MyBP-C pleine longueur, facultativement, où la MyBP-C est S-glutathionylée.

6. Procédé selon l'une quelconque des revendications 1 à 5, dans lequel le taux de MyBP-C est (i) un taux de MyBP-C normalisé selon un taux d'un ou plusieurs fragments de MyBP-C ou (ii) un rapport de [concentration en MyBP-C de 144 kDa] : [somme de la concentration en fragments de MyBP-C], ou dans lequel le taux de MyBP-C ou le taux de fragment de MyBP-C est normalisé selon un taux total de MyBP-C dans l'échantillon,
et dans lequel le taux de MyBP-C S-glutathionylée comprend (i) un taux de MyBP-C S-glutathionylée normalisé selon un taux de MyBP-C ne comprenant pas la S-glutathionylation ou (ii) un taux de MyBP-C S-glutathionylée normalisé selon un taux total de MyBP-C.

7. Procédé selon l'une quelconque des revendications 1 à 6, dans lequel la MyBP-C ou le fragment de MyBP-C est S-glutathionylé(e) au niveau d'un ou de plusieurs de
i) Cys 239, Cys 249, Cys 426, Cys 436, Cys 443, Cys 475, Cys 528, Cys 566, Cys 623, Cys 651 et/ou Cys 719, conformément à la numérotation des positions des acides aminés de SÉQ ID n° : 21, ou
ii) Cys 475, Cys 623 et/ou Cys 651 conformément à la numérotation des positions des acides aminés de SÉQ ID n° : 21.

8. Procédé selon l'une quelconque des revendications 1 à 7, dans lequel le taux de MyBP-C, ou de MyBP-C S-glutathionylée ou du fragment de celle-ci, est déterminé par spectroscopie de masse ou par immunodosage, facultativement dans lequel
l'immunodosage comprend un radio-immunodosage, un dosage immunohistochimique, un dosage par immunofluorescence, un dosage ELISA, facultativement une méthode ELISA en sandwich, un buvardage de type Western, une chromatographie d'affinité ou une combinaison de ceux-ci, facultativement dans lequel l'immunodosage comprend l'utilisation d'un anticorps, d'un fragment de liaison à l'antigène d'un anticorps ou un aptamère qui se lie spécifiquement à un épitope dans MyBP-C, où l'épitope comprend un ou plusieurs de Cys 239, Cys 249, Cys 426, Cys 436, Cys 443, Cys 475, Cys 528, Cys 566, Cys 623, Cys 651 et/ou Cys 719, conformément à la numérotation des positions des acides aminés de SÉQ ID n° : 21, facultativement dans lequel l'épitope comprend un ou plusieurs de Cys 475, Cys 623 et/ou Cys 651 conformément à la numérotation des positions des acides aminés de SÉQ ID n° : 21
facultativement dans lequel un ou plusieurs des résidus Cys est/sont S-glutathionylé(s), facultativement dans lequel l'anticorps, le fragment de liaison à l'antigène d'un anticorps ou l'aptamère ne se lie pas à un ou plusieurs des résidus Cys lorsqu'il(s) n'est/ne sont pas S-glutathionylé(s).

9. Procédé selon l'une quelconque des revendications 1 à 8, dans lequel l'échantillon biologique est du sang ou une fraction de celui-ci, facultativement du plasma.

10. Procédé selon l'une quelconque des revendications 1 à 9, dans lequel le patient :
a) est un être humain, facultativement dans lequel le patient souffre d'essoufflement, facultativement dans lequel le patient souffre d'insuffisance cardiaque ; et/ou
b) ne souffre pas d'infarctus du myocarde ; et/ou
c) ne souffre pas de dysfonction systolique, ou
c) souffre de dysfonction systolique.

11. Agent thérapeutique approprié pour le traitement de la dysfonction systolique ou de l'insuffisance cardiaque diastolique pour une utilisation dans un procédé de traitement desdites conditions chez un patient ayant été :
a) diagnostiqué comme souffrant de dysfonction diastolique ou d'insuffisance cardiaque diastolique, ou
b) déterminé comme nécessitant un traitement pour la dysfonction diastolique ou l'insuffisance cardiaque diastolique, ou
c) identifié comme présentant un risque de dysfonction diastolique ou d'insuffisance cardiaque diastolique,
dans lequel ledit diagnostic, ladite détermination ou ladite identification est effectué(e) à l'aide du procédé selon l'une quelconque des revendications 1 à 10 et dans lequel ledit agent thérapeutique est BH₄ ou la ranolazine ou un agent tel que décrit dans la section intitulée « Agents thérapeutiques pour le traitement de la dysfonction diastolique ou de l'insuffisance cardiaque diastolique ».

12. Procédé selon l'une quelconque des revendications 1 à 11, comprenant en outre la détermination (i) d'un taux de diméthylarginine asymétrique (DMAA) dans l'échantillon biologique prélevé sur le patient, (ii) d'un taux de diméthylarginine symétrique (DMAS) dans l'échantillon biologique prélevé sur le patient, (iii) d'un taux de L-arginine dans l'échantillon biologique prélevé sur le patient, ou (iv) d'une combinaison de ceux-ci,
facultativement lequel procédé comprend en outre la détermination d'un rapport du [taux de L-arginine dans l'échantillon biologique] au [taux de DMAA dans l'échantillon biologique].

13. Anticorps isolé, fragment de liaison à l'antigène d'un anticorps ou aptamère qui se lie spécifiquement à un épitope dans MyBP-C, où l'épitope comprend un ou plusieurs de Cys 239, Cys 249, Cys 426, Cys 436, Cys 443, Cys 475, Cys 528, Cys 566, Cys 623, Cys 651 et/ou Cys 719, conformément à la numérotation des positions des acides aminés de SÉQ ID n° : 21, facultativement dans lequel l'épitope comprend un ou plusieurs de Cys 475, Cys 623 et/ou Cys 651 conformément à la numérotation des positions des acides aminés de SÉQ ID n° : 21
dans lequel un ou plusieurs des résidus Cys est/sont S-glutathionylé(s),
et dans lequel l'anticorps, le fragment de liaison à l'antigène d'un anticorps ou l'aptamère ne se lie pas à un ou plusieurs des résidus Cys lorsqu'il(s) n'est/ne sont pas S-glutathionylé(s).

14. Anticorps isolé, fragment de liaison à l'antigène d'un anticorps ou aptamère selon la revendication 13, conjugué à un fragment hétérologue, facultativement dans lequel le fragment hétérologue est un marqueur détectable, une enzyme, un substrat enzymatique ou un partenaire de liaison d'une interaction de liaison de haute affinité entre deux partenaires de liaison, facultativement dans lequel le fragment hétérologue est l'avidine ou la biotine.

15. Kit comprenant
(A) l'anticorps isolé, le fragment de liaison à l'antigène d'un anticorps ou l'aptamère selon l'une quelconque des revendications 13 ou 14 ; comprenant facultativement
(B) un agent de liaison pour le BNP, et/ou
(C) un agent de liaison pour la diméthylarginine asymétrique (DMAA), un agent de liaison pour la diméthylarginine symétrique (DMAS), un agent de liaison pour la L-arginine ou une combinaison de ceux-ci, et/ou
(D) une unité de recueil de plasma.

16. Utilisation d'un anticorps, d'un fragment de liaison à l'antigène d'un anticorps ou d'un aptamère qui se lie spécifiquement à un épitope dans MyBP-C, où l'épitope comprend un ou plusieurs de Cys 239, Cys 249, Cys 426, Cys 436, Cys 443, Cys 475, Cys 528, Cys 566, Cys 623, Cys 651 et/ou Cys 719, conformément à la numérotation des positions des acides aminés de SÉQ ID n° : 21, facultativement dans lequel l'épitope comprend un ou plusieurs de Cys 475, Cys 623 et/ou Cys 651 conformément à la numérotation des positions des acides aminés de SÉQ ID n° : 21
facultativement dans lequel un ou plusieurs des résidus Cys est/sont S-glutathionylé(s), facultativement dans lequel l'anticorps, le fragment de liaison à l'antigène d'un anticorps ou l'aptamère ne se lie pas à un ou plusieurs des résidus Cys lorsqu'il(s) n'est/ne sont pas S-glutathionylé(s)
dans un procédé selon l'une quelconque des revendications 1 à 8.
